# FASCICULE DE BREVET EUROPEEN

(11) **EP 0 501 875 B1**
(45) Date de publication et mention de la délivrance du brevet: **08.06.1994**
(21) Numéro de dépôt: 92400485.6
(22) Date de dépôt: 25.02.1992
(51) Int. Cl.: C07C 237/42, C07C 237/46, C07C 235/16, A61K 49/04

(54) **Nouveaux composés poly-iodés, procédé de préparation, produit de contraste les contenant**
Polyjodverbindungen, Verfahren zu ihrer Herstellung und diese enthaltende Kontrastmittel
Poly-iodinated compounds, process of preparation, contrast agents containing them

(30) Priorité: 25.02.1991 FR 9102226
(43) Date de publication de la demande: 02.09.1992
(73) Titulaire: GUERBET S.A., F-93420 Villepinte (FR)
(72) Inventeur: Dugast-Zrihen, Maryse, F-75013 Paris (FR); Meyer, Dominique, F-94100 Saint Maur des Fosses (FR)
(74) Mandataire: Polus, Camille

(56) Documents cités:
- EP-A- 0 118 347
- EP-A- 0 308 364
- EP-A- 0 357 467
- DE-A- 3 429 949

## Description

La présente invention concerne des nouveaux composés poly-iodés utilisables dans les produits de constraste pour la radiographie.

L'invention concerne également un procédé de préparation de ces composés ainsi que les produits de contraste les contenant.

EP-0 308364 décrit des dérivés de bis-(3,5 dicarbamoyl-2,4,6-triiodoanilide) utiles en tant qu'agents de contraste en radiologie.

On a à présent découvert des dérivés iodés du biphényle présentant d'excellentes propriétés en tant qu'agents de contraste en radiologie.

L'invention a ainsi pour objet des composés poly-iodés de formule : 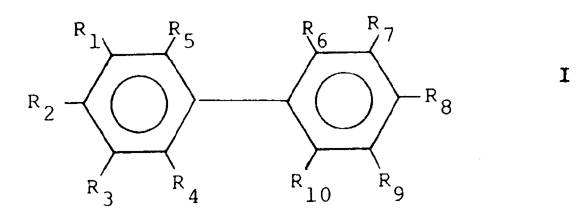
dans laquelle R₁, R₂, R₃, R₄, R₅, R₆, R₇, R₈, R₉ et R₁₀ identiques ou différents entre eux sont choisis parmi un atome d'iode un groupe de formule 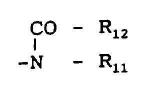
dans laquelle R₁₁ et R₁₂ identiques ou différents entre eux représentent un atome d'hydrogène, un groupe alkyle en C₁-C₆ linéaire ou ramifié, un groupe hydroxy- ou polyhydroxy-alkyle en C₁-C₆ linéaire ou ramifié, comportant en outre éventuellement un ou plusieurs groupes alcoxy en C₁-C₆, notamment méthoxy ou éthoxy, un groupe alkoxy (en C₁-C₆) alkyle en C₁-C₆ linéaire ou ramifié ou un groupe hydroxy- ou polyhydroxy-alkoxy (en C₁-C₆) alkyle en C₁-C₆ linéaire ou ramifié comportant de deux à cinq groupes - OH, un groupe de formule 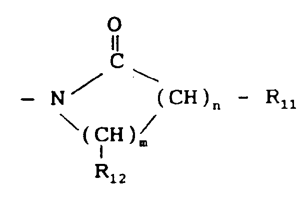
dans laquelle R₁₁ et R₁₂ identiques ou différents sont tels que définis précédemment ou représentent un groupe hydroxy et n et m sont des entiers choisis de façon que n+m soit un nombre de 3 à 6, un groupe -COO⁻M⁺ ou -COOH, M⁺ représentant un cation physiologiquement acceptable minéral ou organique, un groupe de formule 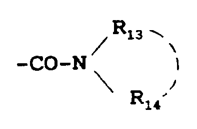
un groupe de formule 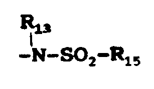
et un groupe de formule 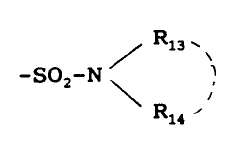
dans lesquelles R₁₃ et R₁₄ identiques ou différents entre eux représentent un atome d'hydrogène, un groupe alkyle en C₁-C₆ linéaire ou ramifié, un groupe hydroxy- ou polyhydroxy-alkyle en C₁-C₆ linéaire ou ramifié, comportant en outre éventuellement un ou plusieurs groupes alcoxy en C₁-C₆, notamment méthoxy ou éthoxy, un groupe alkoxy (en C₁-C₆) alkyle en C₁-C₆ linéaire ou ramifié ou un groupe hydroxy- ou polyhydroxy-alkoxy (en C₁-C₆) alkyle en C₁-C₆ linéaire ou ramifié comportant de deux à cinq groupes - OH, ou R₁₃ et R₁₄ forment ensemble un groupe alkylène en C₄-C₈, hydroxyalkylène en C₄-C₈ ou polyhydroxyalkylène en C₄-C₈, à chaîne linéaire ou ramifiée, de sorte que R₁₃ et R₁₄ forment avec l'atome d'azote auquel ils sont fixés un hétérocycle azoté à 5 ou 6 chaînons éventuellement substitué par un ou plusieurs groupes hydroxy, ou hydroxyalkyle en C₁-C₄ et R₁₅ a les mêmes significations que R₁₃ sauf un atome d'hydrogène, sous réserve qu'au moins deux groupes parmi R₁, R₂, R₃, R₄, R₅, R₆, R₇, R₈, R₉ et R₁₀ représentent un atome d'iode.

L'invention vise en particulier des composés poly-iodés de formule I, dans laquelle R₁, R₂, R₃, R₄, R₅, R₆, R₇, R₈, R₉ et R₁₀ identiques ou différents entre eux sont choisis parmi un atome d'iode, un groupe de formule 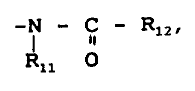
dans laquelle R₁₁ et R₁₂ identiques ou différents entre eux représentent un atome d'hydrogène, un groupe alkyle en C₁-C₆ linéaire ou ramifié, un groupe hydroxy- ou polyhydroxy-alkyle en C₁-C₆ linéaire ou ramifié, ou un groupe hydroxy ou polyhydroxyalkoxyalkyle en C₁-C₆, linéaire ou ramifié, comportant en outre éventuellement un ou plusieurs groupes alcoxy en C₁-C₆, un groupe alkoxyalkyle en C₁-C₆, linéaire ou ramifié contenant de deux à cinq groupes - OH, et un groupe de formule 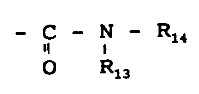
dans laquelle R₁₃ et R₁₄ identiques ou différents entre eux ont les mêmes significations que R₁₁ et R₁₂, sous réserve qu'au moins deux groupes parmi R₁, R₂, R₃, R₄, R₅, R₆, R₇, R₈, R₉ et R₁₀ représentent un atome d'iode.

Un premier groupe de produits préférés comprend les composés tétra-iodés de formule générale II : 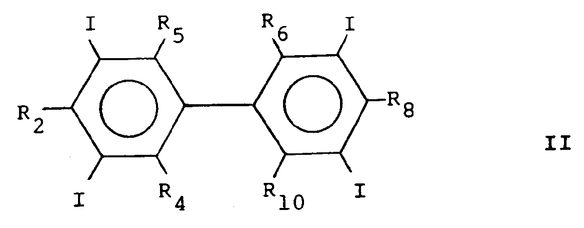
dans laquelle R₂, R₄, R₅, R₆, R₈ et R₁₀ sont tels que définis précédemment.

Un second groupe de produits préférés comprend les composés hexa-iodés de formule générale III : 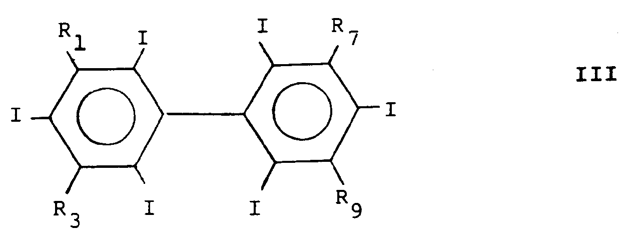
dans laquelle R₁, R₃, R₇ et R₉ sont tels que définis précédemment.

On préfère les composés de formule générale I dans laquelle R₁₁, R₁₂, R₁₃ et R₁₄ sont choisis parmi -H, -CH₃, -CH₂CH₃, -CH₂OH, -CHOH -CH₃, -CH(OCH₃) CH₂OH, -CHOH-CH₂(OCH₃), 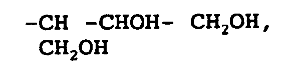 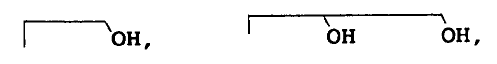 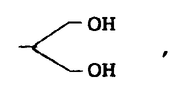
-CH₂-CHOH₃ et CH₂OCH₂CH₃.

Des groupes 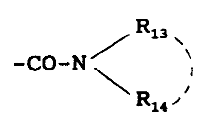
préférés sont ceux dans lesquels :
- R₁₃ représente le groupe 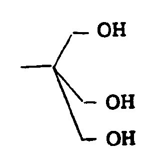 et R₁₄ est choisi parmi -H, -CH₃, -CH₂CH₂OH et -CH₂CHOHCH₂OH;
- R₁₃ représente le groupe 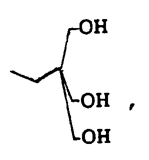 et R₁₄ est choisi parmi H, -CH₃, CH₂CH₂OH et -CH₂CHOHCH₂OH;
- R₁₃ représente le groupe 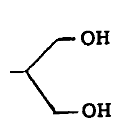 et R₁₄ est choisi parmi -H, -CH₃, -CH₂CH₂OH, -CH₂CHOHCH₂OH et -CH(CH₂OH)₂;
- R₁₃ et R₁₄ forment avec l'atome d'azote auquel ils sont liés un groupe 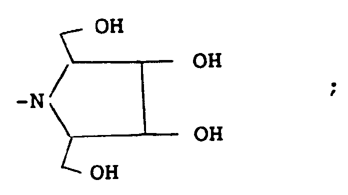
- R₁₃ représente un groupe -CH₂CHOHCHOHCH₂OH et R₁₄ est choisi parmi -H, -CH₃, -CH₂CH₂OH, -CH₂CHOHCH₂OH et -CH(CH₂OH)₂;
- R₁₃ représente un groupe 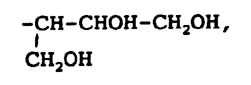 et R₁₄ est choisi parmi -H, -CH₃, -CH₂CH₂OH, -CH₂CHOHCH₂OH et -CH(CH₂OH)₂.

Parmi les groupes préférés 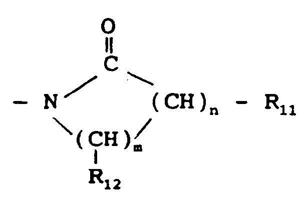
on peut citer les groupes suivants :

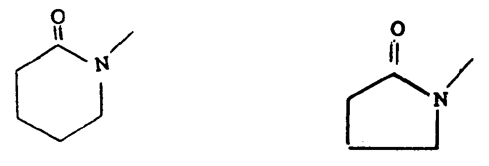

Parmi les composés tétra-iodés de formule générale II, on préfère en particulier ceux dans lesquels:
(I) R₂ et R₈ sont identiques et représentent le groupe 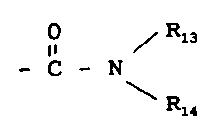 et R₄, R₅, R₆ et R₁₀ sont identiques et représentent le groupe 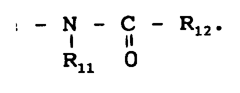
(II) R₂ et R₈ sont identiques et représentent le groupe 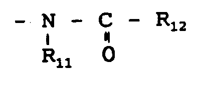 et R₄, R₅, R₆ et R₁₀ sont identiques et représentent le groupe 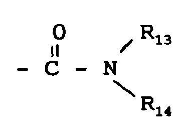
(III) R₂, R₅, R₆ et R₈ sont identiques et représentent le groupe 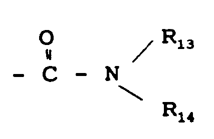 et R₄ et R₁₀ sont idenques et représentent le groupe 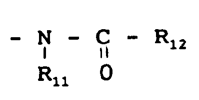
(IV) R₂, R₄, R₅, R₆, R₈, et R₁₀ sont identiques et représentent le groupe 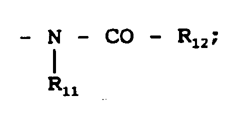 R₁₁, R₁₂, R₁₃ et R₁₄ étant tels que définis précédemment.

Les composés de formule II préférés sont ceux dans lesquels :
- R₂ et R₈ représentent le groupe - CO - NH - CH₂ - CHOH - CH₂OH et R₄, R₅ R₆ et R₁₀ représentent le groupe - NH - CO - CHOH - CH₃ (composé n° 4);
- R₂ et R₈ représentent le groupe - NH - CO - CH₂OH et R₄, R₅, R₆ et R₁₀ représentent le groupe - CO - NH - CH₂ - CH₂OH (composé n° 2);
- R₂ et R₈ représentent le groupe 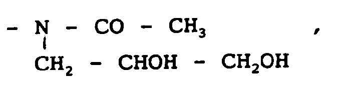 et R₄, R₅, R₆ et R₁₀ représentent le groupe - CO - NH - CH₂ - CH₂OH (composé n° 3);
- R₂ et R₈ représentent le groupe - NH - CO - CH₂OH et R₄, R₅, R₆ et R₁₀ représentent le groupe - CO - NH - CH₂ - CHOH - CH₂OH (composé n ° 1);
- R₂, R₅, R₆ et R₈ représentent le groupe - CO - NH - CH₂ - CHOH - CH₂OH et R₄ et R₁₀ représentent le groupe - NH - CO - CH₂OH (composé n° 7 );
- R₂, R₄, R₅, R₆, R₈ et R₁₀ représentent le groupe - NH - CO - CHOH - CH₃ (composé n° 5 );
- R₂, R₄, R₆ et R₈ représentent le groupe - CO - NH - CH₂ - CH₂OH et R₅ et R₁₀ représentent le groupe - NH - CO - CHOH - CH₃ (composé n° 6).

Parmi les composés hexa-iodés de formule générale III, on préfère en particulier ceux dans lesquels :
(V) R₁ et R₇ sont identiques et représentent le groupe 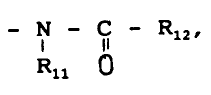 et R₃ et R₉ sont identiques et représentent le groupe 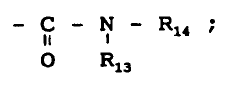 R₁₁, R₁₂, R₁₃ et R₁₄ étant tels que définis précédemment;
(VI) R₁ et R₉ sont identiques et représentent le groupe 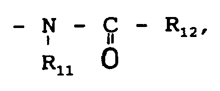 et R₃ et R₇ sont identiques et représentent le groupe 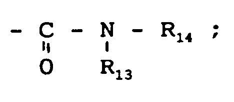 R₁₁, R₁₂, R₁₃ et R₁₄ étant tels que définis précédemment ;
(VII) R₁, R₃, R₇ et R₉ sont identiques et représentent le groupe 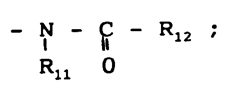 R₁₁, et R₁₂ étant tels que définis précédemment ;
(VIII) R₁, R₃ et R₇ sont identiques et représentent le groupe 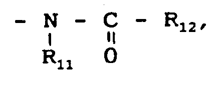 et R₉ représente le groupe 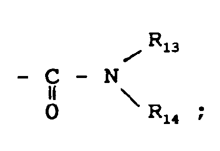 R₁₁, R₁₂, R₁₃ et R₁₄ étant tels que définis précédemment.
(IX) R₁, R₃, R₇ et R₉ sont identiques et représentent le groupe 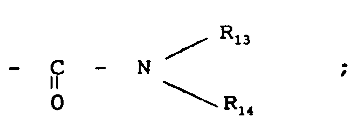 R₁₃ et R₁₄ étant tels que définis précédemment.

Les composés de formule III préférés sont ceux dans lesquels :
- R₁ et R₇ représentent le groupe
- R₁ et R₇ représentent le groupe
   - NH - CO - CH (CH₂OH)₂ ; et R₃ et R₉ représentent le groupe 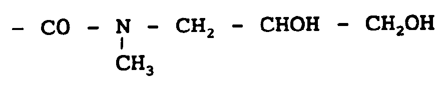 (composé n° 8)
- R₁ et R₇ représentent le groupe - NH - CO - CH(CH₂OH)₂ et R₃ et R₉ représentent le groupe
   - CO - N(CH₂ - CHOH - CH₂OH)₂ (composé n° 9)
- R₁ et R₇ représentent le groupe -NH-CO-CH₂OH et R₃ et R₉ représentent le groupe 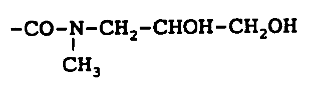 (composé n° 10),
- R₁ et R₇ représentent le groupe -NH-CO-CHOH-CH₂OH et R₃ et R₉ représentent le groupe 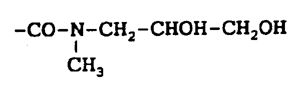 (composé n° 11),
- R₁ et R₇ représentent le groupe -NH-CO-CH₂OH et R₃ et R₉ représentent le groupe 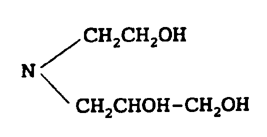 (composé n° 12),
- R₁ et R₇ représentent le groupe 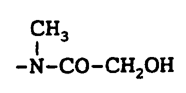 et R₃ et R₉ représentent le groupe 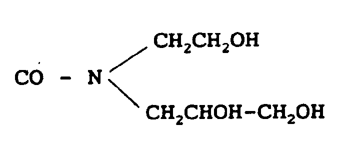 (composé n° 13),
- R₁ et R₇ représentent le groupe -N-(COCH₃)₂ et R₃ et R₉ représentent le groupe 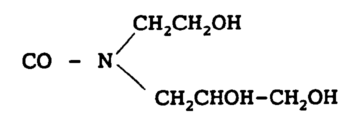 (composé n° 14),
- R₁ et R₇ représentent le groupe 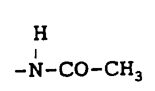 et R₃ et R₉ représentent le groupe 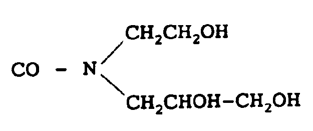 (composé n° 15),
- R₁ et R₇ représentent le groupe 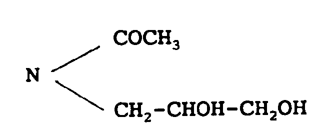 et R₃ et R₉ représentent le le groupe 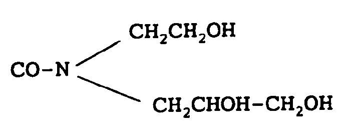 (composé n° 16).

Les composés de formule générale I peuvent être préparés par des réactions d'alkylation et/ou d'acylation.

Les composés de formule générale I peuvent notamment être préparés par un procédé comprenant les étapes suivantes :
a) couplage des dérivés benzèniques de formule IV et V 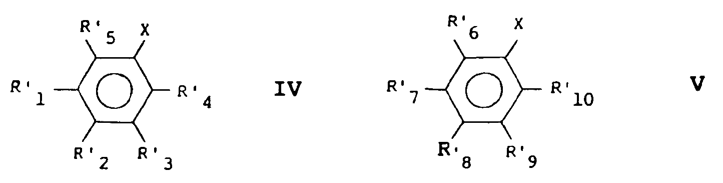 X étant choisi parmi le chlore, le brome et l'iode et R′₁, R′₂, R′₃, R′₄, R′₅, R′₆, R′₇, R′₈, R′₉ et R′₁₀ identiques ou différents étant choisis parmi un atome d'hydrogène et les groupes - NO₂ et - CO₂R avec R représentant un groupe alkyle en C₁-C₆, de façon à obtenir un composé de formule VI : 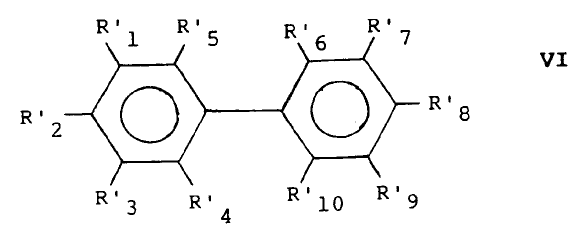 dans laquelle R′₁, R′₂, R′₃, R′₄, R′₅, R′₆, R′₇, R′₈, R′₉, R′₁₀ sont tels que définis précédemment ;
b) amidification des groupes - CO₂R par une amine de formule 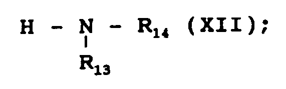
c) réduction des groupes nitro en groupes amino ;
d) iodation dans les conditions classiques ;
e) éventuellement protection des groupes -OH à l'aide de groupes protecteurs usuels,
f) acylation des groupes amino aromatiques par un chlorure d'acide de formule R′₁₂- COCl ;
   R′₁₂ représentant un groupe R₁₂ tel que défini précédemment dont les groupes hydroxy sont protégés à l'aide d'un groupe protecteur usuel ; et, soit
g) éventuellement alkylation des groupes amido par un réactif de formule Z - R₁₁, Z étant un groupe labile, tel que Cl, Br ou I et R₁₁ étant tel que défini précédemment ; et déprotection des groupes hydroxy protégés, soit
h) déprotection des groupes hydroxy protégés, et, éventuellement alkylation des groupes amido par un réactif de formule Z - R₁₁, R₁₁ étant tel que défini précédemment.

La réaction de l'étape a) a lieu de préférence dans un solvant approprié tel que le xylène, le nitrobenzène, le nitrotoluène, le DMF ou la pyridine, en présence d'un catalyseur métallique tel que le cuivre selon la méthode d'ULLMAN (E. FANTA, Chem. Rev. 64, 613, 1964).

La réaction de l'étape c) est une réduction catalytique par l'hydrogène sur charbon palladié ou sur nickel de Ranney ou une réduction chimique.

La réaction d'iodation de l'étape d) a lieu dans des conditions habituelles, telles que par ICl aqueux ou I₂ en présence de KI/éthylamine à des températures comprises entre 0°C et 100°C.

Les réactions d'alkylation des étapes f) et g) sont réalisées dans les conditions classiques, en présence d'une base forte.

Les composés de formule II dans laquelle R₂, R₄, R₅, R₆, R₈ et R₁₀ représentent un groupe 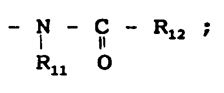
R₁₁ et R₁₂ étant tels que définis précédemment, peuvent en outre être obtenus par un procédé consistant en les étapes suivantes :
a1) couplage d'un dérivé benzénique de formule 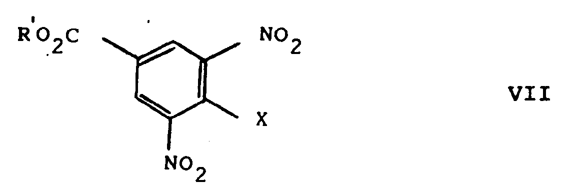 R′ représentant un groupe méthyle ou éthyle, et X étant choisi parmi un atome de chlore, de brome et d'iode suivant la réaction d'ULLMAN, tel que décrit ci-dessus pour obtenir un composé de formule VIII, 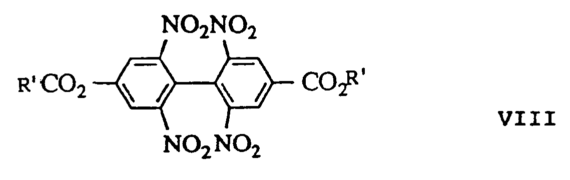 dans laquelle R′ est tel que défini précédemment ;
b1) après saponification des esters, puis hydrolyse,réarrangement selon la réaction de SCHMIDT pour obtenir une diamine de formule IX 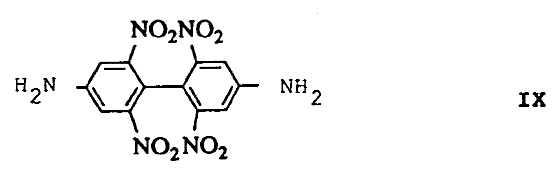
c1) acylation des groupements amino par un chlorure d'acide de formule R′₁₂ - COCl, R′₁₂ correspondant à R₁₂ tel que défini précédemment dont les groupes - OH sont protégés ou non.
d1) réduction catalytique ou réduction chimique des groupements nitro en amino tel que décrit précédemment à l'étape c).
e1) iodation des groupes amino aromatique comme décrit précédemment à l'étape d).
f1) acylation par un chlorure d'acide de formule R′₁₂ - COCl, comme décrit précédemment en f), R′₁₂ étant tel que défini précédemment.
g1) éventuellement déprotection, et
h1) éventuellement alkylation tel que décrit précédemment en g).

Les composés de formule II peuvent également être obtenus par réarrangement d'HOFMANN en série iodée, selon la méthode décrite dans FR-A-84 15 494.

Les composés de formule II dans lesquels R₂, R₄, R₅, R₆ R₈ et R₁₀ représentent le groupe 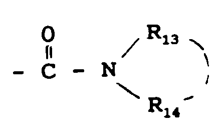
peuvent être obtenus en outre par les procédés classiques selon lesquels les groupes NH₂ aromatiques obtenus en c) sont convertis en groupes CO₂H, par exemple par la réaction de SANDMEYER en série iodée, comme décrit dans le brevet EP-A-32387.

Les composés de formule III dans laquelle R₁, R₃, R₇ et R₉ représentent un groupe 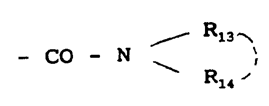
R₁₃ et R₁₄ étant tels que définis précédemment peuvent être préparés par le procédé suivant :
a2) diazotation d'un composé de formule 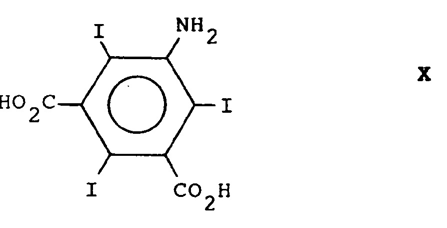 pour obtenir un composé de formule XI 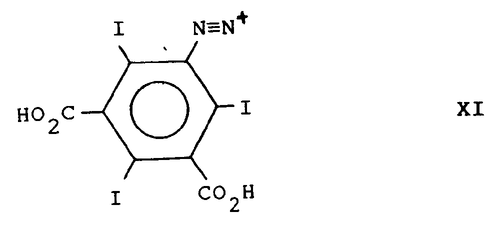
b2) couplage du composé de formule XI pour obtenir un composé de formule XII 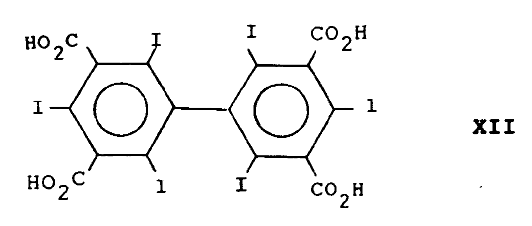
c2) transformation du composé de formule XII en son chlorure d'acide correspondant, et
d2) amidification du composé obtenu avec une amine de formule 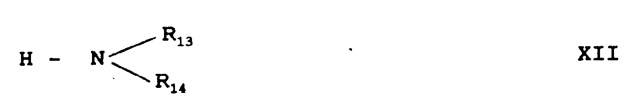 pour obtenir un composé de formule III dans laquelle R₁, R₃, R₇, et R₉ représentent le groupe :

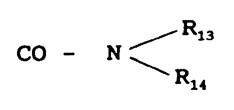

Les composés de formule III dans laquelle R₁ et R₇ représentent 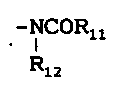
et R₃ et R₉ représentent 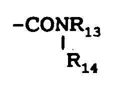
peuvent en outre être obtenus par un procédé consistant en les étapes suivantes :
- couplage des dérivés benzéniques de formule IV et V tel que décrit ci-dessus de façon à obtenir un composé de formule VI bis : 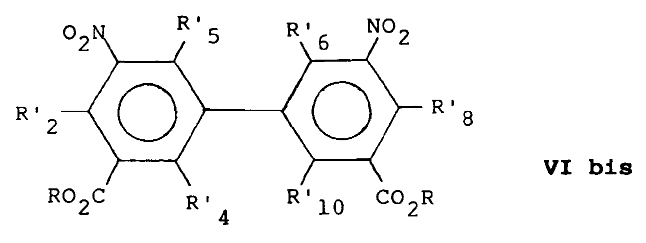
- réduction catalytique des groupes nitro en groupes amino tel que décrit précédemment,
- iodation telle que décrite précédemment,
- chloruration des groupes -CO₂R par un réactif tel que SOCl₂ ou (COCl)₂ dans les conditions classiques,
- acylation des groupes amino par un chlorure d'acide R′₁₂-COCl tel que défini précédemment,
- amidificalion des groupes -COCl par une amine 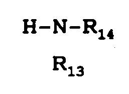 telle que définie précédemment,
- éventuellement déprotection des groupes hydroxy protégés,
- évntuellement alkylation des groupes amido par un réactif de formule Z-R₁₁ tel que défini précédemment.

Les amines de formule générale XII sont pour la plupart d'entre elles connues et disponibles dans le commerce ou peuvent être préparées de la manière suivante.

L'amine-alcool de formule : 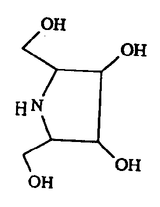
est préparé comme décrit dans Tetrahedron Letters, 31, 6777 (1990), J. Org. Chem., 50, 891 (1985) ou J. Chem. Soc. Chem. Commun. 262 (1987).

L'amine alcool de formule XII dans laquelle R₁₃ représente H et R₁₄ représente 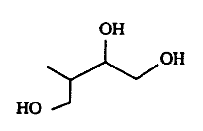
est préparée comme décrit dans US-4 341 756 et US-4 439 613.

L'amine alcool de formule XII dans laquelle R₁₃ représente -CH₃ et R₁₄ représente 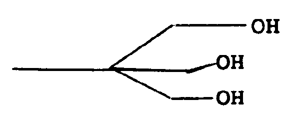
est préparée comme décrit dans Zh. Org. Khim, 22 (2), 298, 1986.

L'amine-alcool de formule XII dans laquelle R₁₃ représente -CH₂CH₂OH et R₁₄ représente 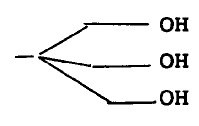
est commercialisée par Eastman Kodak.

L'amine-alcool de formule XII dans laquelle R₁₃ représente 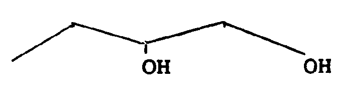
et R₁₄ représente 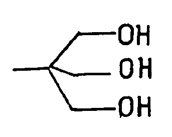
est préparée comme décrit dans J. Am. Chem. Soc. 66, 881, 1944.

L'amine-alcool de formule XII dans laquelle R₁₃ représente H, et R₁₄ représente 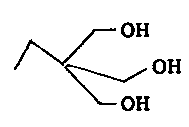
est préparée comme décrit dans Propellants, Explos., Pyrotech. 16(1), 40-42, 1991.

Les amines-alcools de formule générale : 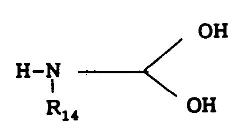
sont préparées de la manière suivante :
- lorsque R₁₄ représente -CH₃, comme décrit dans EP-25083;
- lorsque R₁₄ représente -CH₂CH₂OH, comme décrit dans EP-25083 et J. Med. Chem. 10 (3), 511, 1967;
- lorsque R₁₄ représente 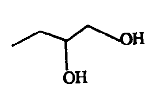 comme décrit dans J. Med. Chem. 10 (3), 511, 1967 et EP-25083.

On décrira ci-après la préparation d'autres amines-alcool de formule XII :

### Préparation de l'amine-alcool n° 1

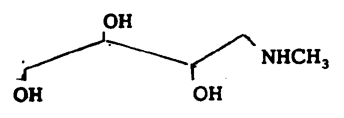

a) Préparation du composé de formule 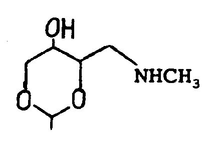
   2 g (13,7 mmoles) d'éthylidène-2,4 D-érythrose obtenu selon le procédé décrit dans J. Am. Chem. Soc. 2301, 1960 Barker R. et al. sont dissous dans 10 cm³ d'eau à 30°C. On ajoute goutte à goutte à 0°C 10 cm³ d'une solution aqueuse de méthylamine (40%). Après retour à la température ambiante, l'agitation est poursuivie pendant 2 h. La solution est ensuite réduite à température ambiante en présence de palladium sur charbon. Le catalyseur est ensuite filtré et le filtrat concentré à sec. Après concrétisation dans l'éther éthylique, 1,7 g de produit du titre sont obtenus, soit un rendement de 77%.
   CCM (dioxanne/H₂O/NH₃:8/3/2) Rf : 0,74
   CCM (CH₂Cl₂/MeOH 8/2) Rf : 0,17.
   RMN¹³C (DMSO) (δ, ppm) 200 MHz 98,2 (C-CH₃); 80,3 (CH-O); 70,5 (CH₂-0); 63,4 (CHOH); 53,1 (CH₂-N); 36,5 (NH-CH₃); 20,7 (C-CH₃).
b) Préparation du composé de formule : 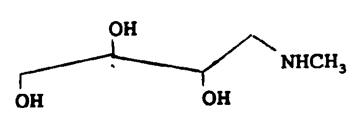
   1,5 g (9,3 mmoles) du produit obtenu en a) sont dissous dans 20 cm³ d'HCl 2N. La solution est agitée à 50°C pendant 5 h. Après concentration, et purification par passage sur résine H⁺, la solution est évaporée à sec. Le résidu est repris dans l'éther éthylique. Après filtration et séchage on obtient 0,8g du produit du titre (Rendement : 64%).
   CCM (dioxanne/H₂O/NH₃:8/3/2) Rf : 0,18
   RMN¹³C (DMSO) (δ,ppm) 200 MHz 74,5 (CH-CH₂OH); 69,6 (CHOHCH₂); 63,3 (CH₂OH); 54,7 (CH₂); 36,12 (NHCH₃) SM (DCI/NH₃) m/z; 153 (M+N⁺H₄); 136 (M+H⁺) pic de base

### Préparation de l'amine alcool n°2

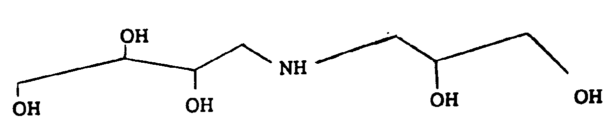

a) Préparation du composé de formule : 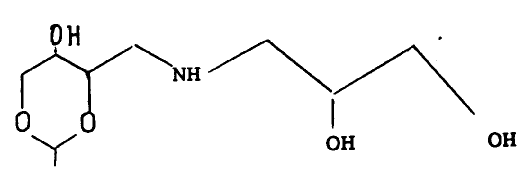
   Le composé est préparé selon la méthode décrite précédemment.
   L'amino-réduction de l'éthylidène-2,4-D-érythrose (6g, 41 mmoles) est effectuée en présence d'aminopropanediol (1,2 équiv.) dans l'éthanol (40 cm³).
   Après chromatographie sur colonne de silice, le produit du titre est obtenu avec un rendement de 73%.
   CCM (dioxanne/H₂O/NH₃:8/3/2) Rf : 0,73
   RMN¹³C (DMSO)(δ,ppm)(200 MHz) (98,C-CH₃); (80,2-80,5, CH-O); (70,2-70,4 CH₂-O); (70,3,CHOH); (64,5-64,6,CH₂₋OH); (62,2-63,1,CHOH); (52,9-53,CH₂); (50,8-51, CH₂); (20,5, CH₃).
b) Préparation du composé de formule : 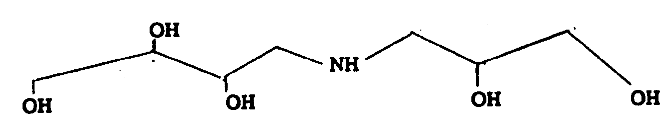
   6g ( 29,8 mmoles ) du produit obtenu à l'étape précédente sont déprotégés par traitement avec HCl 5N (50cm³). Le milieu réactionnel est agité pendant 4h à 50°C. Après évaporation, le résidu obtenu est purifié sur résine H⁺. Après concentration et concrétisation dans l'éther éthylique, 2,6 g du produit du titre sont obtenus (Rdt 54,7%)
   CCM (dioxanne/H₂O/NH₃: 8/3/2) Rf : 0,39
   RMN¹³C (DMSO) (δ, ppm)
   74,3 (CH-CH₂OH chaîne butanetriol ); 70, 3 ( CH-CH₂)x2; 64,5-64,6 (CH₂OH chaîne butanetriol); 63,3 (CH₂OH); 52,8 (CH₂N)X2
   SM (DCI/NH₃) m/z
   196 (M+H⁺)pic de base; 178 ( M+H⁺-H₂O);
   160 (M+H⁺-2H₂O) 136, 122, 109, 92.

### Préparation de l'amine alcool n° 3

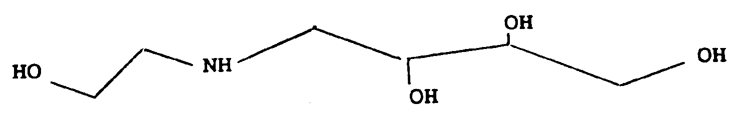

De même que la méthylamine (pour la préparation de l'amine-alcool n° 1) et l'aminopropanediol (pour la préparation de l'amine-alcool n° 2) l'éthanolamine, dans les mêmes conditions d'amino-réduction conduit, en présence d'éthylidène-2,4 D-erythrose au produit du titre.
a) Caractéristiques du composé de formule 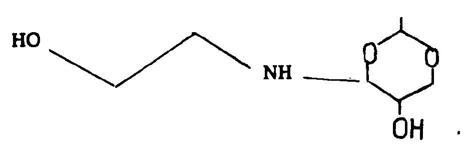 CCM( CH₂Cl₂/MeOH/NH₃ : 8/2/1 ) Rf : 0, 56
   RMN¹³C (DMSO) (δ,ppm) 97,9 (C-CH₃); 80,5 (CH-O); 70,2 (CH₂OH); 62,9 (CHOH); 60,2 (CH₂-O); 51,6(CH₂-N); 50,7 (CH₂-N); 20,4 (CH₃).
b) Caractéristiques du composé de formule 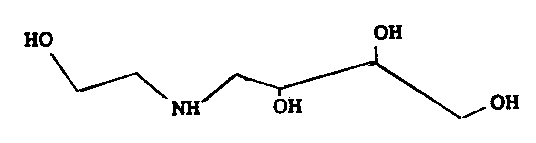 CCM(CH₂Cl₂/MeOH/NH₂ 55/30/10) Rf : 0,25
   CCM(dioxanne/H₂O/NH₃:8/3/2) Rf : 0,48
   RMN¹³C (DMSO) (δ,ppm)
   74,5 (CHOHCH₂OH); 70,2 (CHOH-CH₂); 63,5 (CHOHCH₂OH); 60,4 (CH₂-CH₂OH); 52,5(CH₂-CHOH); 51,8 (CH₂CH₂OH).
   En reprenant les modes opératoires décrits précédemment et en utilisant le sérinol avec l'éthylidène-2,4-D érythrose, on prépare de la même façon l'amine-alcool de formule :

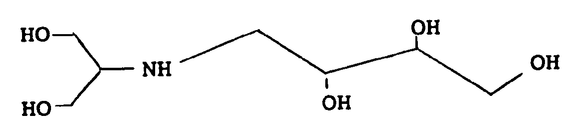

### Préparation de l'amine-alcool n° 5

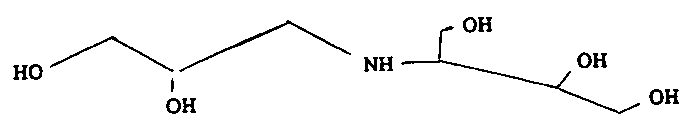

3g (18 mmoles) du époxy-2,3 butanediol-1,4 préparé selon la méthode décrite dans J. Med. Chem. 1976, vol.19, N° 1, 153-158 sont dissous dans 10cm³ de méthanol. On ajoute goutte à goutte à température ambiante 0,9 équiv. d'aminopropanediol dans 10 cm³ de méthanol. Le milieu réactionnel est porté à 45-50°C pendant 48 h. après évaporation, le produit brut est purifié sur résine H⁺ et est concentré à sec. Après reprise à l'éther et séchage, 4g du produit du titre sont obtenus (Rdt 72,7%).
CCM(dioxanne/H₂O/NH₃:8/3/2) Rf : 0,58
(CH₂Cl₂/MeOH/NH₃: 6/3/1) Rf : 0,55
RMN¹³C (DMSO) (δ, ppm)
71-71,2 (CHOH); 64,6(-NH-CH-CH₂OH);
63,5 (CH₂OH chaîne propanediol); 61,4 (-CH-);
61 (CH₂OH ); 51,3 (CH₂-N)

L'ouverture de l'époxyde décrit précédemment peut s'effectuer également par la méthylamine, l'éthanolamine et le sérinol de façon à obtenir respectivement les composés :

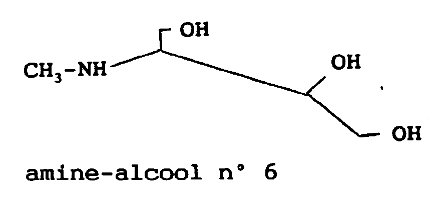

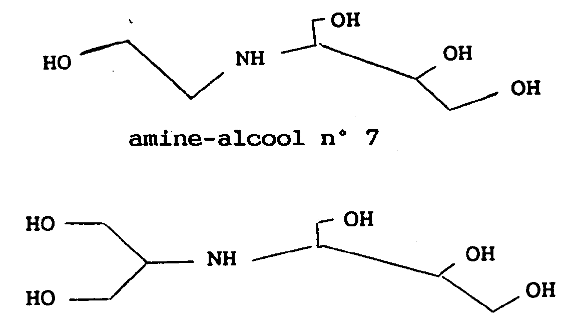

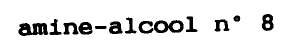

### Préparation du composé de formule :

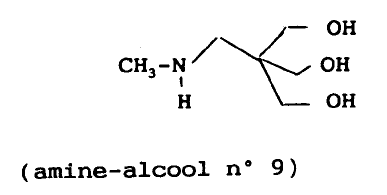

18,1 g (0,1 mole) de 3-bromométhyl-3-hydroxy-méthyloxétane préparé selon la méthode décrite dans Propellants, Explos., Pyrotech., 16(1) 40-42, 1991 sont agités dans 20 ml de méthanol et 76 ml (1 mole) de méthylamine aqueuse à 40% à 50°C pendant 24 h. Le mélange est évaporé à sec et le résidu est dissous dans 100 ml d'acide sulfurique 0,1 N.

La solution est portée au reflux pendant 12 h puis traitée sur résine. Le produit du titre est obtenu par évaporation de l'éluant.

### Préparation du composé de formule :

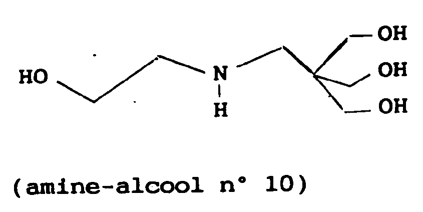

18,1 g (1 mole) de 3-bromométhyl-3-hydroxyméthyloxétane obtenu tel que décrit précédemment sont agités dans 20 ml de méthanol et 60,5 ml (0,1 mole) d'éthanolamine à 50°C pendant 24 h. Le mélange est évaporé à sec et le résidu est dissous dans 100 ml d'acide sulfurique 0,1 N. La solution est portée à reflux pendant 12 h puis traitée sur résine. Le composé du titre est obtenu par évaporation de l'éluant.

Il va de soi que l'invention englobe non seulement les composés de formule I sous forme de mélange racémique mais également les stéréoisomères tels que énantiomères, diastéréoisomères, atropoisomères, isomères SYN-ANTI, ENDO-EXO, E-Z, liés à la présence d'atomes de carbone asymétriques et/ou aux empêchements de rotation dûes à l'encombrement stérique apporté par les atomes d'iode et/ou par les substituants R₁ à R₁₀ des composés de formule I.

La présente invention a également pour objet des produits de contraste qui comprennent au moins un composé de formule I.

Ces produits de contraste sont utilisés chez l'homme et les animaux à des fins radiologiques.

La forme pharmaceutique préférée des produits de contraste selon l'invention est constituée par des solutions aqueuses des composés. Selon une forme de réalisation de l'invention, les composés sont encapsulés à l'intérieur de liposomes.

Les solutions aqueuses contiennent généralement un total de 5 à 100 g de composés pour 100 ml et la quantité injectable de telles solutions peut varier généralement de 1 à 1000 ml. Les solutions peuvent contenir également des additifs tels qu'un sel de sodium notamment le citrate de sodium, l'héparine et l'EDTA calcique de sodium.

Ces compositions peuvent être administrées par toutes les voies classiquement utilisées pour les produits de contraste non ioniques iodés. Ainsi elles peuvent être administrées par voie entérale ou parentérale (voie intraveineuse, intraartérielle, opacification des cavités) et en particulier dans l'espace sous-arachnoïdien.

On donnera ci-après des exemples de préparation de composés selon l'invention.

### Exemple 1

Préparation du 4,4′-di-(hydroxyacétylamino)-2,2′,6,6′-tétrakis-(2,3-dihydroxypropylcarbamoyl) 3,3′,5,5′-tétraiodo biphényle (composé n°1).
1) Préparation de l'acide 2-bromo isophtalique
   125 g (0,676 mole) de 2-bromometaxylène sont ajoutés rapidement à une solution de 440 g (4,1 x 0,676 mole) de permanganate de potassium dans 2,5 l d'eau. Après un reflux de 48 h sous agitation et retour à la température ambiante, la masse réactionnelle est filtrée sur Cellite; après lavage du gateau à l'eau chaude, le filtrat est évaporé jusqu'à réduction du volume à 1,5 l. La solution une fois refroidie à 0° est acidifiée par 100 ml d'HCl 10N jusqu'à pH = 1. Le précipité est filtré, lavé à l'eau puis séché pendant 12 h à 70°. Le produit est obtenu avec un rendement de 70,6% (117 g).
   PF = 222°
   Chromatographie en couches minces (CCM) (isopropanol/acétate d'éthyle (AcOEt)/NH₄OH : 25/25/30): R_{f} = 0,25 CCM (toluène/méthyl-éthyl-cétone/HCOOH 60/25/5):
   R_{f} = 0,5
   Dosage de l'acidité ((t-butyl)₄-N-OH) 103,1% (1ère acidité) 100,6% (2ème acidité)
2) Préparation de l'acide 2-bromo 5-nitro isophtalique
   Sur une solution de 117 g (0,477 mole) du produit précédent dans 892 ml d'H₂SO₄ à 98 %, un mélange de HNO₃ fumant (167 ml) et H₂SO₄ concentré (264 ml) est additionné goutte-à-goutte en maintenant la température inférieure à 15°. La masse réactionnelle est agitée pendant 12 h à température ambiante. Le précipité est filtré, lavé par 3 x 200 ml d'H₂O puis séché à 70° pendant 12 h
   Rdt 93,4%
   PF = 214°
   CCM (isopropanol 25 / AcOEt 25/NH₄OH/30) : R_{f} = 0,45
   RMN ¹H (DMSO) 8,5 ( s 2H arom) 13 (s, COOH 2H)
3) Préparation de l'ester diméthylique de l'acide 2-bromo 5-nitro isophtalique
   880 ml de méthanol contenant 129 g (0,445 mole ) du produit précédent et 90 ml d'H₂SO₄ à 98% sont chauffés à reflux durant 24 h. Le précipité obtenu est filtré, lavé avec 120 ml de méthanol, puis 2 x 120 ml d'H₂0. Après séchage à 65°, on obtient le produit avec un rendement de 74%.
   PF = 134°C
   CCM (toluène 60/méthyl-éthyl-cétone 35/ HCOOH 25) : R_{f}=0,8
   IR 1730 (COOCH₃) 1350-1560 (NO₂)
   RMN ¹H DMSO 4,0 (s COOCH₃ 6H) 8,5 (s 2H arom)
4) Préparation du 4,4′-dinitro-2,2,6,6′-tétra(méthoxycarbonyl) biphényle
   Dans une solution de 100 g (0,314 mole) du produit précédent dans 420 ml de p-xylène préalablement chauffée à 80°, on ajoute 36,0 g (0,566 mole) de cuivre par portions. Après 5 h de reflux (à 140°), 18 g (0,283 mole) de cuivre sont rajoutés dans le milieu réactionnel. La même opération est renouvelée après 12 h de reflux. Le milieu est ensuite filtré sur Cellite après une nouvelle période de 12 h de reflux. Pour éliminer le CuBr qui précipite avec le bon produit, on effectue une extraction par CH₂Cl₂; le filtrat évaporé à sec est repris dans l'éther filtré puis séché à 80°C pour donner le composé 5 avec un rendement de 70%
   PF = 226°
   CCM (CH₂Cl₂) : R_{f} = 0,1
   RMN ¹H(CDCl₃) 3,6 (s, COOCH₃ 12 H) 9,0 (s 4H arom)
5) Préparation du 4,4′-dinitro-2,2′,6,6′-tétrakis-(2,3-dihydroxypropyl carbamoyl) biphényle
   A 89,0 g de 2,3-aminopropanediol dilués dans 450 ml de MeOH chauffé à 40°, on ajoute par portions 49 g (0,103 mole) du produit précédent. Après une agitation vigoureuse pendant 1/2 h à 40°, on additionne une quantité de MeONa suffisante pour obtenir une solution homogène. Après 12 h à température ambiante, le milieu est évaporé, puis repris dans 1 l d'H₂O et passé sur des colonnes de résines échangeuses d'ions H⁺ et OH^{-.} Après évaporation à sec, le produit est cristallisé dans l'isopropanol, filtré, lavé par 2 x 250 ml d'éther puis séché à 70° Rdt 88,3%
   CCM (Dioxanne 9 / H₂O 1) R_{f} = 0,70
   RMN ¹H (DMSO) 2,8-3,2 (CH aliphatiques 20 H) 3,9-4,5 (OH 8 H échangeables 8,2 (s 4H aromatiques) 8,3-8,6 (CONH 4H échangeables)
6) Préparation du 4,4′-diamino 2,2′,6,6′-tétrakis-(2,3-dihydroxypropyl carbamoyl) biphényle
   39 g (0,0550 mole) du produit précédent en solution dans 675 ml de H₂O en présence de 13,4 g de charbon palladié à 10% sont agités sous une pression d'H₂ d'environ 3.10⁵Pa à température ambiante pendant 6 heures.
   Après filtration du catalyseur, évaporation de l'eau et reprise dans l'éther éthylique, la suspension obtenue est filtrée, lavée par 2 x 100 ml d'éther et le produit est séché sous vide à température ambiante.
   Rdt = 96,3 %
   CCM (MeOH 8/CH₂Cl 2) R_{f} = 0,5
   Dosage d'acidité (HClO₄) 93,5% par fonction NH₂
   RMN ¹H (DMSO) 2,7-4 (massif 20 H aliphatiques)
   4,1-6 (massif 8H,OH; 4H NH₂ échangeables)
   6,7 (s 4H arom) 8,1-8,6 (m CONH 4 H échangeables)
7) Préparation 4,4′-diamino 2,2′,6,6′-tétrakis-(2,3-dihydroxypropyl carbamoyl) 3,3′,5,5′-tétraiodo biphényle
   90,5 ml d'ICl à 70% sont ajoutés goutte à goutte sur une solution de 54,5 g (0,0826 mole) du produit précédent dans 545 ml d'eau. Après chauffage à 80°C durant 8 h puis agitation pendant 24 h à température ambiante, le précipité obtenu est filtré, lavé avec 20 ml de bisulfite puis 40 ml d'H₂O.
   Un premier jet est obtenu avec 23,4 % de rendement et une pureté HPLC de 88%
   La récupération du deuxième jet s'effectue par neutralisation des eaux mères à la soude 5N jusqu'à pH5. Le précipité d'I₂ est filtré, la solution évaporée à sec et reprise à l'éthanol. La suspension obtenue est filtrée, lavée par 2 x 200 ml d'éthanol puis 200 ml d'éther. Après séchage à 70°, on obtient 103,5 g de produit contenant 50,7% de NaCl en masse. Après purification par HPLC préparative sur SiO₂ RP 18, on obtient le produit avec une pureté en iode de 98,35%, Rdt : 75 %
   CCM (dioxanne 9/H₂O 1/NH₄OH 1) : R_{f} = 0,45
   RMN ¹H (DMSO d⁶) 2,7-3,7 (m. 20 H aliphatiques)
   4-6 (massifs 8H OH; 4H NH2 échangeables)
   8-8,8 (m. CONH 4H échangeables)
8) Préparation du 4,4′-di-(acétoxyacétylamino) 2,2′,6,6′-tétrakis-(2,3-di-acétoacétoxypropyl carbamoyl) 3,3′,5,5′-tétraiodo biphényle
   Sur une solution de 40,6 g (0,0351 mole) du produit précédent dans 320 ml de DMAC anhydre sont ajoutés 71,9 g (15 x 0,0351 mole) de chlorure d'acide glycolique acétylé. La température du milieu réactionnel est montée à 58° puis la solution est agitée vigoureusement pendant 12 h à 50°C. Le résidu est repris par 500 ml de CH₂Cl₂ et lavé à l'eau. La phase organique est séchée sur Na₂SO₄, filtrée et évaporée au 3/4 pour un relargage à l'éther. La suspension obtenue est filtrée, lavée par 2 x 200 ml d'éther.
   Rdt : 100%; test d'amine négatif.
   RMN ¹H DMSO 2,0-2,3 (s OCOCH₃ 30 H) 4,0-5,5 (m 40 H aliphatiques) 8,3-9,0 (m CONH, 4H échangeables) 10,2-10,4 (m NHCO 2 H échangeables)
9) Préparation du 4,4′-di-(hydroxy-acétylamino) 2,2′,6,6′-tétrakis-(2,3-dihydroxypropyl carbamoyl) 3,3′,5,5′-tétraiodo biphényle (composé n°1)
   75,7 g (0,0351 mole) du produit précédent sont dissous dans 1,1 l de NaOH. Après 1 h 30 d'agitation à température ambiante, le milieu réactionnel est dilué par 700 ml d'H₂O pour un passage sur résines échangeuses et d'ions H⁺ et OH^{-.} La solution obtenue à pH = 7,2 est évaporée.
   On obtient avec un rendement de 60,2% le composé n° 1 CCM (dioxanne 9/H₂O 1/NH₄OH 2) : R_{f} = 0,4
   Pureté en iode 99,29%
   Pureté HPLC = 99,3% en 2 pics isomères (C8 hypersyl 5M.15 cm Tampon NaH₂PO₄/MeOH 92/8
   RMN ¹H (DMSO) 2,6-6 (m. complexe 24 H aliphatiques 10 H OH échangeables) 8-8,8 (m. CONH 4 H échangeables) 9,8-10,4 (m, NHCO, 2H échangeables).

### Exemple 2

Préparation du 4,4′-di-(hydroxy-acétylamino) 2,2′,6,6′-tétra-(2-hydroxyéthyl carbamoyl) 3,3′,5,5′-tétraiodo biphényle (composé n° 2)
1) Préparation du 4,4′-dinitro 2,2′,6,6′-tétra- (2-hydroxyéthyl carbamoyl) biphényle
   On prépare le 4,4'-dinitro 2,2′,6,6′-tétraméthoxycarbonyl biphényle comme indiqué dans l'exemple précédent en 4.
   On ajoute ensuite 35 g du produit obtenu par portions dans une solution d'éthanolamine (42,6 g) dans 350 ml de MeOH à 40°. Après une agitation vigoureuse pendant 1/2 h à 40°, on ajoute du MeONa en poudre jusqu'à ce qu'on obtienne une solution homogène.
   La masse réactionnelle est agitée 12 h à 40°, évaporée, reprise dans 700 ml d'eau puis passée sur résines échangeuses d'ions H⁺ et OH^{-.} Après évaporation à sec, le produit est cristallisé dans l'acétone. On filtre, lave à l'éther et sèche à 70°. Le produit est obtenu avec un rendement de 78,2%.
   CCM (CH₂Cl₂ 8/MeOH 2) R_{f} = 0,75
   RMN ¹H (DMSO) 2,9-3,5 (m. 16 H aliphatiques) 3,6-4,8 (m OH 4 H échangeables) 8,1-8,3 (s 4H arom) 8,4-8,8 (m CONH 4 H échangeables avec D₂0)
2) Préparation du 4,4′-diamino 2,2′,6,6′- tétra-(2-hydroxyéthyl carbamoyl) biphényle
   On ajoute 34 g du produit précédent en solution dans 900 ml d'H₂O et en présence de 10,5 g de charbon palladié à 10%, on soumet à une pression de 3.10⁻⁵ Pa d'H₂ sous agitation à température ambiante pendant 6 h. Après filtration du catalyseur, l'eau est évaporée jusqu'à un volume final de 350 ml qui est utilisé tel quel pour la réaction suivante.
   CCM (MeOH 8/CH₂Cl₂) : R_{f} = 0,55 (produit non isolé)
3) Préparation du 4,4′-diamino 2,2′,6,6′-tétra-(2-hydroxyéthyl carbamoyl) 3,3′,5,5′-tétraiodo biphényle
   On ajoute goutte à goutte à la solution du produit obtenu à l'étape précédente 50 ml d'ICl à 70%. Après chauffage du milieu réactionnel à 80° durant 3 h, on filtre la suspension obtenue et on lave au bisulfite puis abondamment à l'eau. Après séchage à l'éther sous vide à 60°pendant 24 h, le produit désiré est obtenu avec un rendement de 82,3%
   CCM (CHCl₂ 8/MeOH 2) R_{f} = 0,7
   RMN ¹H (DMSO) 2,9-3,5 (m 16 H aliphatiques) 3,8-5 (m 4 H, OH et 4H, NH₂ échangeables avec D₂O) 8,2-8,7 (m 4H, CONH échangeables)
4) Préparation du 4,4′-di-(acétoxyacétylamino) 2,2′,6,6′-tétra-(acétoxy-acétoxy carbamoyl) 3,3′,5,5′-tétraiodo biphényle
   101,2 g (12x 00,0618 moles) de chlorure d'acide glycolique acétyle sont ajoutés rapidement sur une solution de 64 g (0,0618 mole) du produit précédent dans 1,1 l de DMAC anhydre. Après chauffage pendant 12 h à 60°, le milieu est évaporé, le résidu extrait par 700 ml de CH₂Cl₂ et lavé par 250 ml d'H₂0. Après séchage de la phase organique sur MgSO₄ et filtration, celle-ci est traitée par du charbon actif. Après évaporation, le résidu est cristallisé dans l'éther. Après séchage sous vide, le produit désiré est obtenu avec 88,5% de rendement.
   CCM (CH₂Cl₂ 9/MeOH 1) : R_{f} = 0,95
   Pureté en iode 99,4%
   RMN ¹H (DMSO) 1,8-2,2 (d OCOCH₃ 18 H) 2,8-3,5 (m CONH CH₂ 8 H) 3,6-4,2 (m CH-CH₂0CO 8 H) 4,3-4,7 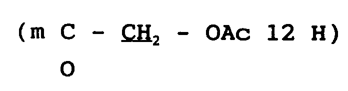 8,2-8,7 (m CONH, 4H échangeables) 9,1-10,2 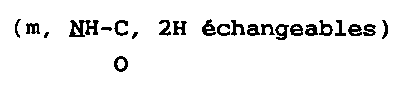
5) Préparation du 4,4′- di-(hydroxy-acétylamino) 2,2′,6,6′-tétra-(2-hydroxyéthyl carbamoyl) 3,3′,5,5′-tétraiodo biphényle
   89 g (0,0544 moles) du produit précédent sont agités en présence de 740 ml de NaOH N à température ambiante pendant 2 h. La solution est ensuite diluée jusqu'à un volume de 3,8 l puis passée sur résines échangeuses d'ions H⁺ et OH^{-.} Les phases aqueuses sont ensuite évaporées à sec puis cristallisées dans l'acétone. Après lavage à l'éther et séchage à l'étuve à 50° on obtient le composé n° 2 avec un rendement de 78,1 %.
   CCM (CHCl₃ 55/MeOH 30/NH₄OH 10) : R_{f} = 0,45
   Pureté en iode : 98,30%
   Teneur en H₂O : 2,15%
   RMN ¹H (DMSO d⁶) 2,6-6 (m 28 H aliphatiques 6 OH échangeables) 8,2-8,6 (m CONH 4H échangeables) 9,9-10,1 (m NHCO 2H échangeables avec D₂O)

### Exemple n°3

Préparation du 4,4′-bis-[N-(2,3-dihydroxy) propyl-acétylamino] 2,2′,6,6′-tétra-(2-hydroxyéthyl carbamoyl) 3,3′,5,5′-tétraiodo-biphényle (composé n° 3)
1) Préparation du 4,4′-diacétylamino 2,2′,6,6′-tétra-(2-acétoxyéthyl carbamoyl) 3,3′,5,5′-tétraiodo biphényle
   60 g du produit obtenu à l'exemple précédent en 3 sont mis en suspension dans 300 ml d'acide acétique et 60 ml d'anhydride acétique puis chauffés à 60°. 2,5 ml d'H₂SO₄ sont versés goutte à goutte en maintenant la température à 70°-80° pendant 1/2 heure. La masse réactionnelle est ensuite évaporée à sec et le résidu repris dans l'éther, filtré, puis séché. Le produit désiré est obtenu avec un rendement de 76%.
   La pureté en iode est de 102%
   CCM (CH₂Cl₂ 90/MeOH 10) : R_{f} = 0,55
   RMN ¹H (DMSO) 2 (s. COCH₃ 18 H) 3,3 et 3,9 (d CH₂-CH₂ 16 H) 8,5 (m CONH 4 H échangeables) 10 (m, NH - CO, 2 H échangeables)
2) Préparation du 4,4′-diacétylamino 2,2′,6,6′-tétra-(2-hydroxy-éthyl carbamoyl)-3,3′,5,5′-tétraiodo biphényle
   On ajoute 99 g du produit précédent dans 1,3 l de méthanol en présence de 21,7 g de K₂CO₃ puis on agite pendant 3 h à température ambiante. Le précipité formé est essoré, lavé avec une quantité minimale de méthanol et par l'éther
   Rdt 100%
   RMN ¹H (DMSO d⁶) 1,8 (s. COCH₃ 6H) 3,2 et 3,7 (2m. CH₂-CH₂, 20 H) 8,5 (m, CONH, 4H échangeables) 10 (m, NHCO, 2H échangeables)
3) Préparation du 4,4′-bis [N-(2,3-dihydroxypropyl)-acetylamino] 2,2′,6,6′-tétra-(2-hydroxyéthyl carbamoyl) 3,3′,5,5′-tétraiodobiphényle (composé n° 3)
   35 g du produit précédent sont mis en suspension dans 200 ml d'éthylèneglycol. On additionne goutte à goutte 0,186 mole de MeONa en solution dans le méthanol et on chauffe le mélange réactionnel pendant 1 h 30 min à 50°C. On ajoute 18,2 ml de chloropropanediol à 10°. Le mélange réactionnel est chauffé pendant 44 h à 50°, en ajoutant toutes les 10 heures 0,062 mole de méthylate de Na en solution et 15 ml de chloropropanediol.
   Après évaporation du méthanol, la pâte résiduelle est reprise dans l'acétone. Après filtration des sels, l'éthylèneglycol est évaporé sous pression réduite. Le résidu obtenu est concrétisé dans un mélange acétone/isopropanol (70:30), puis filtré et lavé par l'éther.
   Le composé n° 3 obtenu brut est ensuite purifié sur colonne HPLC préparative ( SiO₂ C18 ) avec un rendement de 70%
   CCM ( CH₂Cl₂ 80/MeOH 20 ) : R_{f} = 0,15
   CCM ( Dioxanne 90/H₂O 10 ) : R_{f} = 0,7
   Pureté en iode : 98%
   Pureté HPLC 97%
   RMN ¹H DMSO 2 ( s, COCH₃ 6H ) 3 -3, 6 (m partiellement échangeables 26 H aliphatiques et 8 OH ) 8,4 (s, CONH, 4H échangeables )

### Exemple 4

Préparation du 4,4′-bis-(2,3-dihydroxypropyl carbamoyl) 2,2′,6,6′-tétra-(2-hydroxy-propionylamino) 3,3′,5,5′-tétraiodo biphényle (composé n° 4)
1) Préparation de l'acide 4-bromo 3,5-dinitro benzoïque
   Un mélange sulfonitrique composé de 346 ml d'H₂SO₄ et 346 ml d'HNO₃ fumant est additionné goutte à goutte sur 100 g (0,49 mole) d'acide p-bromobenzoïque en suspension dans 900 ml d'H₂SO₄ à 98%. Le mélange est chauffé pendant 8 h à 80°C puis refroidi et versé dans la glace. Le précipité formé est filtré, lavé par 3 x 200 ml d'H₂O puis mis en suspension dans 600 ml d'H₂O. Après avoir ramené le pH à 9-10 par NaOH 5N, le sel de sodium est filtré puis remis en suspension dans 1 l d'eau et acidifié par HCl 5N jusqu'à pH = 1. Le précipité est ensuite filtré, lavé à l'eau et séché.
   Rdt 79% (95 g), PF 188°C
   CCM (toluène 60/méthyléthylcétone 35/ac. formique 25 ): R_{f} = 0,85
   Pureté en brome 99%
   RMN ¹H (DMSO d⁶) 8,5 ppm (s, 2H aromatiques) 10,4 (s OH acide échangeable avec D₂O)
2) Préparation de l'ester méthylique de l'acide 4-bromo 3,5-dinitro benzoïque
   115 g (0,398 m) d'acide 4-bromo 3,5-dinitro benzoique sont dissous dans 840 ml de méthanol. Après addition de 57,5 ml d'H₂SO₄ à 98%, le mélange est porté au reflux pendant 3 heures. Après refroidissement, le produit précipite. La masse réactionnelle est filtrée, lavée par H₂O (3 x 300 ml) puis par 100 ml de méthanol.
   Rdt : 90%
   PF : 122-124° (124 dans la littérature)
   CCM (toluène/méthylethylcétone/Ac. formique : 60/35/25) : R_{f} = 0,95
   RMN ¹H (DMSO d⁶) 4,1 (s, COOCH₃, 3H) 8,75 (s. 2H arom.)
3) Préparation du 4,4′-diméthoxycarbonyl 2,2′,6,6′-tétranitro biphénile
   Sur une solution de 60 g (0,196 mole) du produit précédent dans 260 ml de paraxylène préchauffé à 80°, on verse 12,6 g de cuivre. Le milieu est ensuite chauffé à 160°C pendant 1 h 30 sous forte agitation. On ajoute 6,4 g de cuivre et on laisse à 160° pendant 1 heure. Le mélange est refroidi, puis filtré sur Celite. Après évaporation, le résidu est repris dans l'acétate d'éthyle.
   Rdt : 73%
   PF : 171°C
   CCM (toluène/méthyléthylcétone/Ac. formique : 60/35/25) : R_{f} = 0,9
   RHM ¹H (DMSO d⁶) 4,1 (s COOCH₃, 6H) 9 (s 4H arom.)
4) Préparation du 4,4′-bis-(2,3-dihydroxypropyl carbamoyl 2,2′,6,6′-tétranitro biphényle
   21 g (0,046 mole) du produit précédent sont dissous dans 240 ml de DMAC. Après avoir porté le mélange réactionnel à 60°, on ajoute goutte à goutte 17,5 g d'aminopropanediol dans 50 ml de DMAC. Après agitation pendant 1 h 30 min, on refroidit la masse réactionnelle puis on la verse dans 1,5 l d'eau. Le précipité est alors essoré, lavé par H₂O puis par l'éther.
   PF : 130°C
   CCM (toluène/méthyléthylcétone/Ac. formique : 60/35/25) : R_{f} = 0,15
   RMN ¹H (DMSO) 3,4-3,9 (m CH-CH₂ 12 H) 4,5-5 (m. OH échangeable 4H) 9,1 (s. 4H arom)
5) Préparation du 4,4′-bis-(2,3-dihydroxypropyl carbamoyl) 2,2′,6,6′-tétraamino biphényle
   On ajoute une suspension de 9 g du produit précédent dans un mélange H₂O/MeOH (150 ml/150 ml) en présence de 30 g de Nickel de Raney et on agite sous pression d'H₂ (3.10⁵Pa) durant 24 h à température ambiante. Après filtration sur Celite du milieu réactionnel, le filtrat est évaporé et le résidu cristallisé dans l'éthanol absolu.
   Rdt : 50%, PF = 250°
   CCM (dichlorométhane/méthanol : 80/20) : R_{f} = 0,05
6) Préparation du 4,4′-bis-(2,3-dihydroxypropyl carbamoyl) 2,2′,6,6′-tétraamino 3,3′,5,5′-hexaiodo biphényle
   Une solution de 20,4 g de I₂ (0,084 mole) et 13,3 g (0,0804 mole) de KI dans 20 ml d'H₂O, préparée extemporanément, est additionnée goutte à goutte sur une solution de 6 g du produit précédent dans 600 ml d'H₂0 et 60 ml d'éthylamine à 33%. A la fin de l'addition, le mélange est chauffé pendant 3 h à 80°. Après addition de 10 ml d'une solution de 6,8 g d'I₂ (0,0268 mole) et 4,4 g (0,0268 mole) de KI dans l'eau, on chauffe pendant 3 h à 80°, puis on laisse reposer une nuit à température ambiante. Le précipité est filtré, lavé à l'eau puis par du bisulfite et de l'acétone et séché.
   Rdt = 50%
   CCM (dichlorométhane/méthanol : 80/20) : R_{f} = 0,3 Pureté en iode : 97%
   RMN ¹H (DMSO d⁶) 3,1 (m complexe) 4,4 et 8 (massifs échangeables avec D₂0
7) Préparation du 4,4′-bis-[2,3-di-(2-acétoxy propionyloxy)-propyl-carbamoyl] 2,2′,6,6′-tétra-(2-acétoxypropionyl amino) 3,3′,5,5′-tétraiodo biphényle
   5g (0,033 mole) de chlorure d'acide lactique acétylé et 4,7 ml (0,033 mole) de triéthylamine sont additionnés simultanément dans une solution refroidie à 5° de 2,7 g (0,0024 mole) du produit précédent dans 15 ml de DMAC. Durant l'addition, la température est maintenue en dessous de 10°, puis le mélange est chauffé lentement jusqu'à 40° durant 8 h.
   On ajoute ensuite simultanément 1,08 g (0,0072 mole) de chlorure d'acide lactique acétylé et 1,01 ml (0,0072 mole) de triéthylamine à 10°. La masse réactionnelle est alors portée à 40° durant 12 h. Après filtration du chlorhydrate de triéthylamine formé et évaporation du DMAC, le résidu est versé dans l'eau. Les cristaux obtenus sont lavés par H₂0 puis l'éther.
   Rdt : 80%
   Pureté en iode 105%
   CCM (toluène 60/méthyl-éthyl-cétone 35/ acide formique 25) : R_{f} = 0,6 (3 tâches accolées)
   RMN ¹H DMSO d⁶ 1,3 (massif C-CH₃, 24 H) 2,1 (s.COCH₃ 24 H) 3,1-5,4 (massif partiellement échangeable 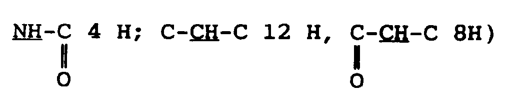
8) Préparation du 4,4′-bis-(2,3-dihydroxypropyl carbamoyl) 2,2′,6,6′-tétra (2-hydroxypropionyl amino)-3,3′,5,5′-tétraiodo biphényle (composé n° 4)
   On ajoute 31,5 g (0,0168 mole) du produit précédent dans une solution de 400 ml de méthanol en présence de 27,6 g de K₂CO₃ et on agite pendant 4 h à température ambiante. La solution est ensuite évaporée puis passée sur résine échangeuse d'ions H⁺. Le produit obtenu après évaporation est lavé par l'acétone.
   Rdt : 50%
   Pureté en iode 96;6%
   Teneur en eau 1,83%
   PF = 220°
   CCM (dichlorométhane/méthanol : 80/20 ) : R_{f} = 0,7
   CCM (butanol/eau/acide acétique : 50/25/11) : R_{f} = 0,35
   RMN ¹H (DMSO) 1,2(s.C-CH₃ 12 H) 3 à 6 (3 massifs complexes 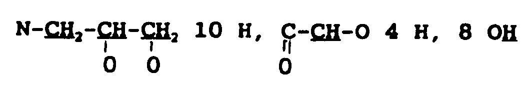 échangeables) 9 à 9,5 (1 massif échangeable avec D₂O, -NH-CO, CO-NH-, 5H).

### Exemple 5

Préparation du 2,2′,4,4′,6,6′-hexa-(2-hydroxypropionyl amino) 3,3′,5,5′-tétraiodo biphényle (composé n° 5)
1) Préparation du 4,4′-dicarboxy 2,2′,6,6′-tétranitro biphényle
   55,4 g ( 0, 123 mole ) du produit obtenu dans l'exemple précédent en 3 sont mis en suspension dans 600 ml d'H₂O contenant 10,6 g (0,27 mole) de NaOH. Le mélange est porté à 80° pendant 6 h puis refroidi. La phase aqueuse est lavée par 2 x 200 ml de dichlorométhane, puis acidifiée par HCl. Le précipité est lavé par H₂O (3 x 300 ml) puis séché.
   Rdt : 100%, PF > 300°
   CCM (toluène/méthyl-éthyl-cétone/Ac. formique : 60/35/25) : R_{f} = 0,75
   RMN ¹H DMSO 8,9 (s. 4H arom) 14 (m 2H COOH échangeables avec D₂0).
2) Préparation du 4,4′-diamino 2,2′,6,6′-tétranitro biphényle
   51 g (0,120 mole) du produit précédent sont dissous dans 320 ml d'oléum à 30%, puis dilués par 280 ml de 1,2-dichloroéthane. Le mélange est chauffé à 45°. On ajoute alors 23 g de NaN₃ par portions, en maintenant une température inférieure à 50°C. Après la fin de l'addition, la masse réactionnelle est portée progressivement à 90°C puis agitée pendant 3 heures. On ajoute alors 9 g de NaN₃ à une température inférieure à 50°. Le mélange est chauffé pendant 2 h à 90°C. Après retour à la température ambiante et décantation, la phase inférieure est versée lentement dans un mélange glace-eau. Le produit précipite et la suspension est agitée pendant 1/2 heure à 60°, puis laissée une nuit à température ambiante. Après filtation, lavage à l'eau, puis l'éther, on obtient le produit désiré avec un rendement de 90%.
   PF > 300°
   CCM (toluène 60/ méthyl-éthyl-cétone 35/ acide formique 25): R_{f} = 0,9
   RMN ¹H DMSO 5,8 (1s NH₂ 4H disparait avec D₂0) 7,5 (s, 4H arom)
3) Préparation du 4,4′-di-(2-acétoxypropionyl amino) 2,2′,6,6′-tétranitro biphényle
   59,5 g (0,163 moles) du produit précédent sont ajoutés par portions sur une solution de 54,3 g (0,358 mole) de chlorure d'acide lactique acétylé dans 300 ml de DMAC anhydre. La température est maintenue en dessous de 10°C durant l'addition, puis on laisse revenir à température ambiante et on agite durant 2 h. Après évaporation du DMAC, le résidu versé dans un mélange glace/eau permet d'obtenir le produit désiré sous forme de cristaux. Après lavage à l'eau puis au méthanol, le rendement est de 83%.
   PF = 264 °
   CCM ( toluène/méthyl-éthyl-cétone/Ac. formique : 60/35/25 ) : R_{f} = 0,7
   RMN ¹H DMSO d⁶ 1,5 (d C-CH₃ 6 H) 2,1 (s, COCH₃ 6H) 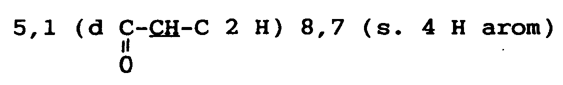 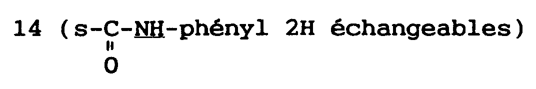
4) Préparation du 4,4′-di-(2-hydroxypropionyl amino) 2,2′,6,6′-tétraamino biphényle
   On ajoute 10 g (0,0167 mole) du produit précédent dans un mélange de MeOH (460 ml) et d'eau (160 ml) en présence de 40 g de Nickel de Raney et on agite sous pression d'H₂ (3.10⁵Pa) durant 24 h à température ambiante. Après filtration du catalyseur et évaporation du filtrat, le résidu est cristallisé dans l'éthanol. Après essorage, on obtient le produit final avec un rendement de 60%
   CCM (dichlorométhane/méthanol : 80/20) : R_{f} = 0,45
   RMN ¹H DMSO d⁶ 1,3-1,5(C-CH₃ 6H) 4,1 (s NH₂ 8H échangeables) 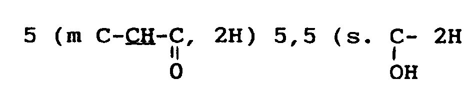 échangeables) 6,4 (s 4H arom) 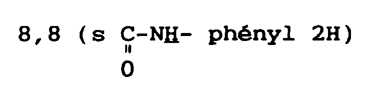
5) Préparation du 4,4′-di-(2-hydroxypropionyl amino) 2,2′,6,6′-tétraamino 3,3′,5,5′-tétraiodo biphényle
   Sur une solution de 6 g (0,0129 mole) du composé dans un mélange d'eau (800 ml) et d'éthylamine (80 ml) à 33%, on additionne goutte à goutte une solution de 100 ml d'H₂0 contenant 41,7 g de I₂ et 31g de KI. Dès la fin de l'addition, le mélange est chauffé pendant 6 heures à 80°C, puis laissé sous agitation une nuit à température ambiante. Le précipité qui se forme est filtré, lavé par H₂O, puis par une solution de bisulfite et enfin séché. Le produit final est obtenu avec 80% de rendement.
   Pureté en iode : 97%
   CCM (toluène/méthyl-éthyl-cétone/Ac. formique : 60/35/25) : R_{f} = 0,2 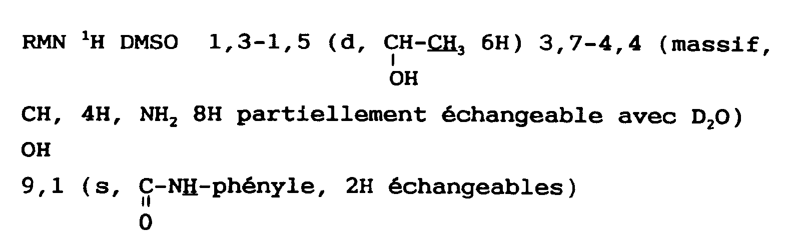
6) Préparation du 4,4′-di[(2-acétoxy 2-propionyloxy)-propyonyl amino] 2,2′,6,6′-tétra-(2-acétoxypropionyl amino)3,3′,5,5′-tétraiodo biphényle
   Sur une solution de 9,5 g (0,01065 mole) du produit précédent dans 100 ml de DMAC anhydre en présence de 20 ml de triéthylamine, on additionne goutte à goutte 21,3 g de chlorure d'acide lactique acétylé. On chauffe la masse réactionnelle pendant 10 h à 50°, puis on maintient pendant 12 h à température ambiante; le DMAC est évaporé sous vide et le résidu versé dans l'eau. Le précipité obtenu est repris dans CHCl₃. La phase chloroformique est lavée à l'eau, séchée sur MgSO₄ et évaporée. Le résidu obtenu cristallise dans l'éther isopropylique et donne le produit final avec un rendement de 73%.
   Pureté en iode : 105%
   CCM (toluène/méthyl-éthyl-cétone/Ac. formique : 60/35/25) : R_{f} = 0,6 (3 taches accolées)
   RMN ¹H DMSO 0,8 à 1,6 (massif CH-CH₃ 24 H) 2ppm (s OCOCH₃ 18H) 4,4 à 5,4 (m - CH 8 H) 7,8 à 12 (massif - NH - 6H échangeables)
7) Préparation du 2,2′,4,4′,6,6′-hexa-(2-hydroxy propionyl amino) 3,3′,5,5′-tétraiodo biphényle (composé n° 5)
   3 g de K₂CO₃ sont ajoutés à une solution de 120 ml de MeOH contenant 6 g (0,0038 mole) du produit précédent. Après agitation pendant une nuit à température ambiante, la solution est évaporée à sec puis diluée dans H₂0. Cette solution est passée sur résine échangeuse d'ions H⁺ puis évaporée. Le résidu est cristallisé dans l'acétone et les cristaux sont lavés à l'éther de pétrole
   Rdt : 35%
   Pureté en iode : 93%
   CCM (dichlorométhane/méthanol : 60/40): R_{f} = 0,9
   CCM (butanol/eau/acide acétique : 50/25/11) : R_{f} = 0,5 - 0,6
   RMN ¹H (DMSO) 0,6 - 1,6 (massif CH CH₃ 18 H) 3,6 - 4,8 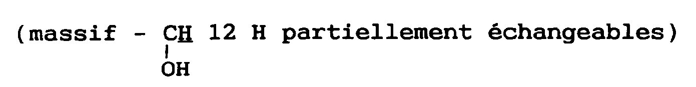 8,5 - 9,8 (massif - NH, 6 H échangeables)

### Exemple 6

Préparation du 2,2′-di-(2-hydroxypropionyl amino) 4,4′,6,6′-tétra-(2-hydroxyéthyl carbamoyl) 3,3′,5,5′-tétraiodo biphényle (composé n° 6)
1) Préparation de l'acide 4-bromo isophtalique
   10 g (54 mmoles) de 4-bromo xylène sont dissous dans 200 ml d'eau en présence de 49 g (0,22 mole) de permanganate de potassium. Le mélange réactionnel est laissé 48 heures au reflux.
   Le dioxyde de manganèse formé est filtré sur Célite et le filtrat est acidifié à froid par une solution de HCl 2N. Le précipité blanc obtenu est filtré, lavé à l'eau et séché à l'étuve.
   Rdt : 43%
   CCM (isopropanol/acétate d'éthyle/ammoniaque 25/35/40) R_{f} : 0,67
   point de fusion > 260°C
   Pureté en brome : 106%
   IR (KBr) : 3000 cm⁻¹ (ν OH), 1680 (ν CO)
   RMN ¹H (DMSO) : disparition des protons méthyliques
2) Préparation de l'acide 4-bromo 5-nitro isophtalique
   235 cm³ de mélange sulfonitrique (HNO₃ : 3,5 ml + H₂SO₄ : 20 ml) sont ajoutés goutte à goutte à 0°C à une solution d'acide 4-bromo isophtalique (10 g) dans 100 cm³ d'acide sulfurique. Le mélange réactionnel est porté à 50°C durant une nuit jusqu'à disparition du produit de départ. Le produit de réaction obtenu est ensuite précipité sur de la glace pilée. Après filtration, lavage à l'eau et séchage, on récupère 10,8 g de produit avec un rendement de 91%.
   CCM (toluène/méthyl-éthyl-cétone/acide formique 60/25/25) : R_{f} : 0,68
   CCM (isopropanol/acétate d'éthyle/ammoniaque 25/35/40) : R_{f} : 0,4
   point de fusion > 260°C
   pureté en brome : 99,3%
   IR (KBr) : 3000 cm⁻¹ (νOH); 1700 (νCO), 1590 (νC = C arom.); 1540 (νN0₂)
   RMN ¹H (DMSO) : δ 8,4 - 8,5 ppm (2 doublets dédoublés, 2 H aromatiques)
3 ) Préparation du 4-bromo 5-nitro isophtalate de diméthyle
   16 ml d'acide sulfurique concentré sont additionnés à une solution de 105 g (0,23 mole) d'acide 4-bromo 5-nitro isophtalique dans 60 cm³ de méthanol. Le mélange réactionnel est porté au reflux pendant 15 heures. Après une nuit à température ambiante, le produit cristallisé est filtré, lavé à l'eau puis séché à l'étuve.
   Rdt : 91%
   CCM (toluène/méthyl-éthyl-cétone/acide formique 60/25/25) : R_{f} : 0,82
   CCM (dichlorométhane) : R_{f} : 0,7
   point de fusion = 90°C
   pureté en brome : 100%
   IR (KBr) : 1735 cm⁻¹ (νCO), 1600 (νC = C); 1540 (νNO₂)

   - RMN ¹H (CDCl₃) :: δ 4 ppm (singulet, 6H, 2 CH₃ des esters)
   δ 8ppm (2 multiplets, 2H aromatiques)
4) Préparation du 2,2′-dinitro 4,4′,6,6′-tétraméthoxycarbonyl biphényle
   40,5 g de cuivre sont additionnés à une solution de 4-bromo 5-nitro isophtalate de diméthyle (71 g, 0,22 mole) dans 240 cm³ de p-xylène. Le mélange est chauffé au reflux durant 24 heures. Après retour à température ambiante, le mélange réactionnel est filtré et le filtrat est évaporé sous pression réduite. Le résidu obtenu est recristallisé dans l'éther isopropylique. On obtient 42,5 g de produit.
   Rdt 80%
   CCM (hexane/acétate d'éthyle v/v) Rf : 0,6
   CCM (CH₂Cl₂) Rf : 0,4
   point de fusion : 116-119°C
   IR (KBr) : 1720 (νCO), 1530 (νNO₂)
   RMN¹H (CDCl₃) : δ 8,8 ppm (multiplet, 4H aromatiques) δ 4 ppm (singulet, 6H, 2 méthyle en 4,4′); δ 3,6 ppm (singulet, 6H, 2 méthyle en 6,6′)
5) Préparation du 2,2′-dinitro 4,4′,6,6′-tétra-(2-hydroxyéthyl carbamoyl) biphényle
   58,8 g d'éthanolamine sont additionnés à une suspension du produit précédent (48,3 g; 0,1 mole) dans 360 cm³ de méthanol. Après 48 heures de reflux, le milieu réactionnel est maintenu pendant 24 heures à température ambiante. Le précipité formé est essoré, puis repris dans l'acétone. Après séchage, on obtient 50,5 g de produit avec un rendement de 84%.
   CCM (dioxane/eau : 9/1) : R_{f} 0,75
   CCM (dichlorométhane/méthanol : 8/2) : R_{f} 0,23
   IR (KBr) : 3430 cm⁻¹ (νN-H), 3200-3500 (νOH), 3090 (νH aromatiques) 2900 (νN-H aliphatiques), 1640 (νCO-NH, 1000 (νC = C arom.), 1545 (νNO₂)
   - RMN ¹H (DMSO, H₂O) :: δ 8,4 et 8,9 ppm (2 doublets, 4H, aromatiques)
   δ 3,25 et 3,6 ppm (2 multiplets, 16 H CH₂ aliphatiques)
6) Préparation du 2,2′-diamino 4,4′,6,6′-tétra (2-hydroxyéthyl carbamoyl) biphényle
   Une solution méthanolique (250 cm³) de 15 g du produit précédent est agitée sous atmosphère d'hydrogène (pression de 3.10⁵Pa) durant 1h30 en présence de charbon palladié à 10% (2,5 g). Le catalyseur est ensuite éliminé par filtration. Après évaporation du solvant sous presion réduite, le résidu est recristallisé dans l'éther, 12,9 g de produit sont obtenus avec un rendement de 90,6%
   CCM (dichlorométhane/méthanol : v/v) : R_{f} 0,45
   RMN ¹H (DMSO) : δ 8,1 et 8,35 ppm (2 massifs, 2H échangeables au D₂O, NH); δ 7,05 ppm (multiplet 4H, aromatiques), 4,4 ppm (massif, 8H échangeables avec D₂O, NH₂, OH); δ 3,05 et ppm (2 multiplets, 16 H, CH₂ aliphatiques)
7) Préparation du 2,2′-diamino 4,4′,6,6′-tétra (2-hydroxyéthyl carbamoyl) 3,3′,5,5′-tétraiodo biphényle 35 cm³ de chlorure d'acide (à 70% en iode) sont ajoutés goutte à goutte sous forte agitation et à température ambiante à une solution du produit précédent (23,6 g) dans 470 cm³ d'eau. Le milieu réactionnel est chauffé à 40°C durant 2 heures. Après une demi-heure à température ambiante, l'excès d'iode est détruit par de l'hydrosulfite de sodium et le précipité est filtré, lavé à l'eau puis à l'éthanol. On sèche à l'étuve à 60°C pour obtenir 40,2 g de produit avec un rendement de 87,5%
   - CCM: (chlorure de méthylène/méthanol v/v) : R_{f} 0,87
   butanol/eau/acide acétique 50/25/11): R_{f} 0,45
   Pureté en iode 103%
   RMN ¹H (DMSO) : δ 7,8-8,5 ppm (2 m, 4H, NH), δ 4,15 ppm (singulet, 4 échangeables, OH); δ 2,6-3,8 ppm (2 massifs, 16 H, CH₂ aliphatiques)

   - HPLC: Lichrospher 100 rp 8,5 »; 12,5 cm
   MeOH 65/eau 35 : 1 ml/min, pureté 99%
8) Préparation de 2′,2′-di(2-acétoxypropionyl amino) 4,4′,6,6′-tétra(2-acétoxyéthyl carbamoyl) 3,3′,5,5′-tétraiodo biphényle
   5,3 g du chlorure de l'acide lactique O-acétylé (34,7 moles) sont additionnés goutte à goutte à 0°C, à une solution contenant 3 g du produit précédent dans 35 cm³ de DMAC anhydre. La suspension est ensuite portée à 60°C pendant 21 heures. Le DMAC est évaporé sous pression réduite. L'huile obtenue est retraitée par le dichlorométhane, lavée à l'eau puis séchée par sulfate de sodium. Après évaporation sous vide, le résidu est précipité dans un mélange de chlorure de méthylène et d'éther isopropylique, puis filtré pour donner 3,9 g de produit avec un rendement de 78,3%

   - CCM: (chlorure de méthylène/méthanol 8/2) : R_{f} : 0,88
   (butanol/eau/acide acétique 50/25/11 ): R_{f} : 0.82
   (isopropanol/acétate d'éthyle/ammoniaque): R_{f} : 0.86
   Pureté en iode 98,2%
   IR (KBr) : 3340 (νN-H), 1730 cm⁻¹ (νCO ester); 1650 (νCO-NH)
   RMN ¹H (CDCl₃) : δ 5 ppm (multiplet, 1H, CH lactique); 4,3 ppm (m, 2H, CH₂-CO); 3,7 ppm (multiplet, 2H, N-CH₂); 2,1 ppm (singulet, 18 H, O-CO-CH₃); 1,5 ppm (d, 18 H, CH₃ lactique)
9) Préparation du 2,6-di-(2-hydroxypropionyl amino)-2,4,4′,6′-tétra-(2-hydroxyéthyl carbamoyl) 3,3′,5,5′-tétraiodo biphényle (composé n° 6)
   Le composé 0-acylé obtenu à l'étape précédente (49 g) dissous dans 600 cm₃ d'éthanol est traité par une solution éthanolique (200 cm³) de soude (16 g). Le milieu réactionnel est maintenu sous agitation durant 3 heures. Le résidu après évaporation du solvant est lavé à l'acétone puis précipité avec l'éther. Le produit filtré est déminéralisé par passages sur résines H⁺ (IRN77) et OH⁻ (IRN 78). l'eau est évaporé sous pression réduite et le résidu est précipité dans de l'acétone puis lavé à l'éther pour obtenir 23 g de produit brut.

### Exemple 7

Préparation du 2′,2′-di-(2-hydroxy-acétylamino)-4,4′,6,6′-tétrakis-(2,3-dihydroxypropyl carbamoyl) 3,3′,5,5′-tétraiodo biphényle (composé n° 7)
1) Préparation du 2,2′-dinitro 4,4′,6,6′-tétrakis-(2,3-dihydroxypropyl carbamoyl) biphényle
   74 g d'aminopropanediol sont additionnés goutte à goutte à une solution préchauffée de produit obtenu dans l'exemple précédent en 4 (38,7 g; 81,3 moles) moles) dans 255 cm³ de méthanol. Le mélange est porté au reflux durant 12 h. Après retour à température ambiante, le milieu réactionnel est filtré et le filtrat est évaporé sous pression réduite. Le résidu est dissous dans 750 cm³ d'eau puis purifié par passage sur une colonne de résine H⁺ (IRN 77). L'eau est éliminée par distillation et le résidu est repris dans l'éther isopropylique. Le produit obtenu est filtré et séché.
   masse obtenue 55,3 g, rendement 95,5%
   CCM (dioxanne/eau : 9/1) R_{f} : 0.65
   RMN ¹H (DMSO) :
   δ 3,6 et 3,3 ppm (2 m, 20 H, -CH₂ et CH aliphatiques)
   δ 4,55 ppm (1 m, 8H échangeables au D₂O, OH)
   δ 8,95 et 8,5 ppm (2 m, 4H aromatiques)
   IR (BKr) 3300 cm⁻¹ (νOH) 1630 (νCO-NH); 1520 et 1310 (νNO₂)
2 ) Préparation du 2,2′-diamino 4,4′,6,6′-tétrakis-(2,3-dihydroxypropyl carbamoyl) biphényle
   Une solution aqueuse (600 cm³) du composé précédent (28,9 g; 40,6 mmol) est agitée sous atmosphère d'hydrogène (pression de 4.10⁵Pa) durant 5 heures en présence de charbon palladié à 10% (6g). le catalyseur est ensuite éliminé par filtration. Après évaporation du solvant sous pression réduite, le résidu est repris dans le méthanol, concentré puis neutralisé dans l'éther isopropylique.
   24,7g de produit sont obtenus avec un rendement de 93,3%
   CCM (dioxanne/eau : 9/1) : R_{f} 0,6
   RMN ¹H (DMSO)
   δ 3,3 et 3,1 ppm (1 massif, 20 H, CH₂ et CH aliphatiques)
   δ 4,45 ppm (1 massif, 12 H échangeables au D₂0, 8 0H + 2 NH₂)
   δ 7,1 ppm (1 massif, 4H aromatiques)
   δ 8,4 et 8,1 ppm (2 massifs, 4H échangeables au D2O, 4NH)
   IR (KBr) 3100-3500 cm⁻¹ (ν OH, NH₂; 1620 (ν COHN)
3) Préparation du 2,2′-diamino 4,4′,6,6′-tétrakis-(2,3-dihydroxypropyl carbamoyl) 3,3′,5,5′-tétraiodo biphényle
   126 cm³ de chlorure d'iode (à 70% en iode) sont ajoutés goutte à goutte, sous forte agitation et à température ambiante dans une solution du composé obtenu précédemment (63,9 g; 98 moles) dans 300 cm³ d'eau. Le milieu réactionnel est maintenu pendant 7 heures à 45°C puis 16 heures à température ambiante. Après évaporation, le résidu est repris dans l'éthanol absolu. Le solide récupéré par filtration est ensuite purifié après dissolution dans 1 l d'eau par passages successifs sur résines H⁺ (IRN 77) et OH⁻ (IRN 78). La solution résultante est évaporée à sec. Après lavage à l'éther et séchage, on isole 73,7 g de produit avec un rendement de 65%
   CCM (dioxanne/eau : 9/1) R_{f} : 0,86
   Pureté en iode : 98,1%
   RMN ¹H (DMSO)
   δ 3,95 ppm (1 massif, 20 H CH₂ et CH aliphatiques)
   δ 4,6 ppm (1 massif, 12 H échangeables au D₂O, 8 OH et 2NH₂)
   δ 8,2 ppm (1 massif, 4H échangeables, NH)
3) Préparation du 2,2′-di-(2-acétoxyacétyl amino) 4,4′,6,6′-tétrakis-[2,3-di(2-acétoxy-acétoxy)-propyl carbamoyl] 3,3′,5,5′-tétraiodo biphényle
   100,4 g du chlorure de l'acide glycolique 0-acétylé (0,735 mole) sont additionnés goutte à goutte à une solution de 42,5 g (36,7 moles) du composé précédent dans 300 cm³ de DMAC anhydre. Le milieu réactionnel est laissé sous agitation à température ambiante durant 22 heures jusqu'à disparition de l'amine de départ (controlée par le test au thiocol). Le DMAC est évaporé sous pression réduite, le résidu obtenu est extrait par le dichlorométhane, lavé à l'eau puis séché sur Naₐ₂SO₄. Après concentration et reprise par l'éther éthylique, on obtient 72,1 g de produit avec un rendement de 91%
   CCM : (dioxanne/eau : 9/1) : R_{f} 0.9
   Pureté en iode : 99,2%
4) Préparation du 2′,2′-di-(2-hydroxy-acétylamino) 4,4′,6,6′-tétrakis-(2,3-dihydroxypropyl carbamoyl 3,3′,5,5′-tétraiodo biphényle (composé n° 7)
   69 g (0,032 mole) du produit décrit à l'étape précédente sont déprotégés en présence de 100 cm³ d'éthanol absolu et de 1,3 1 d'une solution de soude éthanolique 0,5N. Le milieu réactionnel est maintenu sous agitation durant 3/4 heure. Le précipité formé est essoré, séché puis déminéralisé par passages sur résines H⁺(IRN 77) et OH⁻ (IRN 77). L'eau est éliminée par distillation et le résidu est repris dans l'éther éthylique.
   28,7 g de produit sont récupérés puis purifiés par HPLC (RP 18)
   Rendement global : 56,4%
   CCM (dioxanne/eau : 9/1) : R_{f} : 0,55
   Pureté HPLC : 98,3%
   RMN¹H(DMSO)
   δ 8,95 ppm (massif, 2H échangeables au D₂O, -NH-CO)
   δ 8,35 ppm (massif, 4H échangeables au D₂0, -CO-NH)
   δ 5,2 ppm (massif, 2H échangeables au D₂0, -NHCOCH₂0H)
   δ 4,7 ppm (massif, 8H échangeables au D₂0, -COHNCH₂-CHOH-CH₂OH)
   δ 3,4 ppm (massif, 24 H échangeables, protons aliphatiques)

### Exemple 8

Préparation du 3,3′-bis-[(3-hydroxy 2-hydroxy-méthyl) propionyl] amino 5,5′-bis-[N-méthyl - N - (2,3-dihydroxy-propyl)] carbamoyl 2,2′, 4,4′, 6,6′-hexaiodo biphényle (composé n° 8).
1) Préparation de l'acide 3-iodo 5-nitro benzoïque
   A 122 g (0,53 mole) de H₅IO₆ et 400 g (1,57 mole) d'iode dissous à 10°C sous agitation pendant 30 minutes dans 2750 ml d'oléum à 20 %, sont ajoutés 120 g (0,72 mole) d'acide 3-nitro benzoïque. Après 12 heures d'agitation à température ambiante, cette solution est versée lentement dans de la glace. Le précipité formé est essoré puis lavé avec une solution à 20 % de bisulfite de sodium avant d'être dissous dans une solution de soude, puis filtré sur papier. Après acidification par HCl, on obtient 170 g de cristaux blancs qui sont essorés et séchés.
   Rdt = 81 %
   PF = 172°C
   CCM (toluène 60/méthyléthylcétone 25/HCOOH 5) R_{f} = 0,75 Pureté en iode = 99 %
   - RMN ¹H(DMSO): 8,5 ppm (s, 2H aromatiques)
   8,7 ppm (s, 1H aromatique)
   13 ppm (m, COOH 1H échangeable avec D₂O)
2) Préparation de l'ester méthylique de l'acide 3-iodo 5-nitro benzoïque
   180 g (0,614 mole) du composé obtenu en 1 dissous dans 1800 ml de méthanol et 10 ml d'H₂SO₄ à 98 % sont portés au reflux pendant 24 heures. Après évaporation du méthanol aux deux-tiers, la solution obtenue est refroidie et l'ester qui précipite est filtré. Après dissolution du produit dans 2000 ml d'éther, la phase éthérée est lavée par 1000 ml d'H₂O, puis séchée sur
   MgSO₄ et évaporée à sec. On obtient 181 g de cristaux blancs.
   Rdt = 90 %
   PF = 88°C
   pureté en iode 99 %
   CCM (CH₂Cl₂ 70 - MeOH 30) : R_{f} = 0,95

   - IR: 1720 (COOCH₃)
   1520 (NO₂)

   - RMN ¹H ( DMSO ): 3,9 ppm (s, COOCH₃, 3H)
   8,5 ppm (s, 2H aromatiques)
   8,7 ppm (s, 1H aromatique).
3) Préparation du 5,5′-diméthoxycarbonyl 3,3′-dinitro biphényle
   86g (0,28 mole) du composé obtenu à l'étape 2) sont portés à 220°C. Après addition de 86 g de cuivre, la température est augmentée progressivement jusqu'à 270°C et 20 g de cuivre sont de nouveau ajoutés. Le mélange est maintenu à cette température pendant 1 heure avant d'être refroidi. Après extraction par CH₂Cl₂ et filtration sur celite, la phase organique est évaporée à sec.
   La pâte résiduelle est lavée par 2 x 500 ml d'éther de pétrole, puis reprise dans l'éther. Le précipité brun formé est essoré puis purifié par chromatographie sur silice. Après évaporation, on obtient 20g de cristaux bruns.
   Rdt = 40 %
   PF = 159°C
   CCM (CH₂Cl₂) : R_{f} = 0,6

   - RMN ¹H (DMSO) :: 3,9 ppm (s, COOCH₃ 6H)
   8,5 ppm (2s, 4H aromatiques)
   8,7 ppm (1s, 2H aromatiques)
4) Préparation du 5,5′-dicarboxy 3,3′-dinitro biphényle
   14 g (0,038 mole) du composé obtenu en 3) sont portés au reflux pendant 18 h dans 100 ml d'une solution aqueuse de NaOH à 25 %.
   La solution obtenue est refroidie ; après acidification, le précipité formé est extrait par de l'acétate d'éthyle, et lavé par H₂O. Après évaporation et lavage par l'éther, on obtient 12 g de cristaux blancs.
   Rdt = 95 %
   PF > 300°C
   CCM (toluène 60/méthyléthylcétone 25/HCOOH 25) : R_{f} = 0,8
   - RMN ¹H (DMSO):: 5,3 ppm (m, COOH, 2H échangeables avec D₂O)
   8,65 à 8,8 ppm (3s, 6H aromatiques).
5) Préparation du 3,3′-dinitro 5,5′-bis-(N-méthyl N-2,3-dihydroxy propyl carbamoyl biphényle
   1,66 g (0,005 mole) du composé obtenu en 4) sont ajoutés à une solution de 60 ml de SOCl₂ et 0,1 ml de diméthylformamide. La solution est portée au reflux pendant 5 heures. Après distillation du SOCl₂, la pâte obtenue est dissoute dans CH₂ Cl₂ et versée goutte à goutte à 5°C dans une solution contenant 2,1g (0,02 mole) de N-méthyl amino 2,3-propanediol et 2,8 ml (0,02 mole), de triéthylamine dissous dans 20 ml de diméthylacétamide. Après addition, le mélange est agité à température ambiante pendant 12 h. Après filtration du chlorhydrate de triéthylamine, le solvant est évaporé. La pâte obtenue est purifiée par passage sur résine H⁺/OH⁻ puis par chromatographie sur silice. Après évaporation des solvants, on obtient 1,9 g de cristaux blancs.
   Rdt = 70 %
   CCM (CH₂Cl₂ 85/MeOH 15) R_{f} : 0,35 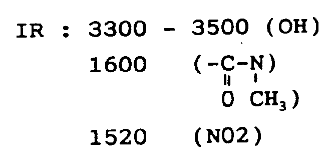 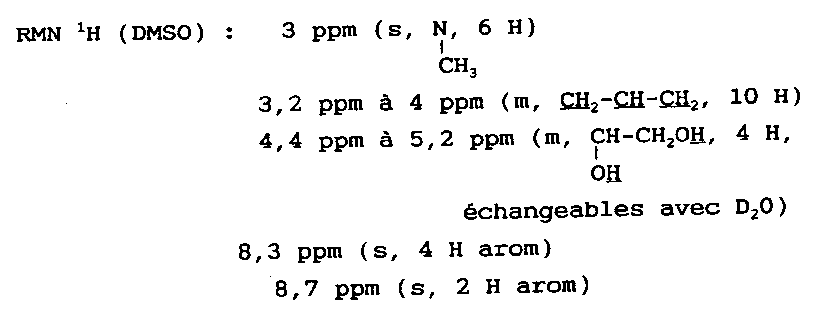
6) Préparation du 3,3′-diamino 5,5′-bis-[N-méthyl N-(2,3-dihydroxypropyl)carbamoyl] 2,2′,4,4′-hexaiodo biphényle
   a) Réduction des groupes nitro :
      Dans un autoclave de 500 ml, 1,6 g (0,0031 mole) du composé obtenu en 5) dissous dans 200 ml de méthanol en présence d'1g de Pd/C aqueux à 10 % sont agités pendant 2h30 à 40°C sous une pression d'hydrogène de 6.10⁵ Pa. Après fixation du catalyseur et évaporation du solvant, on obtient une pâte.
      CCM (CH₂Cl₂ 80/MeOH 20) : R_{f} = 0,1
   b) Iodation par ICl
      A la pâte obtenue en a) dissoute dans 100 ml d'un mélange MeOH/H₂0:50/50, sont ajoutés goutte à goutte 2,1 ml (0,027 mole) d'une solution d'ICl à 70 %. L'addition terminée, le mélange réactionnel est porté à 60°C pendant 4 h puis laissé 12 h à température ambiante. La solution brune obtenue est versée sous agitation dans 200 ml d'eau. Le précipité formé est essoré, puis lavé par une solution de bisulfite de sodium à 20 % et par H₂0. Le produit est repris dans l'éther, puis séché. On obtient 3 g de cristaux blancs
      Rdt = 80 %
      Pureté en iode = 99 %
      CCM (CH₂Cl₂ 85/MeOH 15) : R_{f} = 0,4 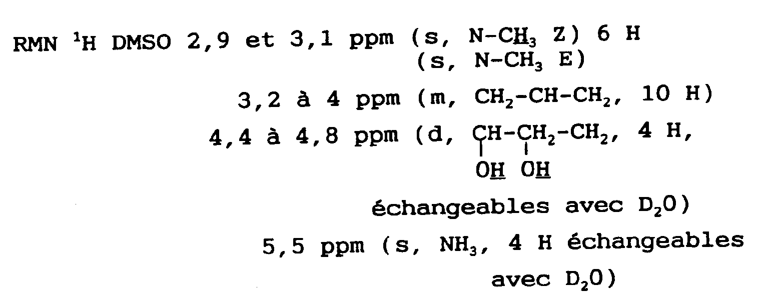
   7) Préparation du 3,3′-diamino 5,5′-bis [N méthylN-(2,3-diacétoxypropyl) carbamoyl] 2,2′,4,4′,6,6′-hexaiodo biphényle
      A 1,2g (0,001 mole) du composé obtenu en 6) dissous dans 12 ml de pyridine est additionné goutte à goutte à 5°C 1,5 ml d'anhydride acétique. Après 18 h d'agitation à température ambiante, le produit réactionnel brut est versé dans de l'eau glacée acidifiée par 30 ml d'HCl 5N. Le précipité formé est essoré puis repris dans CH₂ Cl₂. La phase organique est lavée à l'eau puis séchée sur Mg S0₄. Après évaporation, on obtient 1 g de cristaux blancs qui sont lavés à l'éther et séchés
      Rdt : 73 %
      Pureté en iode : 98 %
      CCM (CH₂Cl₂ 85/MeOH 15) : R_{f} = 0,8 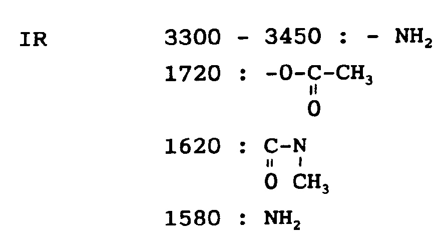 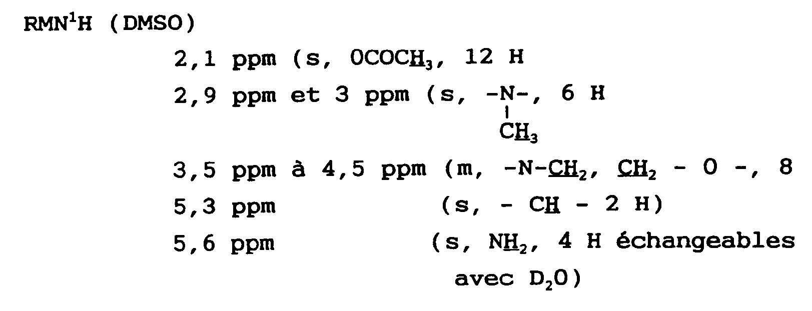
8) Préparation du 3,3′-bis-[5-(2-isopropyl) dioxanne-1,3-yl] carbonylamino 5,5′-bis-[(N-méthyl N-(2,3-diacétoxy propyl) carbamoyl] 2,2′,4,4′,6,6′-hexaiodo biphényle
   a) préparation du chlorure de l'acide 5-(2-isopropyl dioxanne-1,3-yl) carboxylique
      A 0,6 g (0,0035 mole) de l'acide 5-(2-isopropyl dioxanne - 1,3 yl) carboxylique dissous à 0°C dans 5,5 ml de diméthylacétamide est ajouté lentement 0,28 ml de SOCl₂. Après addition, la solution est agitée pendant 5 h à température ambiante.
   b) Après addition par portions de 0,68 g (0,0005 mole) du composé obtenu en 7) à la solution préparé en a), le produit brut réactionnel est chauffé pendant 12 h à 45°C puis versé sur de la glace. Le précipité formé est essoré, repris dans CH₂Cl₂, et lavé à l'eau. Après évaporation et reprise dans l'éther, on obtient 0,7 g de cristaux beiges.
   Rdt : 83 %
   CCM (CH₂Cl₂ 80/MeOH 20) : Rf = 0,7 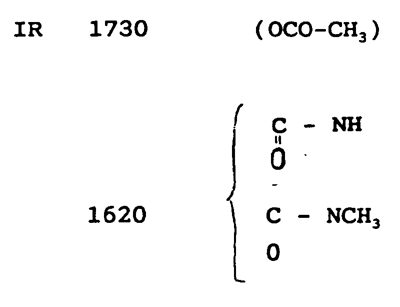 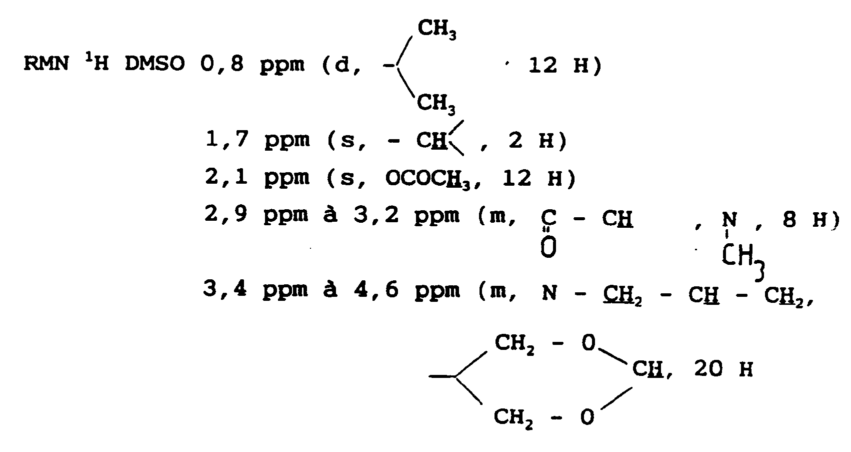
9) Préparation du 3,3′-bis-[5-(2-isopropyl dioxanne-1,3-yl] carbonylamino 5,5′-bis-[N-méthyl N-(2.3-dihydroxypropyl)]carbamoyl 2,2′,4,4′,6,6′ - hexaiodo biphényle
   0,5 g (0,3 mmole) du composé obtenu en 8) dissous dans 10 ml de Me0H comprenant en suspension 80 mg de K₂CO₃ sont agités pendant 24 heures à température ambiante. Après filtration, évaporation du méthanol et concrétisation dans l'éther, on obtient 0,6 g de cristaux blancs.
   Rdt > 100 % (présence de K₂CO₃) 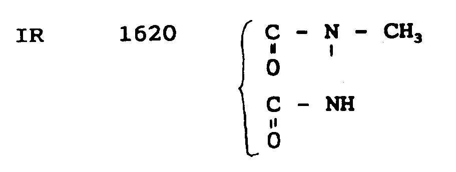
   - 3200 - 3500: CH - OH
   CH₂ 0H
   CCM (CH₂Cl₂ 85/MeOH 15) : R_{f} = 0,3 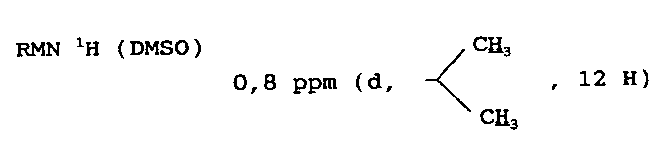
   1,5 ppm à 1,8 ppm (m, -CH , 2 H)
   2,9 ppm à 4,9 ppm (m, échangeable partiellement avec D₂0) 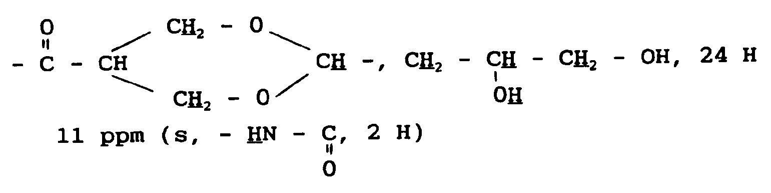
10) Préparation du 3,3′-bis[(3-hydroxy 2-hydroxyméthyl)propyonyl] amino 5,5′-bis [N-méthyl N-(2-3 dihydroxypropyl)] carbamoyl 2,2′,4,4',6,6′ - hexaiodo biphényle
   0,5 mg (0,33 mmole) du composé obtenu en 9) sont agités pendant 6 h à température ambiante en présence de 13 ml d'acide chlorhydrique 5N. Après évaporation, le résidu obtenu est purifié par passage sur résines H⁺ /OH⁻^{,} puis par chromatographie sur silice.
   Après concentration et concrétisation dans l'éther, on obtient 0,3 g de cristaux blancs.
   Rdt : 60 %
   CCM (CH₂Cl₂ 60/MeOH 40) : R _{f} = 0,3
   Pureté en iode = 99,5 %
   - IR : 3200 - 3500: CH - 0H
   CH₂ 0H 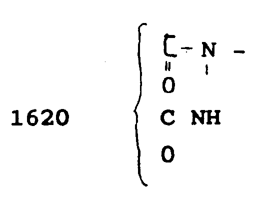
   RMN ¹H DMSO :
   2,6 ppm à 4 ppm
   (m, échangeable partiellement avec D₂0) 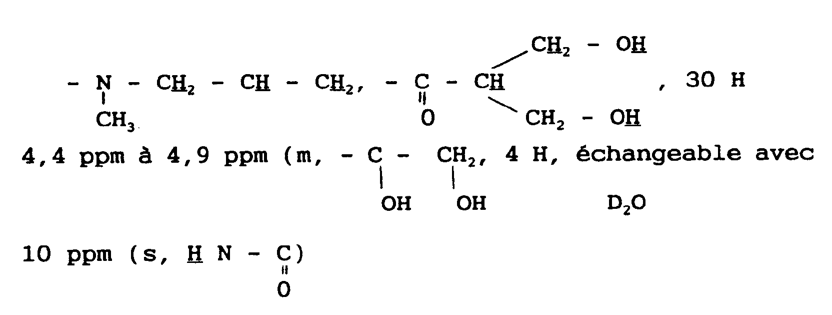
   - RMN ¹³C :: 38,35 ppm, s, N-CH₃
   50,57 ppm, s, N-CH₂

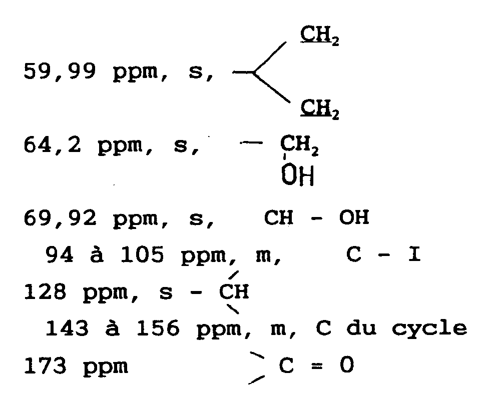

### Exemple 9

Préparation du 3,3' bis-[(3-hydroxy 2 - hydroxyméthyl) propionyl] amino 5,5'-bis-[N-bis(2, 3-dihydroxy)propyl] -carbamoyl 2,2',4,4',6,6' - hexaiodo biphényle
1) Préparation du 3,3'-dinitro 5,5'-bis (2,3-dihydroxy propyl) carbamoyl biphényle
   0,9 g (2,7 mmoles) du composé obtenu à l'Exemple 8 4) sont ajoutés à une solution de 50 ml de SOCl₂ et de 0,05 ml de diméthylformamide.. La solution est portée pendant 6 h au reflux, puis le SOCl₂ est distillé sous vide. La pâte résiduelle obtenue dissoute dans 5 ml de CH₂Cl₂ est versée goutte à goutte à 5°C sur une solution de 1,8 g (0,011 mole) de 2,3-(N-2,3- dihydroxy propyl amino) propanediol et 1,53 ml (0,011 mole) de triéthylamine dissous dans 10 ml de diméthylacétamide. Après addition, le mélange est agité pendant 12 h à température ambiante. Le chlorhydrate de triéthylamine est éliminé par filtration et les solvants sont évaporés. Le résidu est purifié par chromatographie sur silice. Après évaporation, on obtient 0,9 g de cristaux blancs Rdt = 55 %
   CCM (CH₂Cl₂ 60/MeOH 40) : R_{f} = 0,3
   3300 - 3500 (OH)
   IR : 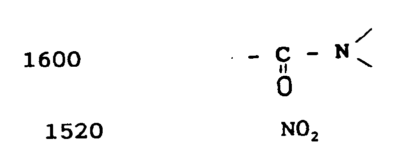
   - RMN ¹H DMSO: 3 à 4 ppm (m, CH₂ - CH - CH₂, 20 H) 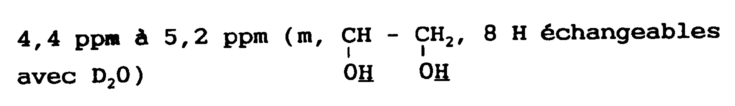 8,3 ppm (s, 4 H arom)
   8,6 ppm (s, 2 H arom)
2) Préparation du 3,3′-diamino 5,5′-bis-[N-bis-(2,3-dihydroxy) propyl] carbamoyl biphényle
   a) Réduction des groupes nitro :
      Dans un autoclave de 500 ml, on agite 0,8 g (1,27 mmole) du composé obtenu en 2) dissous dans 100 ml de MeOH pendant 2h30 à 40°C sous une pression d'hydrogène de 6.10⁵ Pa en présence d'1 g de Pd/C à 10 % aqueux.
      Après filtration du catalyseur et évaporation du solvant, on obtient une pâte.
      CCM (CH₂Cl₂ 50/MeOH 50) : R_{f} = 0,05
   b) Iodation par ICl :
      A la pâte obtenue en a) dissoute dans 60 ml d'un mélange Me0H / H₂0 (50/50) est ajouté goutte à goutte 0,84 ml (11 mmoles) d'une solution d'ICl à 70 %. L'addition terminée, la réaction est portée à 60°C pendant 4 h puis laissée pendant 12 h à température ambiante. Après évaporation à sec de la solution brune, le résidu est concrétisé dans l'acétate d'éthyle. Les cristaux beige foncé sont filtrés, lavés par du tétrachlorure de carbone et de l'éther de pétrole. Après séchage, on obtient 0,7 g de cristaux beiges.
      Rdt = 43 %
      CCM (CH₂Cl₂ 65/MeOH 35) : R_{f} = 0,2
      Pureté en iode : 96 %
      RMN ¹H (DMS0)
      3 à 4,2 ppm (m, partiellement échangeable avec D₂0 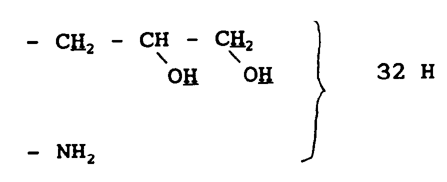
3) Préparation du 3,3′-diamino 5,5′-bis-[N-bis-(2,3-diacétoxy) propyl] carbamoyl 2,2′,4,4′,6,6′ - hexaiodo biphényle
   A 0,3 g (0,23 mmole) du composé obtenu en 2) dissous dans 5 ml de pyridine est additionné goutte à goutte à 5°C 0,7 ml d'anhydride acétique. Après 18 h d'agitation à température ambiante, le mélange réactionnel est versé dans de l'eau glacée acidifiée par 15 ml d'HCl 5 N. Le précipité formé est essoré puis repris dans CH₂Cl₂. La phase organique est lavée par l'eau, puis séchée sur MgS0₄. Après évaporation et concrétisation dans l'éther, on obtient 0,3 g de cristaux blancs.
   Rdt : 80 %
   Pureté en iode = 95 %
   CCM (CH₂Cl₂ 85/Me0H 15) : R_{f} = 0,8
   - IR :: 3300 - 3450 : NH₂ 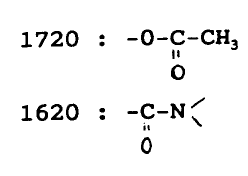 1580 : -NH₂
4) Préparation du 3,3′-bis-[5-(2-isopropyl) dioxanne -1,3 - yl] carbonylamino 5,5′-bis-[N-bis-(2,3-diacétoxy) propyl] carbamoyl 2,2′,4,4′,6,6′ - hexaiodo biphényle)
   a) Préparation du chlorure de l'acide 5-(2-isopropyl dioxanne - 1,3 - yl) carboxylique :
      A 0,24 g (1,38 mmoles) d'acide 5-(2-isopropyl dioxanne-1,3-yl) carboxylique dissous à 0°C dans 2,3 ml de diméthylacétamide est ajouté lentement 0,112 ml de SOCl₂. Après addition, la solution est agitée pendant 5 heures à température ambiante.
   b) Après addition par portions de 0,3 g (0,2 mmole) du composé obtenu en 3) à la solution obtenue en a ), le mélange réactionnel est chauffé pendant 12 h à 45°C puis versé sur de la glace.
      Le précipité formé est filtré, repris dans CH₂Cl₂ et lavé à l'eau. Après évaporation et reprise dans l'éther, on obtient 0,2 g de cristaux beige.
   Rdt : 52 %

   - IR :: 1730 (0COCH₃) 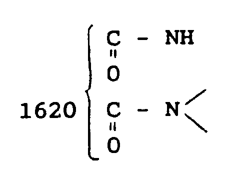
   CCM (CH₂Cl₂ 95/Me0H 5) : R_{f} = 0,45
5) Préparation du 3,3′-bis-[5-(2-isopropyl) dioxanne-1,3-yl]carbonylamino 5,5′-bis - [N-bis-(2,3-dihydroxy propyl)] carbamoyl 2,2′,4,4′,6,6′ - hexaiodo biphényle
   0,2 g (0,1 mmole) du composé obtenu en 4) dissous dans 5 ml de MeOH et 50 mg de K₂C0₃ sont agités pendant 18 h à température ambiante. Après filtration et évaporation du méthanol, on obtient 200 mg de cristaux blancs.
6) Préparation du 3,3′ bis-[(3-hydroxy 2 - hydroxyméthyl) propionyl] amino 5,5′-bis-[N-bis(2,3-dihydroxy)-propyl]carbamoyl 2,2′,4,4′,6,6′ - hexaiodo biphényle
   On agite 0,2 g du composé obtenu en 5) pendant 6 h à température ambiante en présence de 5 ml d'HCl 5N. Après évaporation, le résidu obtenu est purifié par passage sur résines H⁺/OH⁻ et par cristallisation dans l'éther. On obtient 100 mg de cristaux blancs.
   Rdt = 50 % (pour l'ensemble des étapes 5) et 6)
   Pureté en iode = 93 %
   CCM (CH₂Cl₂ 20/MeOH 80) : 2 taches accolées : R_{f} = 0,8
   IR : 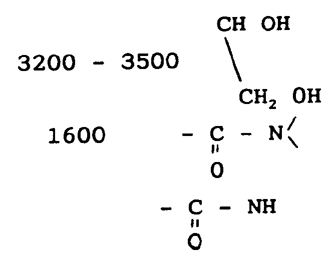
   - RMN ¹H (DMSO): 3 à 5,1 ppm (m, partiellement échangeable avec D₂0) 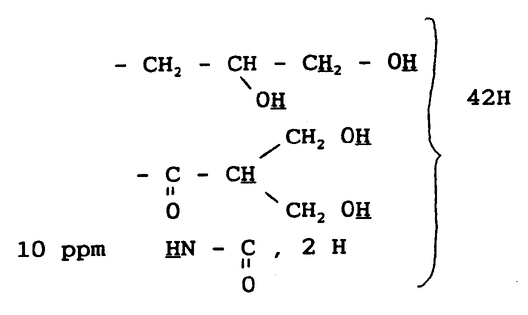
**Exemple 10** Préparation du composé de formule : 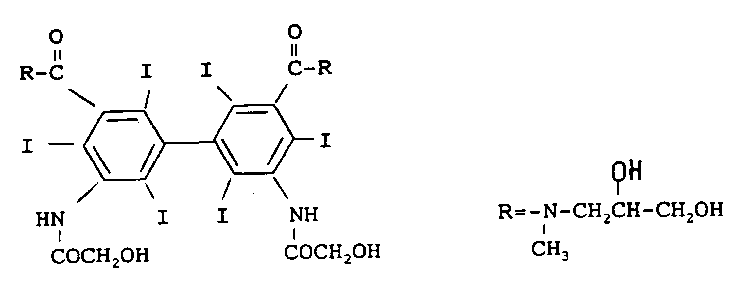
   1 - Préparation du composé de formule : 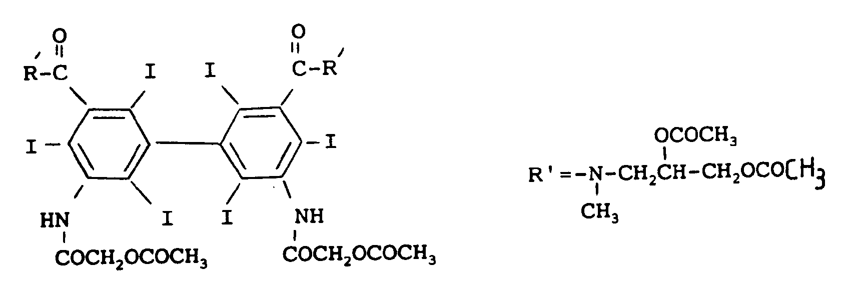
      A 5,5 g (0,0040 mole) du composé obtenu à l'exemple 8-7 dissous dans 15 ml de DMAC est ajouté goutte à goutte 1,4 g (0,01 mole) de 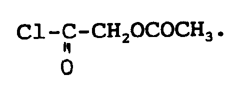 La solution obtenue est agitée à 50°C pendant 16 h, puis versée dans de la glace. Le précipité obtenu est essoré, lavé par H₂O, puis séché. On obtient 3,4 g de cristaux blancs.
      Rendement : 55%
      CCM (acétate d'éthyle) : Rf : 0,3
      I.R. : 1730 (OCO-CH₃) 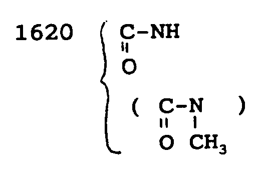 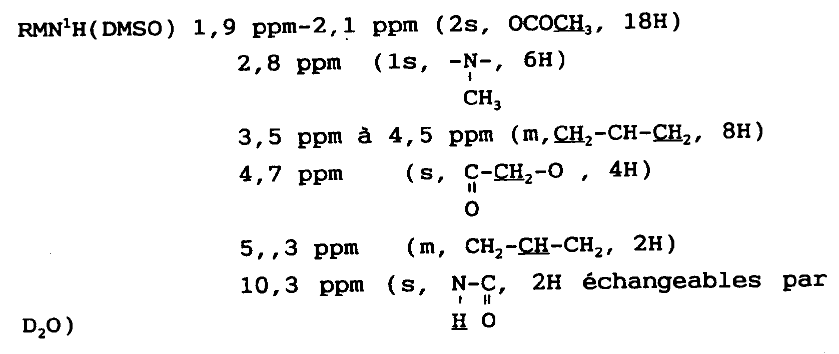
   2 - Préparation du composé de formule : 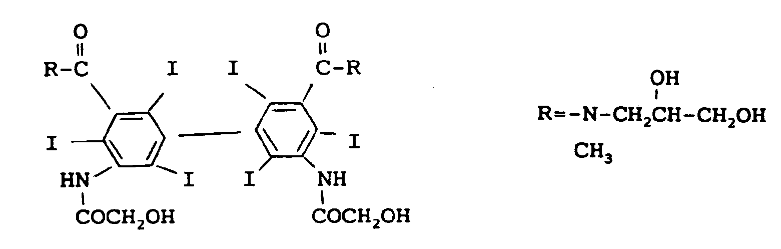
      2,5 g (0,00153 mole) du composé obtenu en 1) dissous dans 100 ml de MeOH et 1,3 g (0,01 mole) de K₂CO₃ sont agités pendant 48 heures à température ambiante. Après filtration et évaporation du méthanol, le résidu est dissous dans 100 ml d'eau, puis passé sur résine H⁺. Après évaporation, le produit obtenu est purifié par chromatographie sur silice. Après évaporation et concrétisation dans l'éther, on obtient 1,6 g de cristaux blancs.
      Rendement : 76 %
      CCM : silice; CH₂Cl₂/MeOH:70/30; Rf:0,4
      Pureté en iode = 99,5% 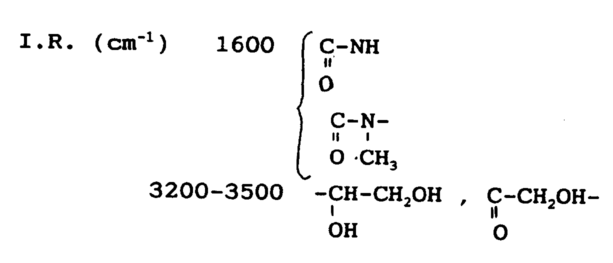 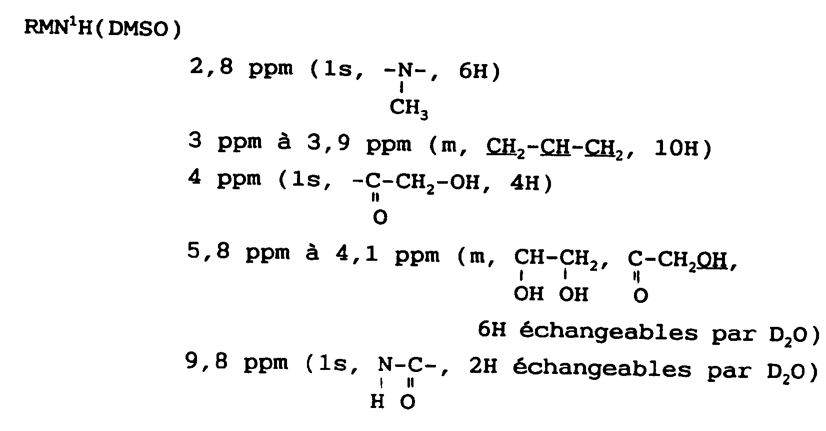 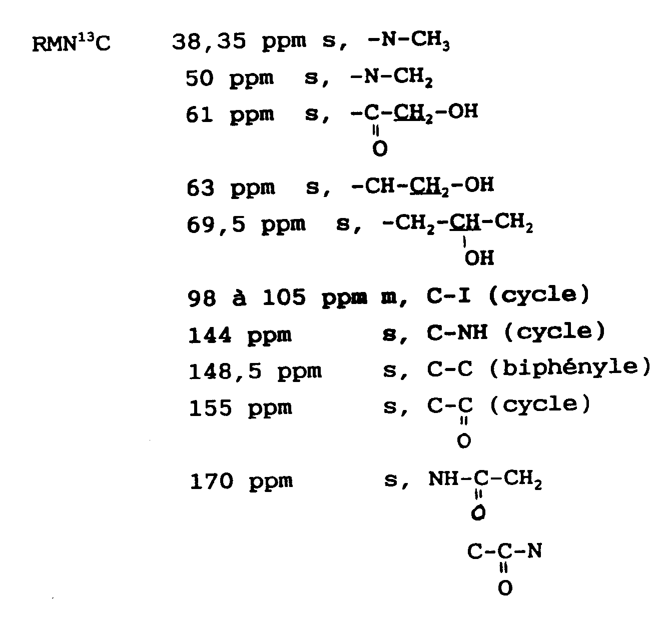
**EXEMPLE 11** Préparation du composé de formule : 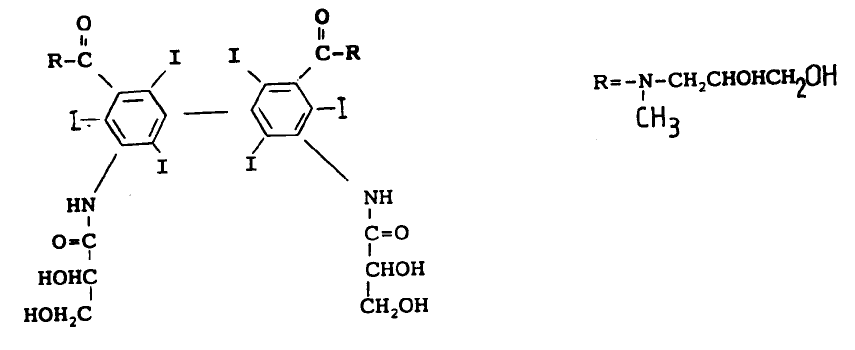
   1 - Préparation du composé de formule : 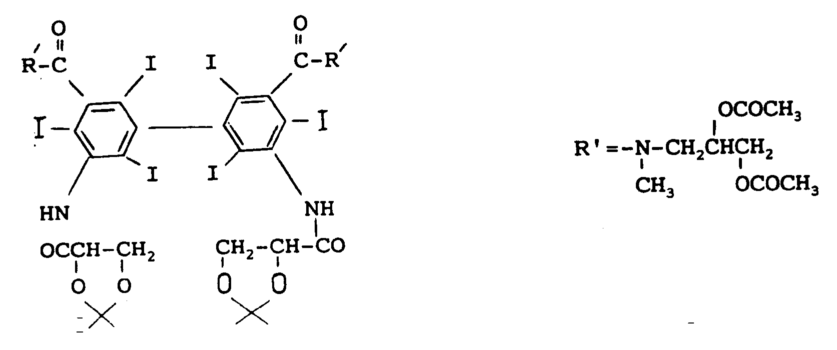
      a) Préparation du composé de formule : 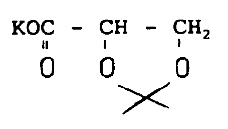
         Dans une solution aqueuse de 150 g (1,14 mole de Solketal^{R} (disponible commercialement auprès de la société Janssen (Pantin)) et de 69,4 g (1,23 mole) de potasse est ajoutée goutte à goutte une solution aqueuse de 294 g (1,75 mole) de KMnO₄, la température étant maintenue à 4-9°C.
         Le milieu réactionnel est agité pendant une nuit à température ambiante. Après filtration, ajustement du pH de la solution à 9 et concentration à siccité, le résidu est repris à l'éthanol. Les sels minéraux sont filtrés et la phase éthanolique est concentrée à sec.
         Rendement : 75% (sel de potassium).
      b) Préparation du composé de formule : 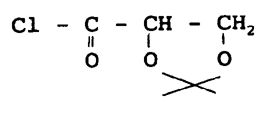
         A 2,2 g (0,012 mole) du produit obtenu en a) ci-dessus mis en suspension dans 12 ml d'éther anhydre est ajouté goutte à goutte 1,15 ml de chlorure d'oxalyle à une température de 0°C. Le mélange est agité pendant deux heures à O°C puis pendant 18 heures à température ambiante.
         Après filtration du KCl, la solution est évaporée à 20°C sous vide.
      c) A 2,7 g (0,002 mole) du composé obtenu dans l'exemple 8-7) dissous dans 10 ml de DMAC est ajoutée goutte à goutte la solution préparée en b) ci-dessus. Le mélange obtenu est agité pendant 17 heures à température ambiante puis versé dans de l'éther. Les cristaux formés sont essorés et séchés. On obtient 3 g de cristaux beiges, soit un rendement de 90%.
         CCM (acétate d'éthyle) : Rf = 0,75
   2 - Préparation du composé de formule 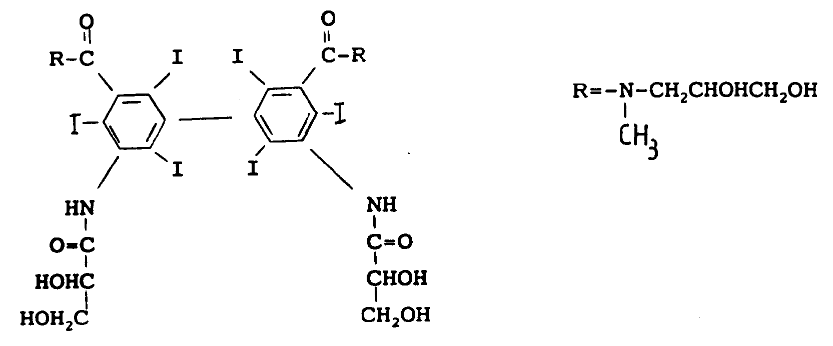
      3 g (0,0018 mole) du composé obtenu à l'étape précédente dans 150 ml de méthanol et 1,8 g de K₂CO₃ (0,015 mole) sont agités pendant 48 heures à température ambiante. Après filtration et évaporation du méthanol, le résidu est dissous dans une solution aqueuse à pH3. La solution est agitée pendant une heure, passée sur résines H⁺ et OH⁻, puis purifiée par chromatographie sur silice. Après évaporation et concrétisation dans l'éther, on obtient 1,2 g de cristaux blancs (Rdt = 46%)
      CCM (CH₂Cl₂/MeOH:50/50) Rf:0,6
      pureté en iode : 99 %. 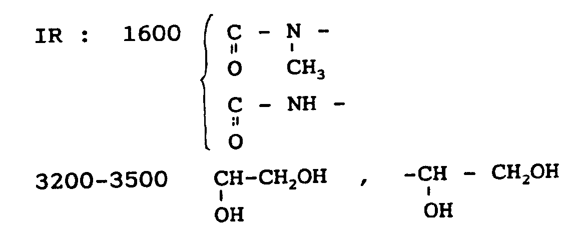 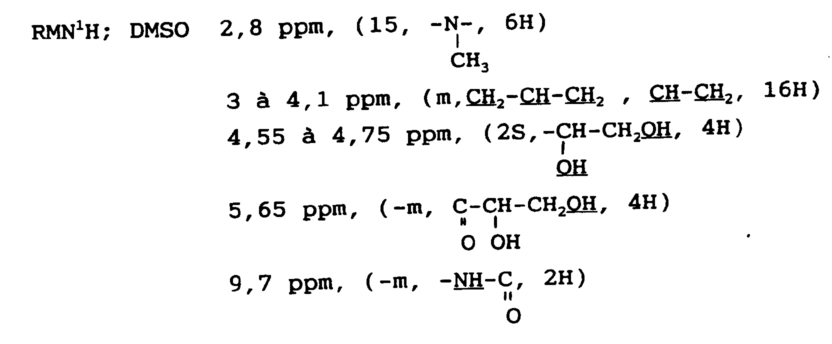 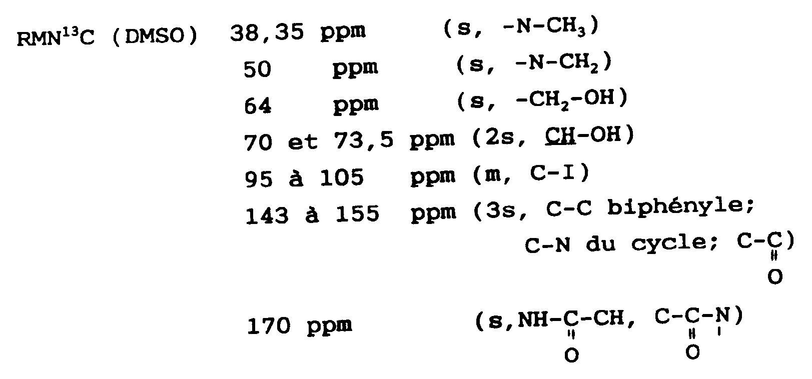
**EXEMPLE 12** Préparation du composé de formule 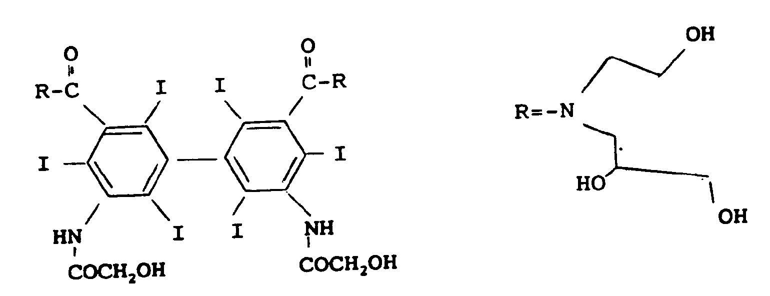
   1 - Préparation du composé de formule : 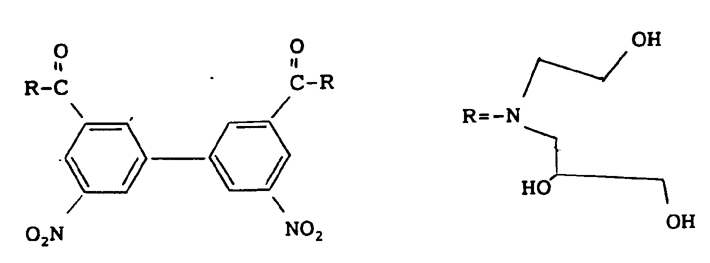
      a) Synthèse du composé de formule : 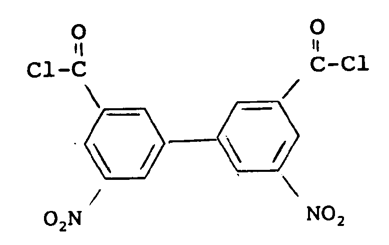
         9,9 g (0,03 mole) du composé obtenu dans l'exemple 8-4) sont ajoutés à une solution de 100 ml de SOCl₂ et 0,1 ml de diméthylformamide. La solution est portée au reflux pendant 5h. Après évaporation du SOCl₂, la pâte obtenue est dissoute dans le dioxanne.
      b) amidification
         Sur 12,15 g (0.09 mole) de 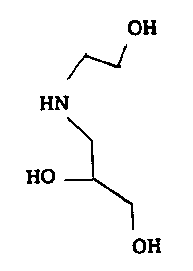 obtenue selon le procédé décrit dans WO 9109007 et 21,4 ml (0,09 mole) de tributylamine dissous à chaud dans 100 ml de dioxanne est versé goutte à goutte à 80°C le produit obtenu en a). Le mélange est agité pendant une nuit à température ambiante. La solution surnageante est décantée et le résidu pâteux est lavé avec CH₂Cl₂ puis à l'éther. On obtient 20 g du composé du titre.
         CCM (CH₂Cl₂/MeOH:75/25) rf:0,7 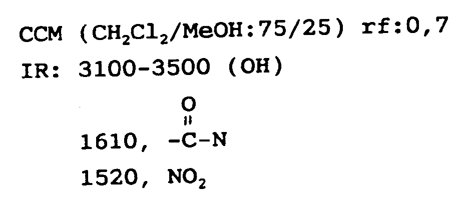 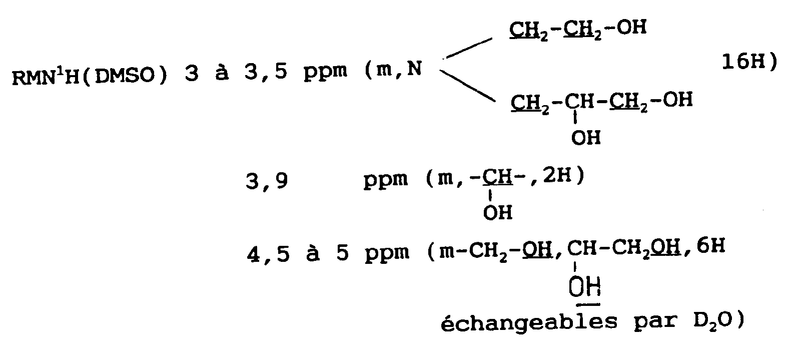 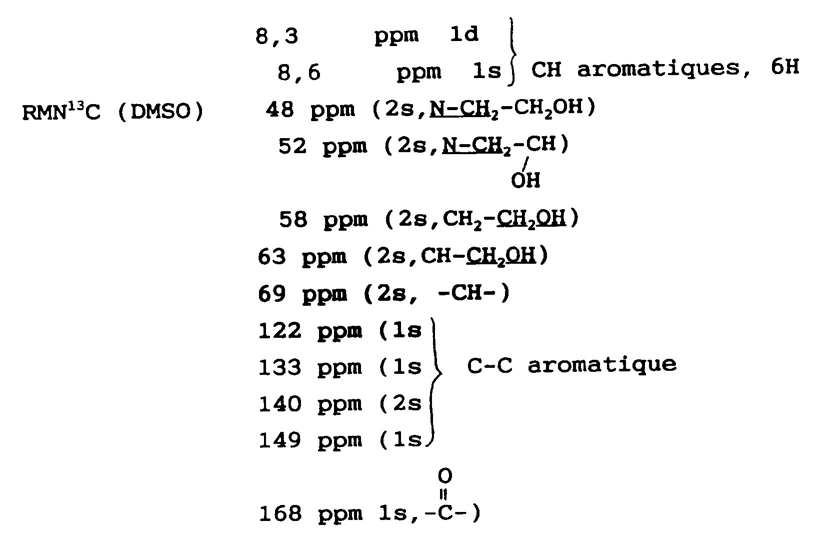
   2 - Préparation du composé de formule : 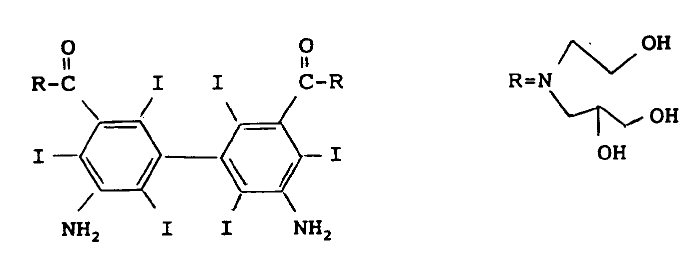
      a) Réduction des groupes nitro :
         Dans un autoclave de 500 ml, 0,03 mole du composé obtenu en 1) dissous dans 300 ml de méthanol, en présence d'1 g de Pd/C à 10% sont agités pendant 5 h à 50°C sous pression d'hydrogène de 6.10⁵Pa. après filtration du catalyseur et évaporation du solvant, on obtient une pâte.
         CCM(CH₂Cl₂/MeOH 50/50) Rf : 0,2
      b) Iodation par ICl :
         A la pâte obtenue en a) dissoute dans 500 ml de méthanol sont ajoutés goutte à goutte 53 ml (0,3 mole) d'une solution d'ICl à 70%. Le mélange réactionnel est chauffé à 60°C pendant 12 h. Après refroidissement, la solution est versée dans de l'eau additionnée de glace. Le précipité obtenu est essoré et lavé par de l'eau bisulfitée.
         Le produit ainsi obtenu est redissous dans le méthanol (V=300 ml) et 10,6 ml (0,06 mole) d'une solution d'ICl à 70% sont ajoutés goutte à goutte. Le mélange est agité pendant 12 h à 60°C, puis versé sur de la glace, et le précipité formé est lavé par de l'eau bisulfitée. Le produit est repris dans l'éther puis séché. On obtient 23 g de cristaux beiges.
         Rendement : 62%
         Pureté en iode : 98%
         CCM (CH₂Cl₂/MeOH:75/25):Rf=0,4 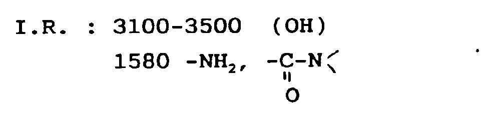 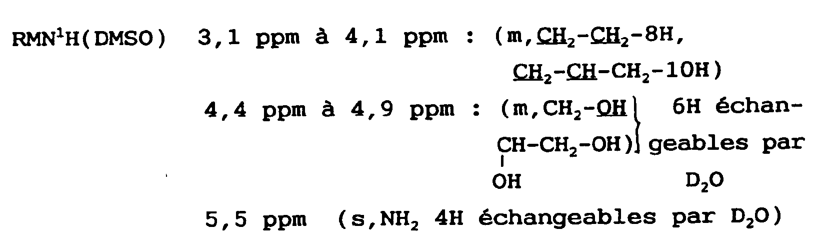
   3 - Préparation du composé de formule : 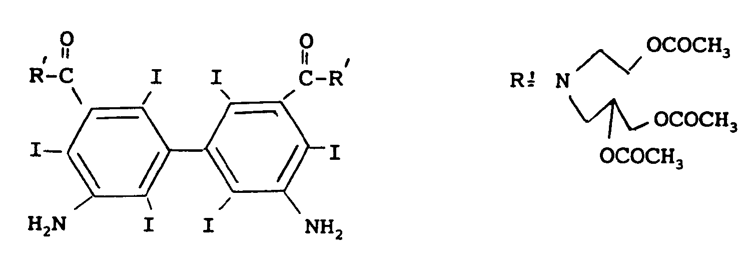
      A 23 g (0,0182 mole) du composé obtenu en 2) dissous dans 280 ml de pyridine sont additionnés goutte à goutte à 5°C, 30 ml d'anhydride acétique. Après 18 h d'agitation à température ambiante, le milieu réactionnel est versé dans de l'eau glacée acidifiée. Le précipité formé est essoré puis repris dans CH₂Cl₂. La phase organique est lavée à l'eau, séchée sur MgSO₄ et purifiée par chromatographie sur silice.
      Après évaporation, on obtient 11 g de cristaux blancs.
      Rdt : 50%
      CCM (acétate d'éthyle/heptane:9/1) Rf:0,6
      Pureté en iode : 97% 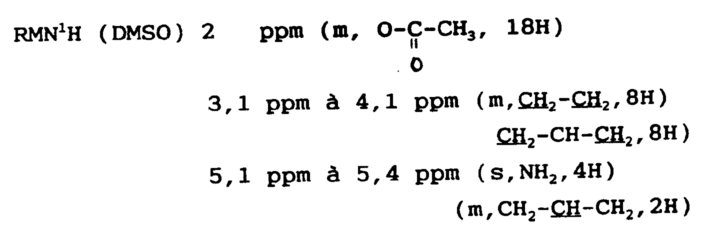
   4 - Préparation du composé de formule : 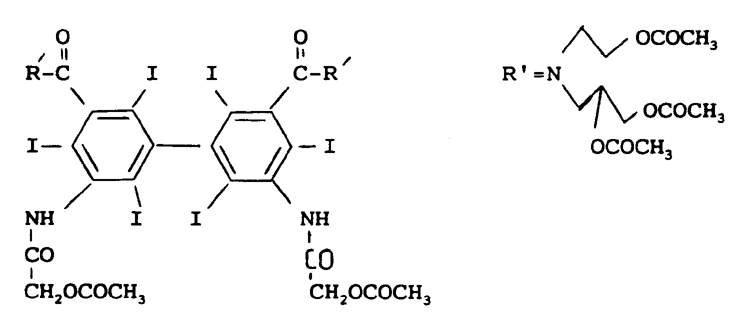
      a) Préparation de 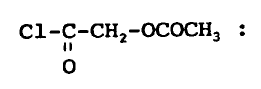
         88 g (0,745 mole) de COOH-CH₂OCOCH₃ sont ajoutés à une solution de 200 ml de SOCl₂, puis portés au reflux pendant 5 heures.
         Après évaporation du SOCl₂, 70 g de produit sont récupérés par distillation.
         Rendement : 70%.
      b) Acylation :
         A 22 g (0,0145 mole) du composé obtenu en 3) dissous dans 150 ml de DMAC anhydre sont additionnés goutte à goutte 10 g (0,073 mole) du chlorure d'acide obtenu en 4a). Le mélange est porté à 60°C pendant 5 h, puis versé sur de la glace. Le précipité formé est repris au CH₂Cl₂ et lavé par H₂O, purifié par chromatographie sur silice puis concrétisé dans l'éther isopropylique. On obtient 14 g de cristaux blancs.
         Rendement : 60%
         Pureté en iode : 99%
         CCM (acétate d'éthyle) Rf : 0,8 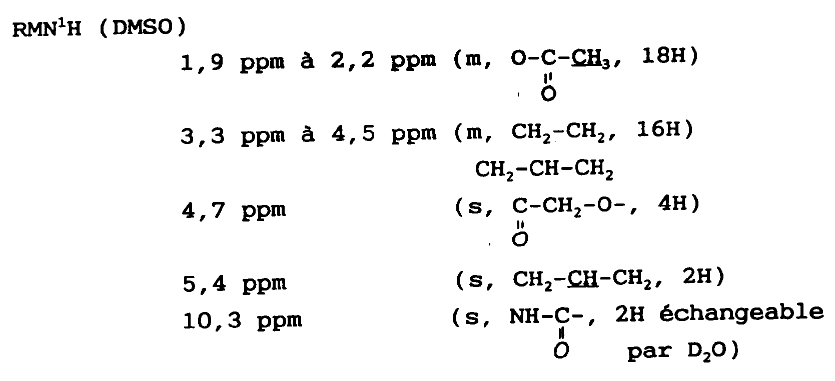
   5 - Préparation du composé de formule : 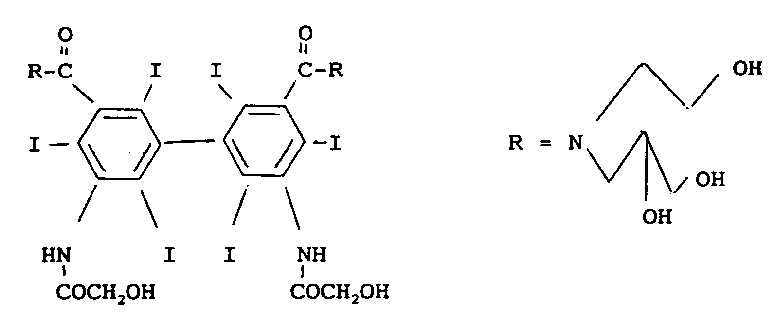
      14 g (0,00816 mole) du composé obtenu en 4b) dissous dans 140 ml de méthanol et 1,4 g de K₂CO₃ sont agités pendant 18 h à température ambiante. Après filtration et évaporation les cristaux blancs obtenus sont purifiés sur résine H⁺ et chromatographiés sur silice. On obtient 8 g de produit.
      Rendement : 80%
      CCM (CH₂Cl₂/MeOH 60/40) : Rf = 0,4
      I.R. : 3100 -3500 - R-OH

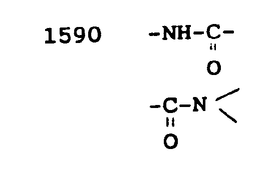

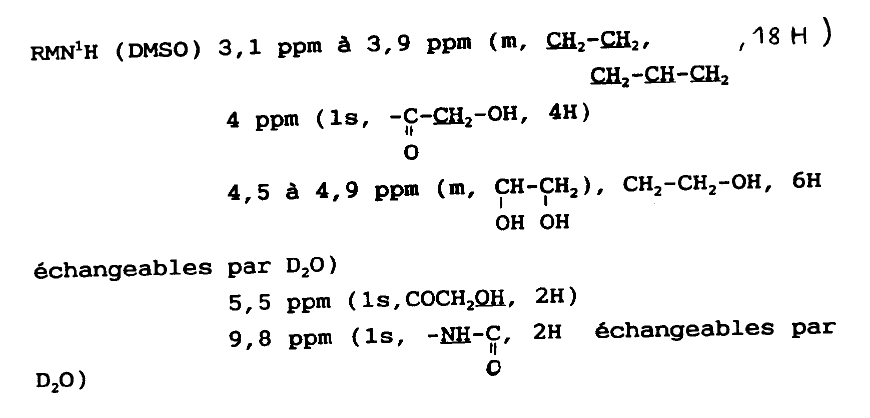

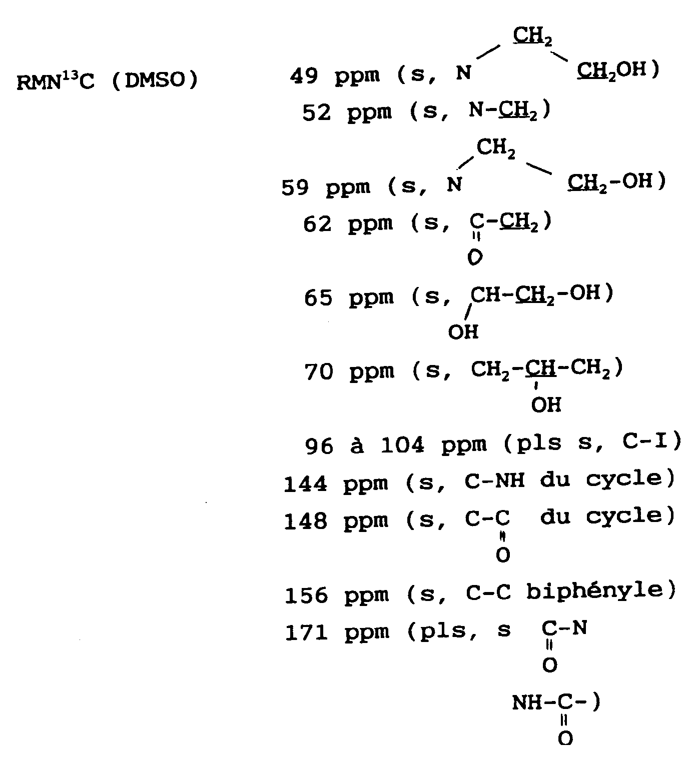

### EXEMPLE 13

Préparation du composé de formule :

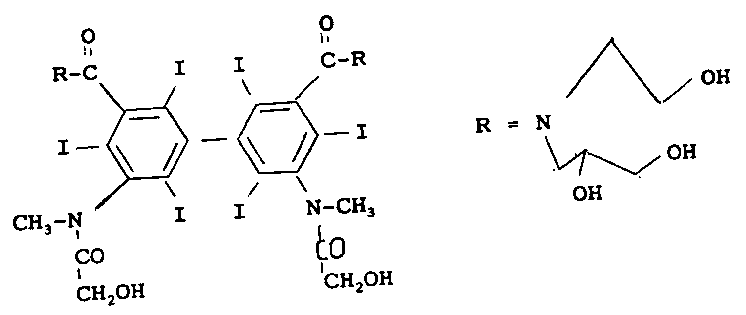

1 g (0,000725 mole) du composé obtenu à l'exemple 12-5) est dissous dans 6 ml de méthanol anhydre contenant 0,0015 mole de méthylate de sodium. Après 1 heure d'agitation, 0,17 ml (0,002 mole) d'iodure de méthyle est ajouté goutte à goutte, puis la solution est portée à 35-40°C pendant 3 heures. Après évaporation du solvant, le résidu est concrétisé dans l'éther, lavé à l'acétone et purifié par chromatographie sur silice. On obtient 600 mg de cristaux blancs.
Rendement : 70%
Pureté en iode : 99%
CCM(CH₂Cl₂/MeOH/NH₄OH:60/30/10):Rf : 0,3

### EXEMPLE 14

Préparation du composé de formule

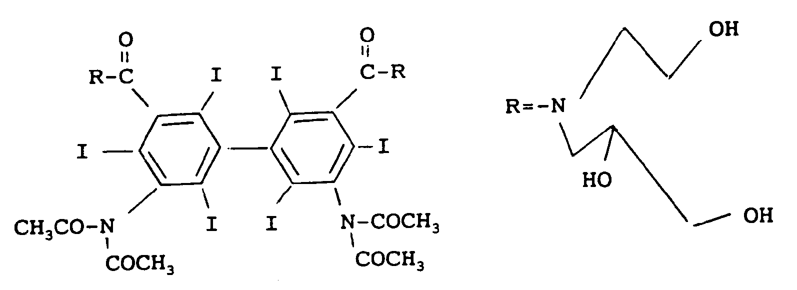

7,1 g (4,7 mmoles) du composé obtenu à l'exemple 12-2b) sont ajoutés à une solution de chlorure d'acétyle (1,2 cm³) (0,0165 mmole) dans 80 cm³ de DMAC. Le milieu réactionnel est porté à 60°C pendant 72 h. Après retour à température ambiante, le milieu réactionnel est précipité dans un mélange eau + glace.

Le produit brut (7,2 g; Rdt 96%) obtenu après filtration et concrétisation dans l'heptane est purifié sur colonne de silice. On obtient 5,8 g de produit.
Rendement : 77 %
CCM (CH₂Cl₂MeOH 90/10) Rf : 0,7
Dosage d'iode : 98,4%
Pureté HPLC : 97% Lichrosphère C₁₈,5»,25cm
CH₃Cn 80,50
H₂O 20,50
- RMN¹H, DMSO, 200 MHz: 5,2 à 5,4 ppm (m); CH(2H);4 à 4,4 ppm(m);
CH₂-OAc (8H)
3,7 à 4 ppm (m) CONCH₂CH₂OAc (8H); 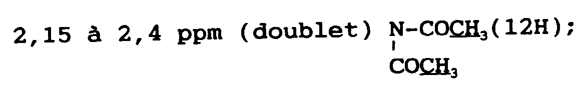 1,8 à 2,2 ppm (s) -OCOCH₃ (18H)

### EXEMPLE 15

Préparation du composé de formule :

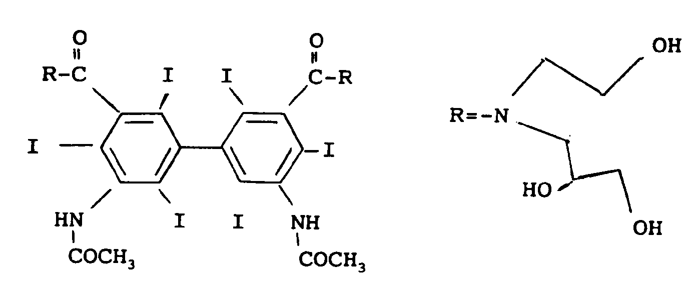

7,5 g (4,45 mmoles) du composé obtenu à l'exemple 14 sont ajoutés à une solution de soude 5N (5cm³) dans un mélange méthanol-eau (30/15). Le milieu réactionnel est agité à 50°C pendant 2 h et à température ambiante pendant une nuit.

Après concentration et purification du milieu réactionnel sur SiO₂, la déminéralisation s'effectue par passages sur résines H⁺ et OH⁻. Après concentration de la phase aqueuse, on obtient 3,2 g d'un solide blanc (Rdt 54%)
Dosage d'iode : 98, 5%
Pureté HPLC:99,9% Lichosphère C₁₈,25cm,5»
M₂O 50
CH₃CN 50

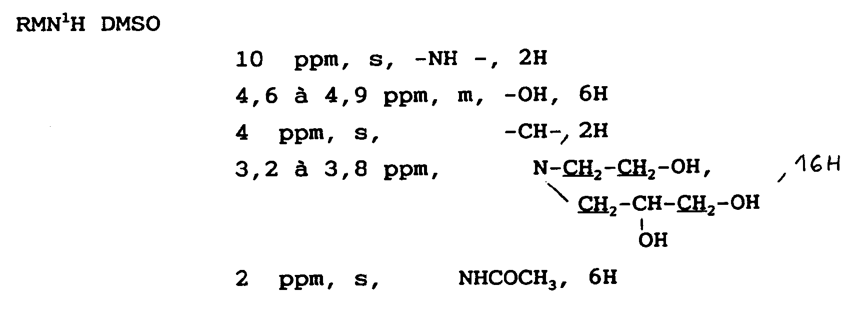

### EXEMPLE 16

Préparation du composé de formule :

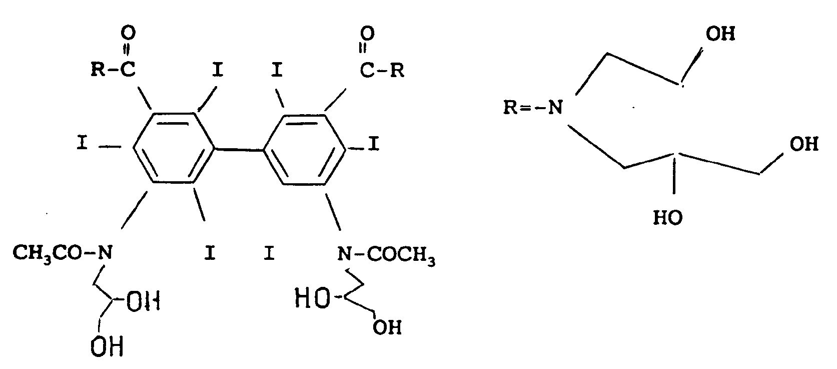

404 mg (3 mmoles) du produit préparé à l'exemple 15 sont solubilisés dans 3 cm³ de méthanol.

1,7 cm³ (8,4 mmoles) d'une solution de méthylate de sodium 5N et 1,25 cm³ (15 mmoles) de 3- chloro-1,2 propanediol sont ajoutés simultanément goutte à goutte.

Le milieu réactionnel est agité pendant 7 jours à 35°C.

Après neutralisation avec HCl 1N et déminéralisation sur résines H⁺ et OH⁻^{,} le produit du titre est obtenu.
CCM:CH₂Cl₂,MeOH 40/60 ; Rf : 0,3
Pureté HPLC : 97,7%
Colonne R sil NH₂ 25F, 5»m, 25cm
CH₃CN 75
H₂O 25

## Revendications

1. Composés poly-iodés de formule : 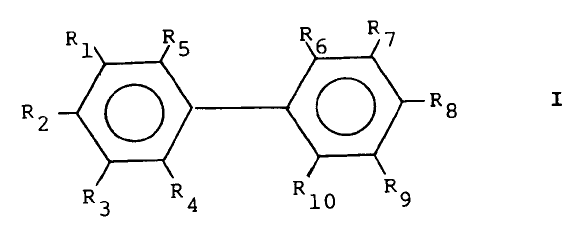 dans laquelle R₁, R₂, R₃, R₄, R₅, R₆, R₇, R₈, R₉ et R₁₀ identiques ou différents entre eux sont choisis parmi un atome d'iode, un groupe de formule 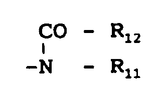 dans laquelle R₁₁ et R₁₂ identiques ou différents entre eux représentent un atome d'hydrogène, un groupe alkyle en C₁-C₆ linéaire ou ramifié, un groupe hydroxy- ou polyhydroxy-alkyle en C₁-C₆ linéaire ou ramifié, comportant en outre éventuellement un ou plusieurs groupes alcoxy en C₁-C₆, un groupe alkoxy (en C₁-C₆) alkyle en C₁-C₆ linéaire ou ramifié ou un groupe hydroxy- ou polyhydroxy-alkoxy (en C₁-C₆) alkyle en C₁-C₆ linéaire ou ramifié comportant de deux à cinq groupes - OH, un groupe de formule 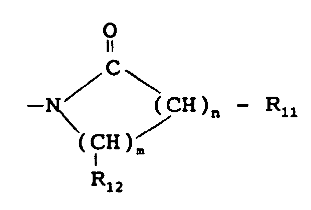 dans laquelle R₁₁, R₁₂ identiques ou différents sont tels que définis précédemment ou représentent un groupe hydroxy et n et m sont des entiers choisis de façon que n+m soit un nombre de 3 à 6, un groupe -COO⁻M⁺ ou -COOH, avec M⁺ représentant un cation physiologiquement acceptable minéral ou organique,
un groupe de formule 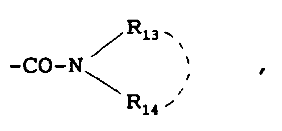 un groupe de formule 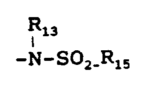 et un groupe de formule 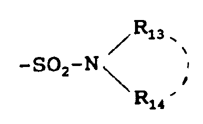 dans lesquelles R₁₃ et R₁₄ identiques ou différents entre eux représentent un atome d'hydrogène, un groupe alkyle en C₁-C₆ linéaire ou ramifié, un groupe hydroxy- ou polyhydroxy-alkyle en C₁-C₆ linéaire ou ramifié, comportant en outre éventuellement un ou plusieurs groupes alcoxy en C₁-C₆, un groupe alkoxy (en C₁-C₆) alkyle en C₁-C₆ linéaire ou ramifié ou un groupe hydroxy- ou polyhydroxy-alkoxy (en C₁-C₆) alkyle en C₁-C₆ linéaire ou ramifié comportant de deux à cinq groupes - OH, ou R₁₃ et R₁₄ forment ensemble un groupe alkylène en C₄-C₈, hydroxyalkylène en C₄-C₈ ou polyhydroxyalkylène en C₄-C₈, à chaîne linéaire ou ramifiée, de sorte que R₁₃ et R₁₄ forment avec l'atome d'azote auquel ils sont fixés un hétérocycle azoté à 5 ou 6 chaînons éventuellement substitué par un ou plusieurs groupes hydroxy, ou hydroxyalkyle en C₁-C₄
et R₁₅ a les mêmes significations que R₁₃ sauf un atome d'hydrogène,
sous réserve qu'au moins deux groupes parmi R₁, R₂, R₃, R₄, R₅, R₆, R₇, R₈, R₉ et R₁₀ représentent un atome d'iode.

2. Composés poly-iodés de formule générale I 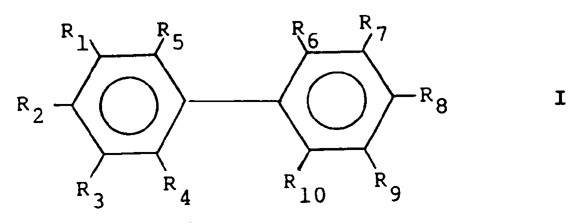 dans laquelle R₁, R₂, R₃, R₄, R₅, R₆, R₇, R₈, R₉ et R₁₀ identiques ou différents entre eux sont choisis parmi un atome d'iode, un groupe de formule 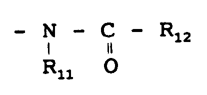 dans laquelle R₁₁ et R₁₂ identiques ou différents entre eux représentent un atome d'hydrogène, un groupe alkyle en C₁-C₆ linéaire ou ramifié, un groupe hydroxy- ou polyhydroxyalkyle en C₁-C₆ linéaire ou ramifié, comportant en outre éventuellement un ou plusieurs groupes alcoxy en C₁-C₆, un groupe alkoxyalkyle en C₁-C₆ linéaire ou ramifié, ou un groupe hydroxy ou polyhydroxyalkoxyalkyle en C₁-C₆, linéaire ou ramifié contenant de deux à cinq groupe -OH, et un groupe de formule 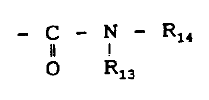 dans laquelle R₁₃ et R₁₄ identiques ou différents entre eux ont les mêmes significations que R₁₁ et R₁₂, sous réserve qu'au moins deux groupes parmi R₁, R₂, R₃, R₄, R₅, R₆, R₇, R₈, R₉ et R₁₀ représentent un atome d'iode.

3. Composés tétra-iodés de formule générale : 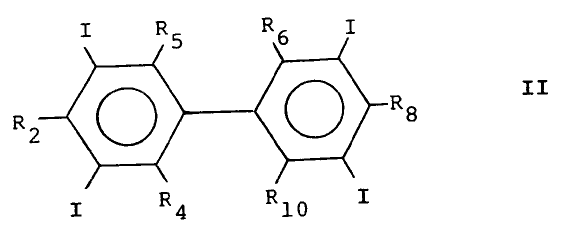 dans laquelle R₂, R₄, R₅, R₆, R₈ et R₁₀ sont tels que définis à la revendication 1 ou 2.

4. Composés poly-iodés de formule I selon la revendication 1 ou 2, dans laquelle le groupe 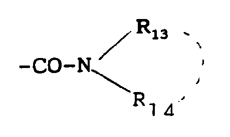 représente 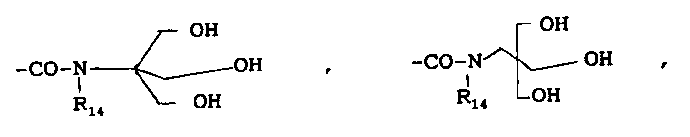 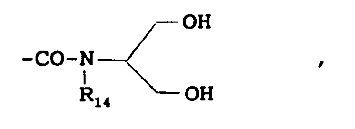 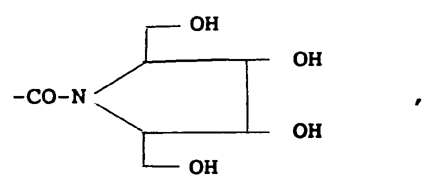 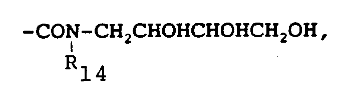 ou 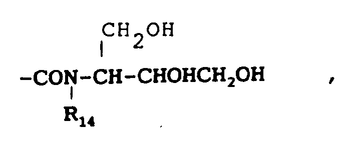 R₁₄ étant tel que défini à la revendication 1 ou 2.

5. Composés hexa-iodés de formule générale : 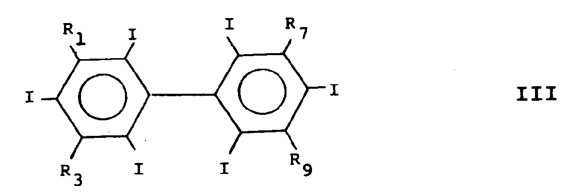 dans laquelle R₁, R₃, R₇, R₉ sont tels que définis à la revendication 1 ou 2.

6. Composés hexa-iodés selon la revendication 6 dans laquelle R₁ et R₉ sont identiques et représentent le groupe 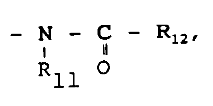 R₁₁ et R₁₂ étant tels que définis à la revendication 1 et R₃ et R₇ sont identiques et représentent le groupe 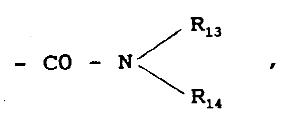 R₁₃ et R₁₄ étant tels que définis à la revendication 1.

7. Composés selon la revendication 3 choisi parmi les composés de formule II, dans laquelle
R₂ et R₈ représentent le groupe - CO - NH - CH₂ - CHOH - CH₂ OH et R₄, R₅, R₆ et R₁₀ représentent le groupe - NH - CO - CHOH - CH₃,
R₂ et R₈ représentent le groupe - NH - CO - CH₂OH et R₄, R₅, R₆ et R₁₀ représentent le groupe - CO - NH - CH₂ - CH₂OH,
R₂ et R₈ représentent le groupe 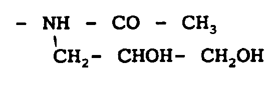 et R₄, R₅, R₆ et R₁₀ représentent le groupe - CO - NH -CH₂ - CH₂OH et R₂ et R₈ représentent le groupe - NH - CO - CH₂OH et R₄, R₅, R₆ et R₁₀ représentent le groupe - CO - NH - CH₂ - CHOH - CH₂OH.

8. Composé de formule II selon la revendication 3 dans laquelle R₂, R₅, R₆ et R₈ représentent le groupe -CO-NH-CH₂-CHOH-CH₂OH et R₄ et R₁₀ représentent le groupe - NH - CO - CH₂OH.

9. Composé de formule II selon la revendication 3 dans laquelle R₂, R₄, R₅, R₆, R₈ et R₁₀ représentent le groupe - NH - CO - CHOH - CH₃.

10. Composé de formule II selon la revendication 3 dans laquelle R₂, R₄, R₆ et R₈ représentent le groupe - CO - NH - CH₂ - CH₂OH et R₅ et R₁₀ représentent le groupe - NH - CO - CHOH - CH₃.

11. Composé selon la revendication 5 choisis parmi les composés de formule III, dans laquelle R₁ et R₇ représentent le groupe - NH - CO - CH (CH₂OH)₂ et R₃ et R₉ représentent le groupe 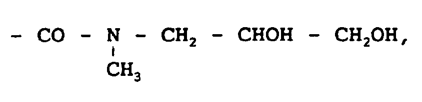 R₁ et R₇ représentent le groupe - NH - CO - CH (CH₂OH)₂ et R₃ et R₉ représentent le groupe - CO - N - (CH₂ - CHOH - CH₂OH)₂,
R₁ et R₇ représentent le groupe -NHCOCH₂OH et R₃ et R₉ représentent le groupe - CO -N CH₂-CHOH-CH₂OH, représentent le groupe 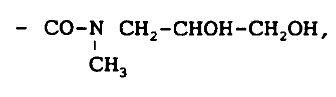 R₁ et R₇ représentent le groupe -NH-CO-CHOH-CH₂OH et R₃ et R₉ représentent le groupe 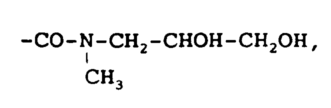 R₁ et R₇ représentent le groupe -NH-CO-CH₂OH et R₃ et R₉ représentent le groupe 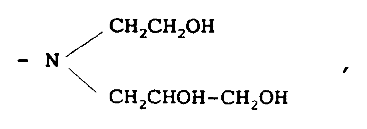 R₁ et R₇ représentent le groupe 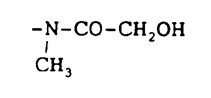 et R₃ et R₉ représentent le groupe 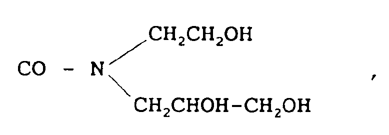 R₁ et R₇ représentent le groupe -N-(COCH₃)₂ et R₃ et R₉
R₁ et R₇ représentent le groupe -NH-CO-CH₃ et R₃ et R₉ représente le groupe 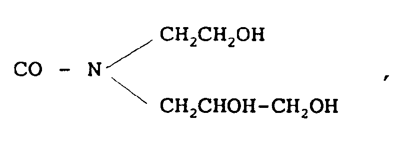 R₁ et R₇ représentent le groupe 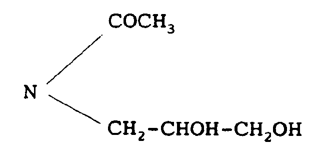 et R₃ et R₉ représentent le groupe

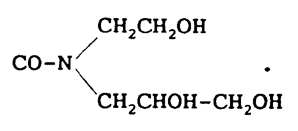

12. Procédé de préparation d'un composé de formule I selon la revendication 1 ou 2, caractérisé en ce que l'on prépare ces composés par des réactions d'acylation et/ou d'alkylation.

13. Procédé selon la revendication 12 de préparation des composés de formule I, caractérisé en ce que l'on effectue
a) un couplage de composés de formule 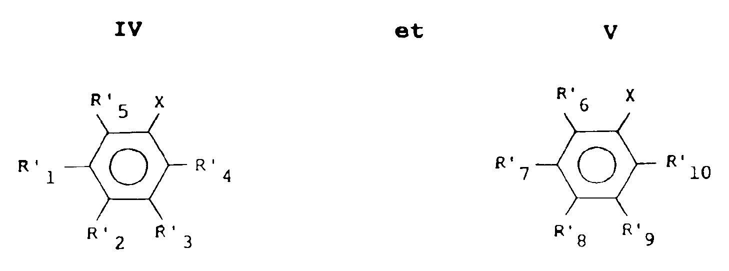 X étant choisi parmi le chlore, le brome et l'iode et R′₁₂, R′₂, R′₃, R′₄, R′₅, R′₆, R′₇, R′₈, R′₉ et R′₁₀ identiques ou différents étant choisis parmi les groupes NO₂ et - CO₂R, R étant un groupe alkyle en C₁-C₆ dans un solvant approprié en présence d'un catalyseur métallique pour obtenir un composé de formule VI : 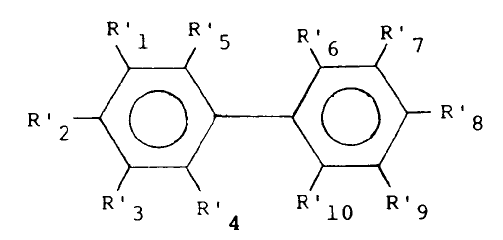
b) une amidification des groupes -COOR par une amine de formule 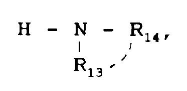 R₁₃ et R₁₄ étant tels que définis à la revendication 1,
c) une réduction des groupes nitro en amine :
d) une iodation dans des conditions classiques ;
e) éventuellement une protection des groupes hydroxy à l'aide des groupes protecteurs usuels ;
f) une acylation des groupements amino aromatique, en présence de chlorure d'acide de formule R′₁₂-COCl, R′₁₂ correspondant à R₁₂ tel que défini à la revendication 1 dont les groupes OH sont protégés, et soit ;
g) éventuellement une alkylation des groupes amido en présence d'une base forte et d'un réactif alkylant de formule Z - R₁₁, Z étant un groupe labile choisi parmi Cl, Br et I et R₁₁ étant tel que défini à la revendication 1 ; et une déprotection des fonctions hydroxy protégées, soit
h) une déprotection des fonctions hydroxy protégées et éventuellement une alkylation des groupes amido par un réactif alkylant de formule Z - R₁₁, Z étant un groupe labile choisi parmi Cl, Br et I et R₁₁ étant tel que défini à la revendication 1.

14. Procédé selon la revendication 12, de préparation des composés de formule II dans laquelle R₂, R₄, R₅, R₆, R₈, et R₁₀ représentent un groupe 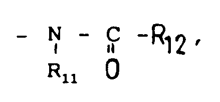 R₁₁ et R₁₂ étant tels que définis à la revendication 1, caractérisé en ce que l'on effectue
a) un couplage d'un composé de formule VII 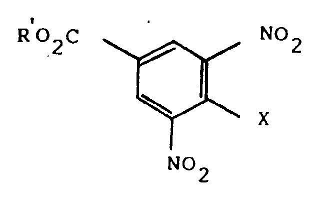 R′ représentant un groupe méthyle ou éthyle, et X étant choisi parmi un atome de chlore, de brome et d'iode, pour obtenir un composé de formule VIII 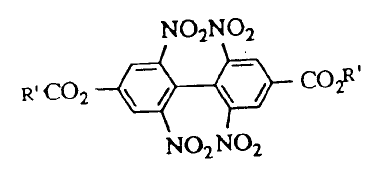
b) après saponification des esters et hydrolyse, un réarrangement pour obtenir une diamine de formule IX 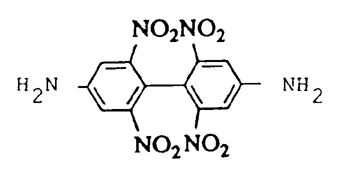
c) une acylation des groupements amino en présence d'un chlorure d'acide de formule R′₁₂ - COCl, R′₁₂ correspondant à R₁₂ tel que défini à la revendication 1 dont les groupes - OH sont protégés ou non ;
d) une réduction des groupes nitro en amino;
e) une iodation des groupes amino aromatique dans des conditions classiques ;
f) une acylation par un chlorure d'acide de formule R′₁₂ -COCl, R′₁₂ correspondant à R₁₂ tel que défini à la revendication 1 dont les groupes - OH sont protégés ;
g) éventuellement une déprotection ; et
h) éventuellement une alkylation des groupes amido par un réactif alkylant de formule Z - R₁₁, Z étant un groupe labile choisi parmi le chlore, le brome et l'iode et R₁₁ étant tel que défini à la revendication 1.

15. Procédé de préparation de composés de formule III selon la revendication 8, caractérisé en ce que l'on effectue :
a) une diazotation d'un composé de formule X : 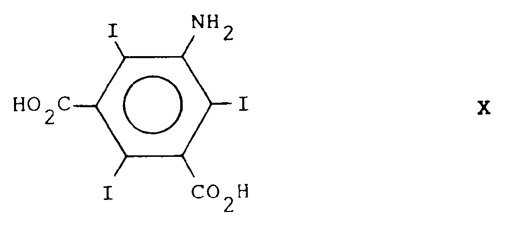 pour obtenir un composé de formule XI : 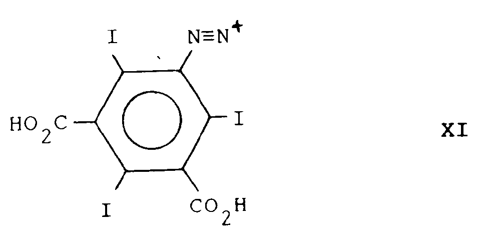
b) un couplage du composé de fomule XI pour obtenir un composé de formule XII : 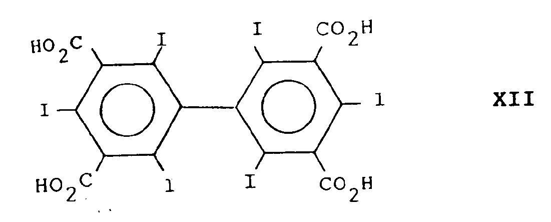
c) une transformation du composé de formule XII en son chlorure d'acide correspondant, et
d) une amidification du composé obtenu avec une amine de formule 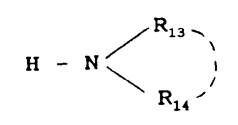 R₁₃ et R₁₄ étant tels que définis à la revendication 1 pour obtenir un composé de formule III.

16. Produit de contraste, caractérisé en ce qu'il contient au moins un composé selon l'une quelconque des revendications 1 à 12.

17. Produit de contraste selon la revendication 16 caractérisé en ce qu'il est constitué par une solution aqueuse du ou des composés.

18. Produit de contraste pour la radiologie par rayons X, caractérisé en ce qu'il contient au moins un composé selon l'une quelconque des revendications 1 à 11.

## Patentansprüche

1. Polyjodierte Verbindungen der Formel: 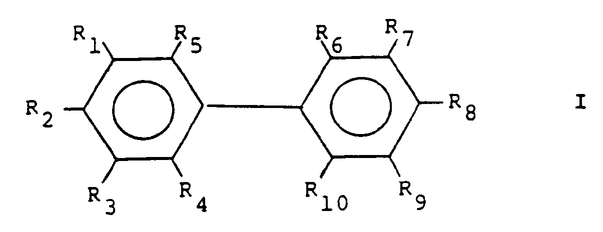 in der R₁, R₂, R₃, R₄, R₅, R₆, R₇, R₈, R₉ und R₁₀ untereinander gleich oder verschieden sind und ausgewählt sind aus einem Jodatom, einer Gruppe der Formel 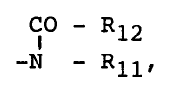 in der R₁₁ und R₁₂ untereinander gleich oder verschieden sind und ein Wasserstoffatom, eine verzweigte oder unverzweigte (C₁-C₆)-Alkylgruppe, eine verzweigte oder unverzweigte Hydroxy- oder Polyhydroxy- (C1-C6)-alkyl-Gruppe darstellen und außerdem ggf. eine oder mehrere (C₁-C₆)-Alkoxygruppen, eine verzweigte oder unverzweigte (C₁-C₆)-Alkoxy-(C₁-C₆)-alkyl-Gruppe oder eine verzweigte oder unverzweigte Hydroxy- oder Polyhydroxy-(C₁-C₆)-alkoxy-(C₁-C₆)-alkyl-Gruppe mit zwei bis fünf -OH-Gruppen umfassen, einer Gruppe der Formel 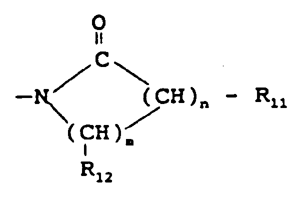 in der R₁₁, R₁₂ untereinander gleich oder verschieden sind und die oben angegebenen Bedeutungen haben oder eine Hydroxygruppe darstellen und n und m ganze Zahlen sind, die so gewählt sind, daß n+m eine Zahl von 3 bis 6 ist, einer -COO⁻M⁺ oder -COOH-Gruppe, wobei M⁺- ein physiologisch akzeptables mineralisches oder organisches Cation darstellt,
einer Gruppe der Formel 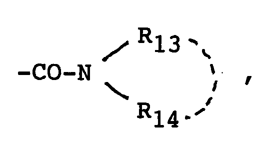 einer Gruppe der Formel 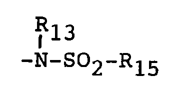 und einer Gruppe der Formel 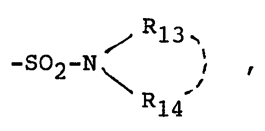 in denen R₁₃ und R₁₄ untereinander gleich oder verschieden sind und ein Wasserstoffatom, eine verzweigte oder unverzweigte (C₁-C₆)-Alkylgruppe, eine verzweigte oder unverzweigte Hydroxy- oder Polyhydroxy-(C₁-C₆)-alkylgruppe und außerdem ggf. eine oder mehrere (C₁-C₆)-Alkoxygruppen, eine verzweigte oder unverzweigte (C₁-C₆)-Alkoxy-(C₁-C₆)-alkyl-Gruppe oder eine verzweigte oder unverzweigte Hydroxy- oder Polyhydroxy-(C₁-C₆)-alkoxy-(C₁-C₆)-alkyl-Gruppe mit zwei bis fünf -OH-Gruppen, oder R₁₃ und R₁₄ zusammen eine (C₄-C₈)-Alkylengruppe, eine Hydroxy-(C₄-C₈)-alkylen-Gruppe oder Polyhydroxy-(C₄-C₈)-alkylengruppe in einer verzweigten oder unverzweigten Kette bilden, so daß R₁₃ und R₁₄ mit dem Stickstoffatom, an das sie fixiert sind, einen nitrierten Heterozyklus mit 5 oder 6 Gliedern bilden, der ggf. durch ein oder mehrere Hydroxy- oder Hydroxy-(C₁- C₄)-alkylgruppen substituiert ist,
und R₁₅ dieselben Bedeutungen wie R₁₃ hat, abgesehen von einem Wasserstoffatom,
sofern wenigstens zwei Gruppen aus R₁, R₂, R₃, R₄, R₅, R₆, R₇, R₈, R₉ und R₁₀ ein Jodatom darstellen.

2. Polyjodierte Verbindungen der allgemeinen Formel I 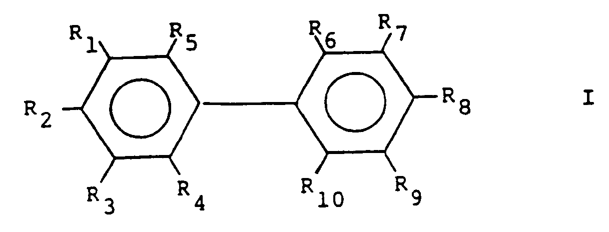 in der R₁, R₂, R₃, R₄, R₅, R₆, R₇, R₈, R₉ und R₁₀ untereinander gleich oder verschieden sind und ausgewählt sind aus einem Jodatom, einer Gruppe der Formel 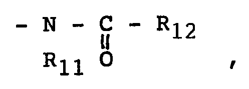 in der R₁₁ und R₁₂ untereinander gleich oder verschieden sind und ein Wasserstoffatom, eine verzweigte oder unverzweigte (C₁-C₆)-Alkylgruppe, eine verzweigte oder unverzweigte Hydroxy- oder Polyhydroxy-(C₁-C₆)-alkyl-Gruppe darstellen und außerdem ggf. eine oder mehrere (C₁-C₆)-Alkoxygruppen, eine verzweigte oder unverzweigte (C₁-C₆)-Alkoxyalkylgruppe oder eine verzweigte oder unverzweigte Hydroxy- oder Polyhydroxy-(C₁-C₆)-alkoxyalkylgruppe mit zwei bis fünf -OH-Gruppen umfassen, und eine Gruppe der Formel 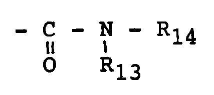 in der R₁₃ und R₁₄ untereinander gleich oder verschieden sind und dieselben Bedeutungen wie R₁₁ und R₁₂ haben, sofern wenigstens zwei Gruppen unter R₁, R₂, R₃, R₄, R₅, R₆, R₇, R₈, R₉ und R₁₀ ein Jodatom darstellen.

3. Tetrajodierte Verbindungen der allgemeinen Formel 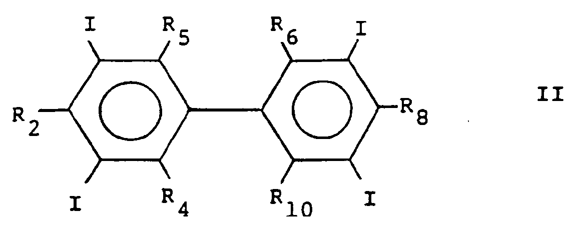 in der R₂, R₄, R₅, R₆, R₈ und R₁₀ die in Anspruch 1 oder 2 angegebenen Bedeutungen haben.

4. Polyjodierte Verbindungen der Formel I nach Anspruch 1 oder 2, in der die Gruppe 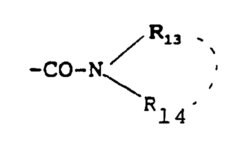 bedeutet: 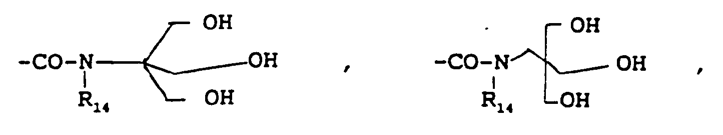 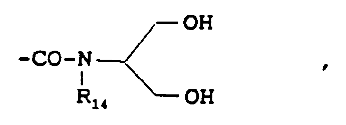 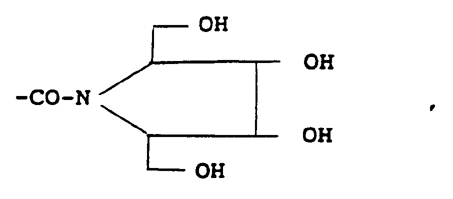 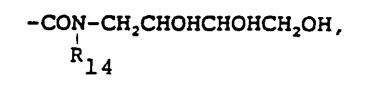 oder 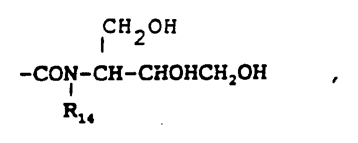 wobei R₁₄ die in Anspruch 1 oder 2 angegebenen Bedeutungen hat.

5. Hexajodierte Verbindungen der allgemeinen Formel: 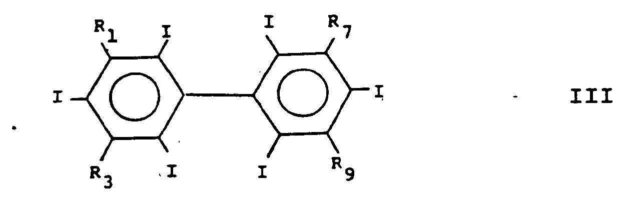 in der R₁, R₃, R₇, R₉ die in Anspruch 1 oder 2 angegebenen Bedeutungen haben.

6. Hexajodierte Verbindungen nach Anspruch 6, worin R₁ und R₉ gleich sind und die Gruppe 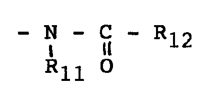 darstellen, R₁₁ und R₁₂ die in Anspruch 1 angegebenen Bedeutungen haben und R₃ und R₇ gleich sind und die Gruppe 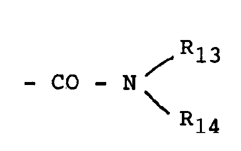 darstellen, wobei R₁₃ und R₁₄ die im Anspruch 1 angegebenen Bedeutungen haben.

7. Verbindungen nach Anspruch 3, ausgewählt aus den Verbindungen der Formel II, in der
R₂ und R₈ die Gruppe - CO - NH - CH₂ - CHOH - CH₂ OH darstellen und R₄, R₅, R₆ und R₁₀ die Gruppe - NH - CO - CHOH - CH₃ darstellen,
R₂ und R₈ die Gruppe - NH - CO - CH₂OH darstellen und R₄, R₅, R₆ und R₁₀ die Gruppe - CO - NH - CH₂ - CH₂OH darstellen,
R₂ und R₈ die Gruppe 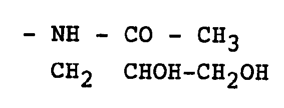 darstellen
und R₄, R₅, R₆ und R₁₀ die Gruppe - CO - NH - CH₂ - CH₂OH darstellen und R₂ und R₈ die Gruppe - NH - CO - CH₂OH darstellen und R₄, R₅, R₆ und R₁₀ die Gruppe - CO - NH - CH₂ - CHOH - CH₂OH darstellen.

8. Verbindung der Formel II nach Anspruch 3, in der R₂, R₅, R₆ und R₈ die Gruppe - CO - NH - CH₂ - CHOH - CH₂OH darstellen und R₄ und R₁₀ die Gruppe - NH - CO - CH₂OH darstellen.

9. Verbindung der Formel II nach Anspruch 3, in der R₂, R₄, R₅, R₆, R₈ und R₁₀ die Gruppe - NH - CO - CHOH - CH₃ darstellen.

10. Verbindung der Formel II nach Anspruch 3, in der R₂, R₄, R₆ und R₈ die Gruppe - CO - NH - CH₂ - CH₂OH darstellen und R₅ und R₁₀ die Gruppe - NH - CO - CHOH - CH₃ darstellen.

11. Verbindungen nach Anspruch 5, ausgewählt aus den Verbindungen der Formel III, in der
R₁ und R₇ die Gruppe - NH - CO - CH (CH₂OH)₂ und R₃ und R₉ die Gruppe 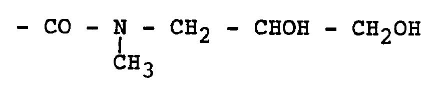 darstellen
R₁ und R₇ die Gruppe - NH - CO - CH (CH₂OH)₂ darstellen
und R₃ und R₉ die Gruppe - CO - N - (CH₂ - CHOH - CH₂OH)₂ darstellen,
R₁ und R₇ die Gruppe - NHCOCH₂OH darstellen und R₃ und R₉ die Gruppe 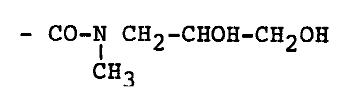 darstellen,
R₁ und R₇ die Gruppe -NH-CO-CHOH-CH₂OH darstellen und R₃ und R₉ die Gruppe 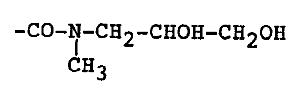 darstellen,
R₁ und R₇ die Gruppe -NH-CO-CH₂OH darstellen und R₃ und R₉ die Gruppe 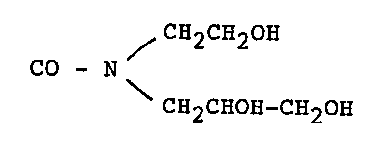 darstellen,
R₁ und R₇ die Gruppe 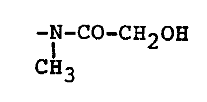 darstellen und
R₃ und R₉ die Gruppe 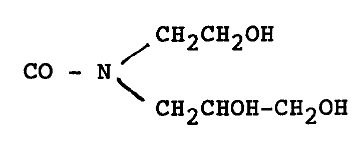 darstellen,
R₁ und R₇ die Gruppe -N-(COCH₃)₂ darstellen und R₃ und R₉ R₁ und R₇ die Gruppe -NH-CO-CH₃ darstellen und R₃ und R₉ die Gruppe 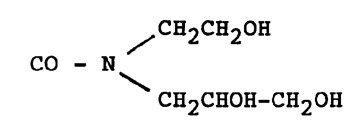 darstellen,
R₁ und R₇ die Gruppe 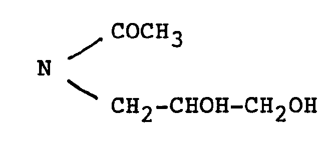 darstellen,
und R₃ und R₉ die Gruppe 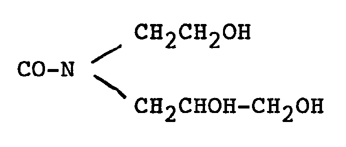 darstellen.

12. Verfahren zur Herstellung einer Verbindung der Formel I nach Anspruch 1 oder 2, dadurch gekennzeichnet, daß diese Verbindungen durch Acylierungs- und/oder Alkylierungsreaktionen hergestellt werden.

13. Verfahren nach Anspruch 12 zur Herstellung der Verbindungen der Formel I, gekennzeichnet durch
a) ein Kopplung der Verbindungen der Formel 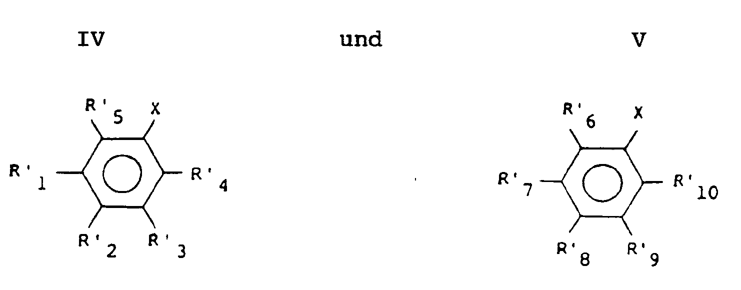 wobei X ausgewählt ist aus Chlor, Brom und Jod und R′₁₂, R′₂, R′₃, R′₄, R′₅, R′₆, R′₇, R′₈, R′₉ und R′₁₀ gleich oder verschieden sind und ausgewählt sind aus den Gruppen NO₂ und - CO₂R, wobei R eine (C₁-C₆)-Alkylgruppe ist, in einem geeigneten Lösungsmittel unter Gegenwart eines metallischen Katalysators, um eine Verbindung der Formel VI zu erhalten: 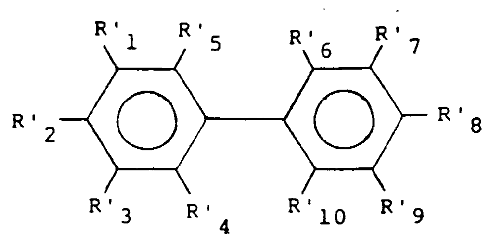
b) eine Amidierung der Gruppen -COOR durch ein Amin der Formel 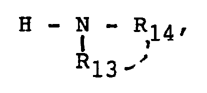 wobei R₁₃ und R₁₄ die Bedeutungen von Anspruch 1 haben,
c) eine Reduzierung der Nitrogruppen zu Amin;
d) eine Jodierung unter gebräuchlichen Bedingungen;
e) ggf. einen Schutz der Hydroxygruppen durch gebräuchliche Schutzgruppen;
f) eine Acylierung der aromatischen Aminogruppen in Anwesenheit von Säurechlorid der Formel R′₁₂-COCl, wobei R′₁₂ R₁₂ entspricht, wie es in Anspruch 1 definiert ist, deren OH-Gruppen geschützt sind, und entweder
g) ggf. eine Alkylierung der Amido-Gruppen in Anwesenheit einer starken Base und eines alkylierenden Reagenz der Formel Z - R₁₁, in der Z eine aus Cl, Br und I ausgewählte unbeständige Gruppe ist und R₁₁ die in Anspruch 1 angegebenen Bedeutungen hat; und eine Entfernung des Schutzes der geschützten Hydroxy-Funktionen, oder
h) eine Entfernung des Schutzes der geschützten HydroxyFunktionen und ggf. eine Alkylierung der Amido-Gruppen durch ein alkylierendes Reagenz der Formel Z - R₁₁, in der Z eine aus Cl, Br und I ausgewählte unbeständige Gruppe ist und R₁₁ die in Anspruch 1 angegebenen Bedeutungen hat.

14. Verfahren nach Anspruch 12 zur Herstellung der Verbinddungen der Formel II, in der R₂, R₄, R₅, R₆, R₈ und R₁₀ eine Gruppe 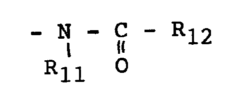 darstellen, wobei R₁₁ und R₁₂ die in Anspruch 1 genannten Bedeutungen haben, dadurch gekennzeichnet, daß folgende Schritte durchgeführt werden:
a) eine Kopplung einer Verbindung der Formel VII 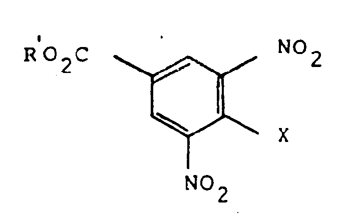 in der R′ eine Methyl- oder Ethylgruppe ist und X ausgewählt ist aus einem Chlor-, Brom- und Jodatom, um eine Verbindung der Formel VIII zu erhalten: 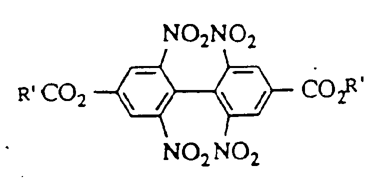
b) nach Verseifung der Erster und hydrolyse eine Umaordnung, um ein diamin der Formel IX zu erhalten 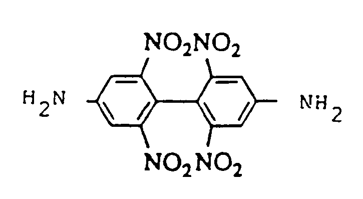
c) eine Acylierung der Aminogruppen in Anwesenheit eines säurechlorids der Formel R′₁₂ - COCl, worin R′₁₂ R₁₂ mit den in Anspruch 1 genannten Bedeutungen entspricht, deren -OH-Gruppen geschützt sind oder nicht;
d) eine Reduzierung der Nitrogruppen zu Aminogruppen;
e) eine Jodierung der aromatischen Aminogruppen unter gebräuchlichen Bedingungen;
f) eine Acylierung durch ein Säurechlorid der Formel R′₁₂ - COCl, worin R′₁₂ R₁₂ mit den in Anspruch 1 genannten Bedeutungen entspricht, deren -OH-Gruppen geschützt sind;
g) ggf. eine Entfernung des Schutzes; und
h) ggf. eine Alkylierung der Amidogruppen durch ein alkylierendes Reagenz der Formel Z - R₁₁, worin Z eine aus Chlor, Brom und Jod ausgewählte unbeständige Gruppe ist und R₁₁ die in Anspruch 1 genannten Bedeutungen hat.

15. Verfahren zur Herstellung von Verbindungen der Formel III nach Anspruch 8, dadurch gekennzeichnet, daß folgende Schritte durchgeführt werden:
a) eine Diazotierung einer Verbindung der Formel X: 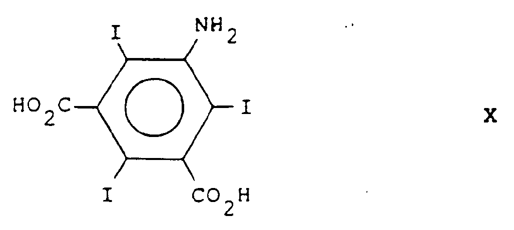 um eine Verbindung der Formel XI zu erhalten: 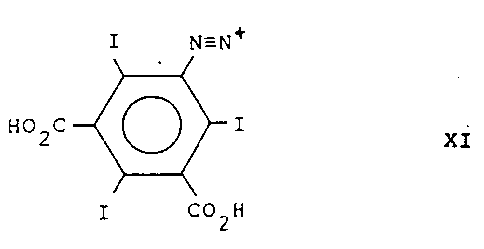
b) eine Kopplung der Verbindung der Formel XI, um eine Verbindung der Formel XII zu erhalten: 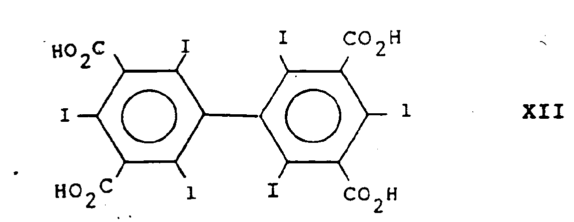
c) eine Umwandlung der Verbindung der Formel XII in ihr entsprechendes Säurechlorid und
d) eine Amidierung der erhaltenen Verbindung mit einem Amin der Formel 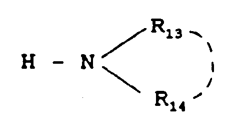
wobei R₁₃ und R₁₄ die in Anspruch 1 genannten Bedeutungen haben, um eine Verbindung der Formel III zu erhalten.

16. Kontrastprodukt, dadurch gekennzeichnet, daß es wenigstens eine Verbindung nach einem der Ansprüche 1 bis 12 enthält.

17. Kontrastprodukt nach Anspruch 16, dadurch gekennzeichnet, daß es aus einer wässrigen Lösung der Verbindung bzw. Verbindungen besteht.

18. Kontrastprodukt für die Röntgenradiologie, dadurch gekennzeichnet, daß es wenigstens eine Verbindung nach einem der Ansprüche 1 bis 11 enthält.

## Claims

1. Poly-iodinated compounds of formula: 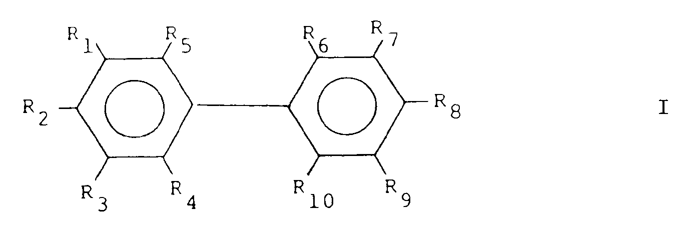 in which R₁, R₂, R₃, R₄, R₅, R₆, R₇, R₈, R₉ and R₁₀, identical or different, are selected from an iodine atom, a group of formula 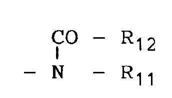 in which R₁₁ and R₁₂, identical or different, represent a hydrogen atom, a linear or branched C₁-C₆ alkyl group, a linear or branched C₁-C₆ hydroxy- or polyhydroxy-alkyl group, optionally containing in addition one or more C₁-C₆ alkoxy groups, a linear or branched C₁-C₆ alkoxy-C₁-C₆ alkyl group or a linear or branched hydroxy- or polyhydroxy- C₁-C₆ alkoxy-C₁-C₆ alkyl group containing from two to five -OH groups, a group of formula: 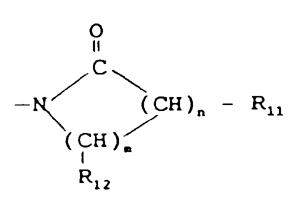 in which R₁₁, R₁₂, identical or different are as defined previously or represent a hydroxy group and n and m are integers selected so that n+m is a number from 3 to 6, a COOH⁻M⁺ or -COOH group, with M⁺ representing a physiologically acceptable mineral or organic cation, a group of formula 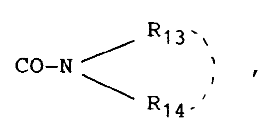 a group of formula 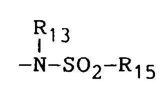 and a group of formula 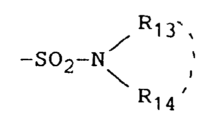 in which R₁₃ and R₁₄, identical or different, represent a hydrogen atom, a linear or branched C₁-C₆ alkyl group, a linear or branched C₁-C₆ hydroxy- or polyhydroxy-alkyl group, optionally containing in addition one or more C₁-C₆ alkoxy groups, a linear or branched C₁-C₆ alkoxy-C₁-C₆ alkyl group or a linear or branched hydroxy- or polyhydroxy-C₁-C₆-alkoxy-C₁-C₆ alkyl group containing from two to five -OH groups, or R₁₃ or R₁₄ together form a C₄-C₈ alkylene group, a C₄-C₈ hydroxyalkylene group or a C₄-C₈ polyhydroxyalkylene group, with a linear or branched chain such that R₁₃ and R₁₄ form with the nitrogen atom to which they are attached a 5- or 6-membered nitrogenous heterocycle optionally substituted by one or more hydroxy or C₁-C₄ hydroxyalkyl groups,
and R₁₅ has the same meanings as R₁₃ with the exception of a hydrogen atom,
provided that at least two of the R₁, R₂, R₃, R₄, R₅, R₆, R₇, R₈, R₉ and R₁₀ groups represent an iodine atom.

2. Poly-iodinated compounds of general formula I: 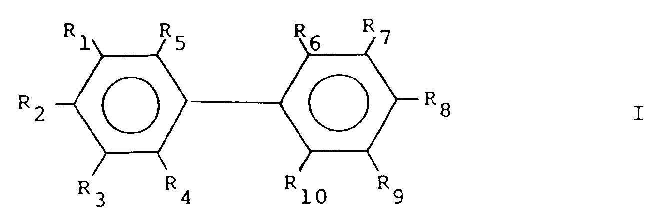 in which R₁, R₂, R₃, R₄, R₅, R₆, R₇, R₈, R₉ and R₁₀, identical or different, are selected from an iodine atom, a group of formula 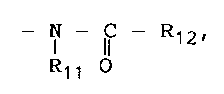 in which R₁₁ and R₁₂, identical or different represent a hydrogen atom, a linear or branched C₁-C₆ alkyl group, a linear or branched C₁-C₆ hydroxy- or polyhydroxy-alkyl group, optionally containing in addition one or more C₁-C₆ alkoxy groups, a linear or branched C₁-C₆ alkoxyalkyl group, or a linear or branched C₁-C₆ hydroxy- or polyhydroxy-alkoxyalkyl group containing from two to five -OH groups, and a group of formula 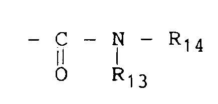 in which R₁₃ and R₁₄, identical or different, have the same meanings as R₁₁ and R₁₂, provided that at least two of the R₁, R₂, R₃, R₄, R₅, R₆, R₇, R₈, R₉ and R₁₀ groups represent an iodine atom.

3. Tetra-iodinated compounds of general formula: 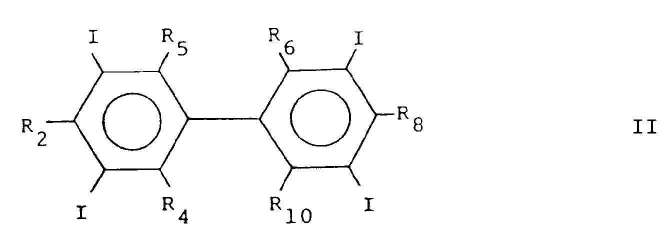 in which R₂, R₄, R₅, R₆, R₈ and R₁₀ are as defined in claim 1 or 2.

4. Poly-iodinated compounds of formula I according to claim 1 or 2, in which the group 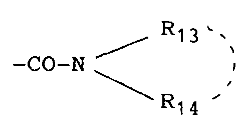 represents 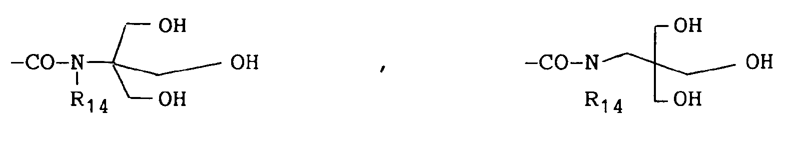 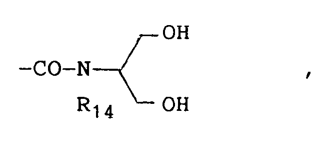 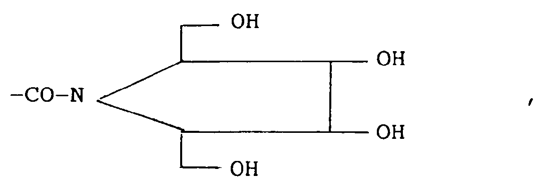 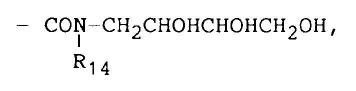 or 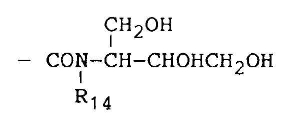 R₁₄ being as defined in claim 1 or 2.

5. Hexa-iodinated compounds of general formula: 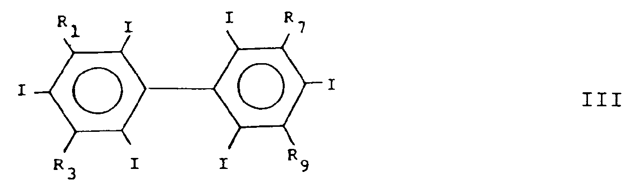 in which R₁, R₃, R₇, R₉ are as defined in claim 1 or 2.

6. Hexa-iodinated compounds according to claim 6 in which R₁ and R₉ are identical and represent the group 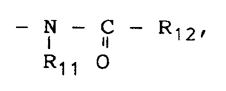 R₁₁ and R₁₂ being as defined in claim 1 and R₃ and R₇ are identical and represent the group 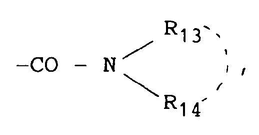 R₁₃ and R₁₄ being as defined in claim 1.

7. Compounds according to claim 3 chosen from the compounds of formula II in which
R₂ and R₈ represent the group - CO - NH - CH₂ - CHOH - CH₂- OH and R₄, R₅, R₆ and R₁₀ represent the group - NH - CO - CHOH - CH₃,
R₂ and R₈ represent the group - NH - CO - CH₂OH and R₄, R₅, R₆ and R₁₀ represent the group - CO -NH - CH₂ - CH₂OH,
R₂ and R₈ represent the group 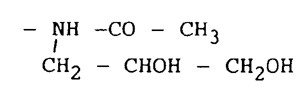 and R₄, R₅, R₆ and R₁₀ represent the group - CO -NH - CH₂ - CH₂OH
and R₂ and R₈ represent the group - NH - CO - CH₂OH and R₄, R₅, R₆ and R₁₀ represent the group - CO - NH - CH₂ - CHOH -CH₂OH.

8. Compound of formula II according to claim 3 in which R₂, R₅, R₆ and R₈ represent the group -CO-NH-CH₂-CHOH-CH₂OH and R₄ and R₁₀ represent the group - NH - CO - CH₂OH.

9. Compound of formula II according to claim 3 in which R₂, R₄, R₅, R₆, R₈ and R₁₀ represent the group - NH - CO - CHOH - CH₃.

10. Compound of formula II according to claim 3 in which R₂, R₄, R₆ and R₈ represent the group - CO - NH - CH₂ - CH₂OH and R₅ and R₁₀ represent the group - NH - CO - CHOH - CH₃.

11. Compounds according to claim 5 chosen from the compounds of formula III in which R₁ and R₇ represent the group - NH - CO - CH(CH₂OH)₂ and R₃ and R₉ represent the group 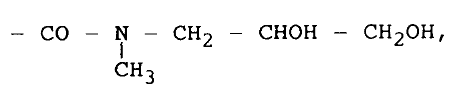 R₁ and R₇ represent the group - NH - CO - CH(CH₂OH)₂ and R₃ and R₉ represent the group - CO - N - (CH₂ - CHOH - CH₂OH)₂,
R₁ and R₇ represent the group -NHCOCH₂OH and R₃ and R₉ represent the group 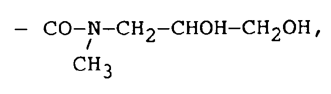 R₁ and R₇ represent the group -NH-CO-CHOH-CH₂OH and R₃ and R₉ represent the group 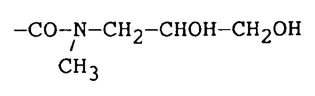 R₁ and R₇ represent the group -NH-CO-CH₂OH and R₃ and R₉ represent the group 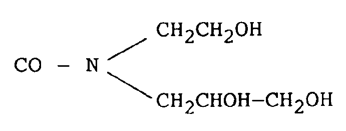 R₁ and R₇ represent the group 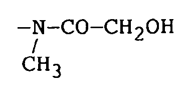 and R₃ and R₉ represent the group 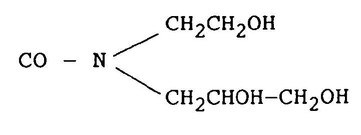 R₁ and R₇ represent the group -N-(COCH₃)₂ and R₃ and R₉
R₁ and R₇ represent the group -NH-CO-CH₃ and R₃ and R₉ represent the group 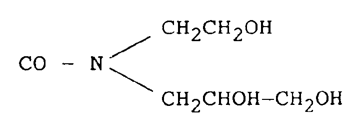 R₁ and R₇ represent the group 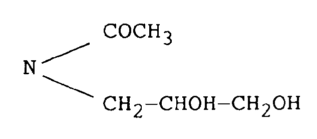 and R₃ and R₉ represent the group

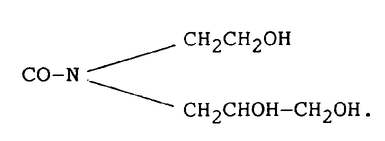

12. Preparation process for a compound of formula I according to claim 1 or 2, characterized in that these compounds are prepared by acetylation and/or alkylation reactions.

13. Process according to claim 12 for the preparation of compounds of formula I, characterized in that the following operations are carried out:
a) a couplins of compounds of formula 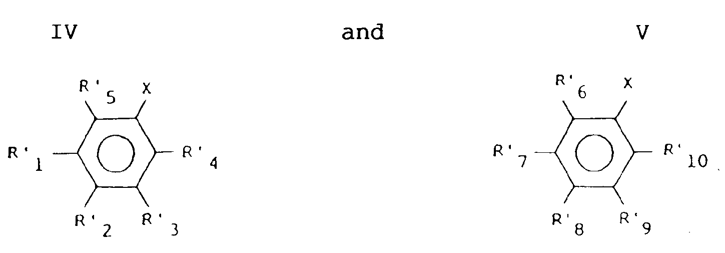 X being chosen from chlorine, bromine and iodine and R′₁, R′₂, R′₃, R′₄, R′₅, R′₆, R′₇, R′₈, R′₉ and R′ ₁₀, identical or different, being selected from the NO₂ and -CO₂R groups, R being a C₁-C₆ alkyl group, in a suitable solvent in the presence of a metal catalyst in order to obtain a compound of formula VI: 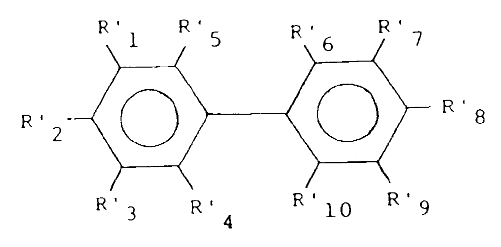
b) an amidation of the -COOR groups by an amine of formula: 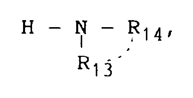 R₁₃ and R₁₄ being defined as in claim 1,
c) a reduction of the nitro groups to amine groups;
d) an iodination under standard conditions;
e) an optional protection of the hydroxy groups using the usual protecting groups;
f) an acylation of the aromatic amino groups in the presence of an acid chloride of formula R′₁₂-COCl, R′₁₂ corresponding to R₁₂ as defined in claim 1, the -OH groups of which are protected, and either:
g) an optional alkylation of the amido groups in the presence of a strong base and an alkylating reagent of formula Z - R₁₁, Z being a labile group chosen from Cl, Br and I and R₁₁ being as defined in claim 1; and a deprotection of the protected hydroxy functions, or:
h) a deprotection of the protected hydroxy functions and optional alkylation of the amido groups by an alkylating reagent of formula Z - R₁₁, Z being a labile group chosen from Cl, Br and I and R₁₁ being as defined in claim 1.

14. Process according to claim 12 for the preparation of the compounds of formula II in which R₂, R₄, R₅, R₆, R₈ and R₁₀ represent an 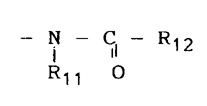 group,
R₁₁ and R₁₂ being as defined in claim 1, characterized in that the following operations are carried out:
a) a coupling of a compound of formula VII 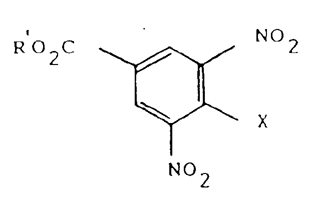 R′ representing a methyl or ethyl group and X being chosen from a chlorine, bromine or iodine atom, in order to obtain a compound of formula VIII 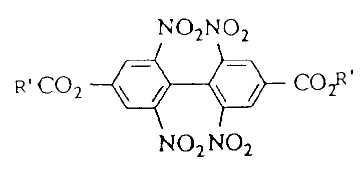
b) a rearrangement, after saponification of the esters and hydrolysis, in order to obtain a diamine of formula IX 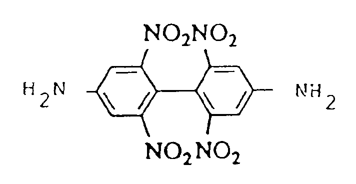
c) an acylation of the amino groups in the presence of an acid chloride of formula R′₁₂ - COCl, R′₁₂ corresponding to R₁₂ as defined in claim 1, the -OH groups of which are or are not protected;
d) a reduction of the nitro groups to amino groups;
e) an iodination of the aromatic amino groups under standard conditions;
f) an acylation by an acid chloride of formula R′₁₂ - COCl, R′₁₂ corresponding to R′₁₂ as defined in claim 1, the -OH groups of which are protected;
g) an optional deprotection; and
h) an optional alkylation of the amido groups by means of an alkylating reagent of formula Z - R₁₁, Z being a labile group chosen from chlorine, bromine and iodine and R₁₁ being as defined in claim 1.

15. Preparation process for compounds of formula III according to claim 8, characterized in that the following operations are carried out:
a) a diazotation of a compound of formula X: 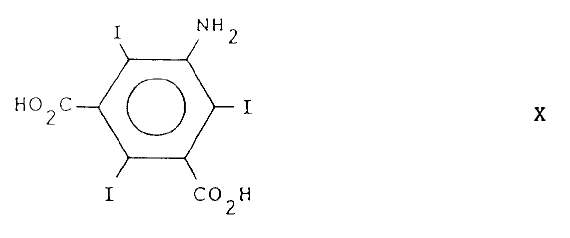 in order to obtain a compound of formula XI: 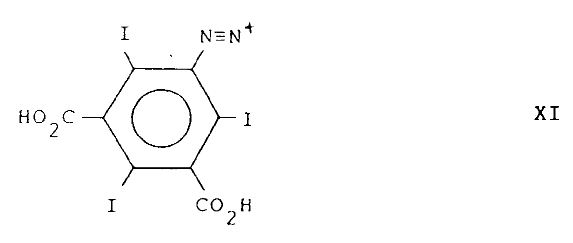
b) a coupling of the compound of formula XI in order to obtain a compound of formula XII: 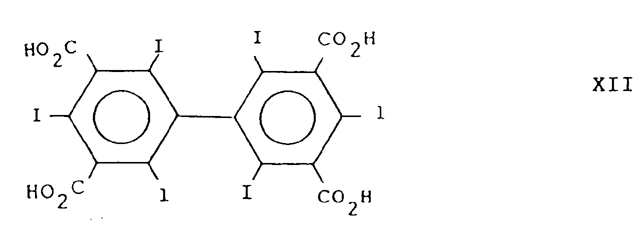
c) a conversion of the compound of formula XII into its corresponding acid chloride, and
d) an amidation of the compound obtained with an amine of formula: 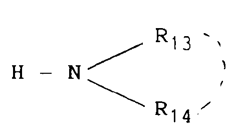 R₁₃ and R₁₄ being as defined in claim 1 in order to obtain a compound of formula III.

16. Contrast medium characterized in that it contains at least one compound according to any one of claims 1 to 12.

17. Contrast medium according to claim 16 characterized in that it is constituted by an aqueous solution of the compound or compounds.

18. Contrast medium for X-ray radiology characterized in that it contains at least one compound according to any one of claims 1 to 11.
